# EUROPÄISCHE PATENTSCHRIFT

(11) **EP 1 763 334 B1**
(45) Veröffentlichungstag und Bekanntmachung des Hinweises auf die Patenterteilung: **17.12.2008**
(21) Anmeldenummer: 05744792.2
(22) Anmeldetag: 24.05.2005
(51) Int. Cl.: A61K 8/64, A61Q 3/00, A61Q 5/12, A61Q 19/00

(54) **KERATIN-BINDENDE POLYPEPTIDE**
KERATIN-BINDING POLYPEPTIDES
POLYPEPTIDE LIANT LA KERATINE

(30) Priorität: 24.05.2004 DE 102004025805; 14.03.2005 DE 102005011988
(43) Veröffentlichungstag der Anmeldung: 21.03.2007
(73) Patentinhaber: BASF SE, 67056 Ludwigshafen (DE)
(72) Erfinder: BARG, Heiko, 67346 Speyer (DE); SUBKOWSKI, Thomas, 68526 Ladenburg (DE); LEMAIRE, Hans-Georg, 67117 Limburgerhof (DE); BOLLSCHWEILER, Claus, 69118 Heidelberg (DE); PTOCK, Arne, 67067 Ludwigshafen (DE)
(86) Internationale Anmeldenummer: PCT/EP2005/005599
(87) Internationale Veröffentlichungsnummer: WO 2005/115306

(56) Entgegenhaltungen:
- WO-A-00/55320
- US-A1- 2005 170 366
- CHEONG J E LAI ET AL: "Molecular abnormalities of the desmosomal protein desmoplakin in human disease." CLINICAL AND EXPERIMENTAL DERMATOLOGY. MAY 2005, Bd. 30, Nr. 3, Mai 2005 (2005-05), Seiten 261-266, XP002355355 ISSN: 0307-6938

### SEQUENCE LISTING

<110> BASF Aktiengesellschaft
<120> Die vorliegende Erfindung betrifft die Verwendung von Keratin-bindende Polypeptiden und ihre Herstellung
<130> AE20040313
<140> PF556.18
<141> 2005 05 23
<160> 1
<170> PatentIn version 3.1
<210> 1
<211> 2871
<212> PRT
<213> Homo sapiens
<400> 1
   Met Ser Cys Asn Gly Gly Ser His Pro Arg Ile Asn Thr Leu Gly Arg
   1               5                   10                  15
   Met Ile Arg Ala Glu Ser Gly Pro Asp Leu Arg Tyr Glu Val Thr Ser
               20                  25                  30
   Gly Gly Gly Gly Thr Ser Arg Met Tyr Tyr Ser Arg Arg Gly Val Ile
           35                  40                  45
   Thr Asp Gln Asn Ser Asp Gly Tyr Cys Gln Thr Gly Thr Met Ser Arg
   50 55 60
   His Gln Asn Gln Asn Thr Ile Gln Glu Leu Leu Gln Asn Cys Ser Asp
   65 70 75 80
   Cys Leu Met Arg Ala Glu Leu Ile Val Gln Pro Glu Leu Lys Tyr Gly
   85 90 95
   Asp Gly Ile Gln Leu Thr Arg Ser Arg Glu Leu Asp Glu Cys Phe Ala
   100 105 110
   Gln Ala Asn Asp Gln Met Glu Ile Leu Asp Ser Leu Ile Arg Glu Met
   115 120 125
   Arg Gln Met Gly Gln Pro Cys Asp Ala Tyr Gln Lys Arg Leu Leu Gln
   130 135 140
   Leu Gln Glu Gln Met Arg Ala Leu Tyr Lys Ala Ile Ser Val Pro Arg
   145 150 155 160
   Val Arg Arg Ala Ser Ser Lys Gly Gly Gly Gly Tyr Thr Cys Gln Ser
   165 170 175
   Gly Ser Gly Trp Asp Glu Phe Thr Lys His Val Thr Ser Glu Cys Leu
   180 185 190
   Gly Trp Met Arg Gln Gln Arg Ala Glu Met Asp Met Val Ala Trp Gly
   195 200 205
   Val Asp Leu Ala Ser Val Glu Gln His Ile Asn Ser His Arg Gly Ile
   210 215 220
   His Asn Ser Ile Gly Asp Tyr Arg Trp Gln Leu Asp Lys Ile Lys Ala
   225 230 235 240
   Asp Leu Arg Glu Lys Ser Ala Ile Tyr Gln Leu Glu Glu Glu Tyr Glu
   245 250 255
   Asn Leu Leu Lys Ala Ser Phe Glu Arg Met Asp His Leu Arg Gln Leu
   260 265 270
   Gln Asn Ile Ile Gln Ala Thr Ser Arg Glu Ile Met Trp Ile Asn Asp
   275 280 285
   Cys Glu Glu Glu Glu Leu Leu Tyr Asp Trp Ser Asp Lys Asn Thr Asn
   290 295 300
   Ile Ala Gln Lys Gln Glu Ala Phe Ser Ile Arg Met Ser Gln Leu Glu
   305 310 315 320
   Val Lys Glu Lys Glu Leu Asn Lys Leu Lys Gln Glu Ser Asp Gln Leu
   325 330 335
   Val Leu Asn Gln His Pro Ala Ser Asp Lys Ile Glu Ala Tyr Met Asp
   340 345 350
   Thr Leu Gln Thr Gln Trp Ser Trp Ile Leu Gln Ile Thr Lys Cys Ile
   355 360 365
   Asp Val His Leu Lys Glu Asn Ala Ala Tyr Phe Gln Phe Phe Glu Glu
   370 375 380
   Ala Gln Ser Thr Glu Ala Tyr Leu Lys Gly Leu Gln Asp Ser Ile Arg
   385 390 395 400
   Lys Lys Tyr Pro Cys Asp Lys Asn Met Pro Leu Gln His Leu Leu Glu
   405 410 415
   Gln Ile Lys Glu Leu Glu Lys Glu Arg Glu Lys Ile Leu Glu Tyr Lys
   420 425 430
   Arg Gln Val Gln Asn Leu Val Asn Lys Ser Lys Lys Ile Val Gln Leu
   435 440 445
   Lys Pro Arg Asn Pro Asp Tyr Arg Ser Asn Lys Pro Ile Ile Leu Arg
   450 455 460
   Ala Leu Cys Asp Tyr Lys Gln Asp Gln Lys Ile Val His Lys Gly Asp
   465 470 475 480
   Glu Cys Ile Leu Lys Asp Asn Asn Glu Arg Ser Lys Trp Tyr Val Thr
   485 490 495
   Gly Pro Gly Gly Val Asp Met Leu Val Pro Ser Val Gly Leu Ile Ile
   500 505 510
   Pro Pro Pro Asn Pro Leu Ala Val Asp Leu Ser Cys Lys Ile Glu Gln
   515 520 525
   Tyr Tyr Glu Ala Ile Leu Ala Leu Trp Asn Gln Leu Tyr Ile Asn Met
   530 535 540
   Lys Ser Leu Val Ser Trp His Tyr Cys Met Ile Asp Ile Glu Lys Ile
   545 550 555 560
   Arg Ala Met Thr Ile Ala Lys Leu Lys Thr Met Arg Gln Glu Asp Tyr
   565 570 575
   Met Lys Thr Ile Ala Asp Leu Glu Leu His Tyr Gln Glu Phe Ile Arg
   580 585 590
   Asn Ser Gln Gly Ser Glu Met Phe Gly Asp Asp Asp Lys Arg Lys Ile
   595 600 605
   Gln Ser Gln Phe Thr Asp Ala Gln Lys His Tyr Gln Thr Leu Val Ile
   610 615 620
   Gln Leu Pro Gly Tyr Pro Gln His Gln Thr Val Thr Thr Thr Glu Ile
   625 630 635 640
   Thr His His Gly Thr Cys Gln Asp Val Asn His Asn Lys Val Ile Glu
   645 650 655
   Thr Asn Arg Glu Asn Asp Lys Gln Glu Thr Trp Met Leu Met Glu Leu
   660 665 670
   Gln Lys Ile Arg Arg Gln Ile Glu His Cys Glu Gly Arg Met Thr Leu
   675 680 685
   Lys Asn Leu Pro Leu Ala Asp Gln Gly Ser Ser His His Ile Thr Val
   690 695 700
   Lys Ile Asn Glu Leu Lys Ser Val Gln Asn Asp Ser Gln Ala Ile Ala
   705 710 715 720
   Glu Val Leu Asn Gln Leu Lys Asp Met Leu Ala Asn Phe Arg Gly Ser
   725 730 735
   Glu Lys Tyr Cys Tyr Leu Gln Asn Glu Val Phe Gly Leu Phe Gln Lys
   740 745 750
   Leu Glu Asn Ile Asn Gly Val Thr Asp Gly Tyr Leu Asn Ser Leu Cys
   755 760 765
   Thr Val Arg Ala Leu Leu Gln Ala Ile Leu Gln Thr Glu Asp Met Leu
   770 775 780
   Lys Val Tyr Glu Ala Arg Leu Thr Glu Glu Glu Thr Val Cys Leu Asp
   785 790 795 800
   Leu Asp Lys Val Glu Ala Tyr Arg Cys Gly Leu Lys Lys Ile Lys Asn
   805 810 815
   Asp Leu Asn Leu Lys Lys Ser Leu Leu Ala Thr Met Lys Thr Glu Leu
   820 825 830
   Gln Lys Ala Gln Gln Ile His Ser Gln Thr Ser Gln Gln Tyr Pro Leu
   835 840 845
   Tyr Asp Leu Asp Leu Gly Lys Phe Gly Glu Lys Val Thr Gln Leu Thr
   850 855 860
   Asp Arg Trp Gln Arg Ile Asp Lys Gln Ile Asp Phe Arg Leu Trp Asp
   865 870 875 880
   Leu Glu Lys Gln Ile Lys Gln Leu Arg Asn Tyr Arg Asp Asn Tyr Gln
   885 890 895
   Ala Phe Cys Lys Trp Leu Tyr Asp Arg Lys Arg Arg Gln Asp Ser Leu
   900 905 910
   Glu Ser Met Lys Phe Gly Asp Ser Asn Thr Val Met Arg Phe Leu Asn
   915 920 925
   Glu Gln Lys Asn Leu His Ser Glu Ile Ser Gly Lys Arg Asp Lys Ser
   930 935 940
   Glu Glu Val Gln Lys Ile Ala Glu Leu Cys Ala Asn Ser Ile Lys Asp
   945 950 955 960
   Tyr Glu Leu Gln Leu Ala Ser Tyr Thr Ser Gly Leu Glu Thr Leu Leu
   965 970 975
   Asn Ile Pro Ile Lys Arg Thr Met Ile Gln Ser Pro Ser Gly Val Ile
   980 985 990
   Leu Gln Glu Ala Ala Asp Val His Ala Arg Tyr Ile Glu Leu Leu Thr
   995 1000 1005
   Arg Ser Gly Asp Tyr Tyr Arg Phe Leu Ser Glu Met Leu Lys Ser
   1010 1015 1020
   Leu Glu Asp Leu Lys Leu Lys Asn Thr Lys Ile Glu Val Leu Glu
   1025 1030 1035
   Glu Glu Leu Arg Leu Ala Arg Asp Ala Asn Ser Glu Asn Cys Asn
   1040 1045 1050
   Lys Asn Lys Phe Leu Asp Gln Asn Leu Gln Lys Tyr Gln Ala Glu
   1055 1060 1065
   Cys Ser Gln Phe Lys Ala Lys Leu Ala Ser Leu Glu Glu Leu Lys
   1070 1075 1080
   Arg Gln Ala Glu Leu Asp Gly Lys Ser Ala Lys Gln Asn Leu Asp
   1085 1090 1095
   Lys Cys Tyr Gly Gln Ile Lys Glu Leu Asn Glu Lys Ile Thr Arg
   1100 1105 1110
   Leu Thr Tyr Glu Ile Glu Asp Glu Lys Arg Arg Arg Lys Ser Val
   1115 1120 1125
   Glu Asp Arg Phe Asp Gln Gln Lys Asn Asp Tyr Asp Gln Leu Gln
   1130 1135 1140
   Lys Ala Arg Gln Cys Glu Lys Glu Asn Leu Gly Trp Gln Lys Leu
   1145 1150 1155
   Glu Ser Glu Lys Ala Ile Lys Glu Lys Glu Tyr Glu Ile Glu Arg
   1160 1165 1170
   Leu Arg Val Leu Leu Gln Glu Glu Gly Thr Arg Lys Arg Glu Tyr
   1175 1180 1185
   Glu Asn Glu Leu Ala Lys Val Arg Asn His Tyr Asn Glu Glu Met
   1190 1195 1200
   Ser Asn Leu Arg Asn Lys Tyr Glu Thr Glu Ile Asn Ile Thr Lys
   1205 1210 1215
   Thr Thr Ile Lys Glu Ile Ser Met Gln Lys Glu Asp Asp Ser Lys
   1220 1225 1230
   Asn Leu Arg Asn Gln Leu Asp Arg Leu Ser Arg Glu Asn Arg Asp
   1235 1240 1245
   Leu Lys Asp Glu Ile Val Arg Leu Asn Asp Ser Ile Leu Gln Ala
   1250 1255 1260
   Thr Glu Gln Arg Arg Arg Ala Glu Glu Asn Ala Leu Gln Gln Lys
   1265 1270 1275
   Ala Cys Gly Ser Glu Ile Met Gln Lys Lys Gln His Leu Glu Ile
   1280 1285 1290
   Glu Leu Lys Gln Val Met Gln Gln Arg Ser Glu Asp Asn Ala Arg
   1295 1300 1305
   His Lys Gln Ser Leu Glu Glu Ala Ala Lys Thr Ile Gln Asp Lys
   1310 1315 1320
   Asn Lys Glu Ile Glu Arg Leu Lys Ala Glu Phe Gln Glu Glu Ala
   1325 1330 1335
   Lys Arg Arg Trp Glu Tyr Glu Asn Glu Leu Ser Lys Val Arg Asn
   1340 1345 1350
   Asn Tyr Asp Glu Glu Ile Ile Ser Leu Lys Asn Gln Phe Glu Thr
   1355 1360 1365
   Glu Ile Asn Ile Thr Lys Thr Thr Ile His Gln Leu Thr Met Gln
   1370 1375 1380
   Lys Glu Glu Asp Thr Ser Gly Tyr Arg Ala Gln Ile Asp Asn Leu
   1385 1390 1395
   Thr Arg Glu Asn Arg Ser Leu Ser Glu Glu Ile Lys Arg Leu Lys
   1400 1405 1410
   Asn Thr Leu Thr Gln Thr Thr Glu Asn Leu Arg Arg Val Glu Glu
   1415 1420 1425
   Asp Ile Gln Gln Gln Lys Ala Thr Gly Ser Glu Val Ser Gln Arg
   1430 1435 1440
   Lys Gln Gln Leu Glu Val Glu Leu Arg Gln Val Thr Gln Met Arg
   1445 1450 1455
   Thr Glu Glu Ser Val Arg Tyr Lys Gln Ser Leu Asp Asp Ala Ala
   1460 1465 1470
   Lys Thr Ile Gln Asp Lys Asn Lys Glu Ile Glu Arg Leu Lys Gln
   1475 1480 1485
   Leu Ile Asp Lys Glu Thr Asn Asp Arg Lys Cys Leu Glu Asp Glu
   1490 1495 1500
   Asn Ala Arg Leu Gln Arg Val Gln Tyr Asp Leu Gln Lys Ala Asn
   1505 1510 1515
   Ser Ser Ala Thr Glu Thr Ile Asn Lys Leu Lys Val Gln Glu Gln
   1520 1525 1530
   Glu Leu Thr Arg Leu Arg Ile Asp Tyr Glu Arg Val Ser Gln Glu
   1535 1540 1545
   Arg Thr Val Lys Asp Gln Asp Ile Thr Arg Phe Gln Asn Ser Leu
   1550 1555 1560
   Lys Glu Leu Gln Leu Gln Lys Gln Lys Val Glu Glu Glu Leu Asn
   1565 1570 1575
   Arg Leu Lys Arg Thr Ala Ser Glu Asp Ser Cys Lys Arg Lys Lys
   1580 1585 1590
   Leu Glu Glu Glu Leu Glu Gly Met Arg Arg Ser Leu Lys Glu Gln
   1595 1600 1605
   Ala Ile Lys Ile Thr Asn Leu Thr Gln Gln Leu Glu Gln Ala Ser
   1610 1615 1620
   Ile Val Lys Lys Arg Ser Glu Asp Asp Leu Arg Gln Gln Arg Asp
   1625 1630 1635
   Val Leu Asp Gly His Leu Arg Glu Lys Gln Arg Thr Gln Glu Glu
   1640 1645 1650
   Leu Arg Arg Leu Ser Ser Glu Val Glu Ala Leu Arg Arg Gln Leu
   1655 1660 1665
   Leu Gln Glu Gln Glu Ser Val Lys Gln Ala His Leu Arg Asn Glu
   1670 1675 1680
   His Phe Gln Lys Ala Ile Glu Asp Lys Ser Arg Ser Leu Asn Glu
   1685 1690 1695
   Ser Lys Ile Glu Ile Glu Arg Leu Gln Ser Leu Thr Glu Asn Leu
   1700 1705 1710
   Thr Lys Glu His Leu Met Leu Glu Glu Glu Leu Arg Asn Leu Arg
   1715 1720 1725
   Leu Glu Tyr Asp Asp Leu Arg Arg Gly Arg Ser Glu Ala Asp Ser
   1730 1735 1740
   Asp Lys Asn Ala Thr Ile Leu Glu Leu Arg Ser Gln Leu Gln Ile
   1745 1750 1755
   Ser Asn Asn Arg Thr Leu Glu Leu Gln Gly Leu Ile Asn Asp Leu
   1760 1765 1770
   Gln Arg Glu Arg Glu Asn Leu Arg Gln Glu Ile Glu Lys Phe Gln
   1775 1780 1785
   Lys Gln Ala Leu Glu Ala Ser Asn Arg Ile Gln Glu Ser Lys Asn
   1790 1795 1800
   Gln Cys Thr Gln Val Val Gln Glu Arg Glu Ser Leu Leu Val Lys
   1805 1810 1815
   Ile Lys Val Leu Glu Gln Asp Lys Ala Arg Leu Gln Arg Leu Glu
   1820 1825 1830
   Asp Glu Leu Asn Arg Ala Lys Ser Thr Leu Glu Ala Glu Thr Arg
   1835 1840 1845
   Val Lys Gln Arg Leu Glu Cys Glu Lys Gln Gln Ile Gln Asn Asp
   1850 1855 1860
   Leu Asn Gln Trp Lys Thr Gln Tyr Ser Arg Lys Glu Glu Ala Ile
   1865 1870 1875
   Arg Lys Ile Glu Ser Glu Arg Glu Lys Ser Glu Arg Glu Lys Asn
   1880 1885 1890
   Ser Leu Arg Ser Glu Ile Glu Arg Leu Gln Ala Glu Ile Lys Arg
   1895 1900 1905
   Ile Glu Glu Arg Cys Arg Arg Lys Leu Glu Asp Ser Thr Arg Glu
   1910 1915 1920
   Thr Gln Ser Gln Leu Glu Thr Glu Arg Ser Arg Tyr Gln Arg Glu
   1925 1930 1935
   Ile Asp Lys Leu Arg Gln Arg Pro Tyr Gly Ser His Arg Glu Thr
   1940 1945 1950
   Gln Thr Glu Cys Glu Trp Thr Val Asp Thr Ser Lys Leu Val Phe
   1955 1960 1965
   Asp Gly Leu Arg Lys Lys Val Thr Ala Met Gln Leu Tyr Glu Cys
   1970 1975 1980
   Gln Leu Ile Asp Lys Thr Thr Leu Asp Lys Leu Leu Lys Gly Lys
   1985 1990 1995
   Lys Ser Val Glu Glu Val Ala Ser Glu Ile Gln Pro Phe Leu Arg
   2000 2005 2010
   Gly Ala Gly Ser Ile Ala Gly Ala Ser Ala Ser Pro Lys Glu Lys
   2015 2020 2025
   Tyr Ser Leu Val Glu Ala Lys Arg Lys Lys Leu Ile Ser Pro Glu
   2030 2035 2040
   Ser Thr Val Met Leu Leu Glu Ala Gln Ala Ala Thr Gly Gly Ile
   2045 2050 2055
   Ile Asp Pro His Arg Asn Glu Lys Leu Thr Val Asp Ser Ala Ile
   2060 2065 2070
   Ala Arg Asp Leu Ile Asp Phe Asp Asp Arg Gln Gln Ile Tyr Ala
   2075 2080 2085
   Ala Glu Lys Ala Ile Thr Gly Phe Asp Asp Pro Phe Ser Gly Lys
   2090 2095 2100
   Thr Val Ser Val Ser Glu Ala Ile Lys Lys Asn Leu Ile Asp Arg
   2105 2110 2115
   Glu Thr Gly Met Arg Leu Leu Glu Ala Gln Ile Ala Ser Gly Gly
   2120 2125 2130
   Val Val Asp Pro Val Asn Ser Val Phe Leu Pro Lys Asp Val Ala
   2135 2140 2145
   Leu Ala Arg Gly Leu Ile Asp Arg Asp Leu Tyr Arg Ser Leu Asn
   2150 2155 2160
   Asp Pro Arg Asp Ser Gln Lys Asn Phe Val Asp Pro Val Thr Lys
   2165 2170 2175
   Lys Lys Val Ser Tyr Val Gln Leu Lys Glu Arg Cys Arg Ile Glu
   2180 2185 2190
   Pro His Thr Gly Leu Leu Leu Leu Ser Val Gln Lys Arg Ser Met
   2195 2200 2205
   Ser Phe Gln Gly Ile Arg Gln Pro Val Thr Val Thr Glu Leu Val
   2210 2215 2220
   Asp Ser Gly Ile Leu Arg Pro Ser Thr Val Asn Glu Leu Glu Ser
   2225 2230 2235
   Gly Gln Ile Ser Tyr Asp Glu Val Gly Glu Arg Ile Lys Asp Phe
   2240 2245 2250
   Leu Gln Gly Ser Ser Cys Ile Ala Gly Ile Tyr Asn Glu Thr Thr
   2255 2260 2265
   Lys Gln Lys Leu Gly Ile Tyr Glu Ala Met Lys Ile Gly Leu Val
   2270 2275 2280
   Arg Pro Gly Thr Ala Leu Glu Leu Leu Glu Ala Gln Ala Ala Thr
   2285 2290 2295
   Gly Phe Ile Val Asp Pro Val Ser Asn Leu Arg Leu Pro Val Glu
   2300 2305 2310
   Glu Ala Tyr Lys Arg Gly Leu Val Gly Ile Glu Phe Lys Glu Lys
   2315 2320 2325
   Leu Leu Ser Ala Glu Arg Ala Val Thr Gly Tyr Asn Asp Pro Glu
   2330 2335 2340
   Thr Gly Asn Ile Ile Ser Leu Phe Gln Ala Met Asn Lys Glu Leu
   2345 2350 2355
   Ile Glu Lys Gly His Gly Ile Arg Leu Leu Glu Ala Gln Ile Ala
   2360 2365 2370
   Thr Gly Gly Ile Ile Asp Pro Lys Glu Ser His Arg Leu Pro Val
   2375 2380 2385
   Asp Ile Ala Tyr Lys Arg Gly Tyr Phe Asn Glu Glu Leu Ser Glu
   2390 2395 2400
   Ile Leu Ser Asp Pro Ser Asp Asp Thr Lys Gly Phe Phe Asp Pro
   2405 2410 2415
   Asn Thr Glu Glu Asn Leu Thr Tyr Leu Gln Leu Lys Glu Arg Cys
   2420 2425 2430
   Ile Lys Asp Glu Glu Thr Gly Leu Cys Leu Leu Pro Leu Lys Glu
   2435 2440 2445
   Lys Lys Lys Gln Val Gln Thr Ser Gln Lys Asn Thr Leu Arg Lys
   2450 2455 2460
   Arg Arg Val Val Ile Val Asp Pro Glu Thr Asn Lys Glu Met Ser
   2465 2470 2475
   Val Gln Glu Ala Tyr Lys Lys Gly Leu Ile Asp Tyr Glu Thr Phe
   2480 2485 2490
   Lys Glu Leu Cys Glu Gln Glu Cys Glu Trp Glu Glu Ile Thr Ile
   2495 2500 2505
   Thr Gly Ser Asp Gly Ser Thr Arg Val Val Leu Val Asp Arg Lys
   2510 2515 2520
   Thr Gly Ser Gln Tyr Asp Ile Gln Asp Ala Ile Asp Lys Gly Leu
   2525 2530 2535
   Val Asp Arg Lys Phe Phe Asp Gln Tyr Arg Ser Gly Ser Leu Ser
   2540 2545 2550
   Leu Thr Gln Phe Ala Asp Met Ile Ser Leu Lys Asn Gly Val Gly
   2555 2560 2565
   Thr Ser Ser Ser Met Gly Ser Gly Val Ser Asp Asp Val Phe Ser
   2570 2575 2580
   Ser Ser Arg His Glu Ser Val Ser Lys Ile Ser Thr Ile Ser Ser
   2585 2590 2595
   Val Arg Asn Leu Thr Ile Arg Ser Ser Ser Phe Ser Asp Thr Leu
   2600 2605 2610
   Glu Glu Ser Ser Pro Ile Ala Ala Ile Phe Asp Thr Glu Asn Leu
   2615 2620 2625
   Glu Lys Ile Ser Ile Thr Glu Gly Ile Glu Arg Gly Ile Val Asp
   2630 2635 2640
   Ser Ile Thr Gly Gln Arg Leu Leu Glu Ala Gln Ala Cys Thr Gly
   2645 2650 2655
   Gly Ile Ile His Pro Thr Thr Gly Gln Lys Leu Ser Leu Gln Asp
   2660 2665 2670
   Ala Val Ser Gln Gly Val Ile Asp Gln Asp Met Ala Thr Ser Val
   2675 2680 2685
   Lys Pro Ala Gln Lys Ala Phe Ile Gly Phe Glu Gly Val Lys Gly
   2690 2695 2700
   Lys Lys Lys Met Ser Ala Ala Glu Ala Val Lys Glu Lys Trp Leu
   2705 2710 2715
   Pro Tyr Glu Ala Gly Gln Arg Phe Leu Glu Phe Gln Tyr Leu Thr
   2720 2725 2730
   Gly Gly Leu Val Asp Pro Glu Val His Gly Arg Ile Ser Thr Glu
   2735 2740 2745
   Glu Ala Ile Arg Lys Gly Phe Ile Asp Gly Arg Ala Ala Gln Arg
   2750 2755 2760
   Leu Gln Asp Thr Ser Ser Tyr Ala Lys Ile Leu Thr Cys Pro Lys
   2765 2770 2775
   Thr Lys Leu Lys Ile Ser Tyr Lys Asp Ala Ile Asn Arg Ser Met
   2780 2785 2790
   Val Glu Asp Ile Thr Gly Leu Arg Leu Leu Glu Ala Ala Ser Val
   2795 2800 2805
   Ser Ser Lys Gly Leu Pro Ser Pro Tyr Asn Met Ser Ser Ala Pro
   2810 2815 2820
   Gly Ser Arg Ser Gly Ser Arg Ser Gly Ser Arg Ser Gly Ser Arg
   2825 2830 2835
   Ser Gly Ser Arg Ser Gly Ser Arg Arg Gly Ser Phe Asp Ala Thr
   2840 2845 2850
   Gly Asn Ser Ser Tyr Ser Tyr Ser Tyr Ser Phe Ser Ser Ser Ser
   2855 2860 2865
   Ile Gly His
   2870

## Beschreibung

### Stand der Technik

Vertebratenzellen enthalten Filamente, von denen eine Gruppe aus Keratinen aufgebaut ist. An diese Keratine, die auch in Haaren, Haut und Nägeln vorkommen, binden spezifische Proteine wie beispielsweise Desmoplakin mittels eines speziellen Sequenzmotifs, einer sogenannten Keratin-bindenden Domäne (Fontao L, Favre B, Riou S, Geerts D, Jaunin F, Saurat JH, Green KJ, Sonnenberg A, Borradori L, Interaction of the bullous pemphigoid antigen 1 (BP230) and desmoplakin with intermediate filaments is mediated by distinct sequences within their COOH terminus., Mol Biol Cell. 2003 May; 4(5):1978-92. Epub 2003 Jan 26; Hopkinson SB, Jones JC., The N terminus of the transmembrane protein BP180 interacts with the N-terminal domain of BP230, thereby mediating keratin cytoskeleton anchorage to the cell surface at the site of the hemidesmosome, Mol Biol Cell. 2000 Jan;11(1):277-86).

### Aufgabenstellung

Aufgabe der vorliegenden Erfindung war es, neue Polypeptide bereitzustellen, die eine hohe Affinität zu Keratin bzw. keratinhaltigen Stoffen wie Haut oder Haare besitzen. Solche Polypeptide eignen sich für die kosmetische und pharmazeutische Behandlung von keratinhaltigen Strukturen, insbesondere von Haaren und Haut.

### Beschreibung der Erfindung

Gegenstand der Erfindung sind kosmetische Zusammensetzungen zur Behandlung von keratinhaltigen Materialien, enthaltend mindestens eine Keratin-bindende Polypeptidsequenz (i) in einem kosmetisch verträglichen Medium, wobei die keratin-bindende polypeptidsequenz (i) dadurch gekennzeichnet ist, dass sie mindestens eine der folgenden Polypeptidsequenzen umfasst,
(a) die Polypeptidsequenz SEQ ID NO:1 Position 2193 bis 2481
(b) die Polypeptidsequenz SEQ ID NO:1 Position 2606 bis 2871
(c) eine Polypeptidsequenz, die gegenüber (a) in bis zu 60 % der Aminosäuren verändert ist,
(d) eine Polypeptidsequenz, die gegenüber (b) in bis zu 50 % der Aminosäuren verändert ist;
mit der Maßgabe, dass die Keratin-Bindung der Polypeptidsequenz (c) oder (d) mindestens 10 % des Wertes beträgt, den die Polypeptidsequenz (a) oder (b) aufweist, gemessen in dem Test gemäß Beispiel 9 oder 10.

### Polypeptidsequenzen (i)

Die Polypeptidsequenz (i) besitzt eine Bindungsaffinität zu einem Keratin. Die Bindung der Polypeptidsequenz (i) zu einem Keratin kann unter den in Beispiel 8, 9 und 10 beschriebenen Bedingungen getestet werden.

Besonders gut geeignete Keratin-bindende Polypeptide sind solche Sequenzen, die im humanen Desmoplakin enthalten sind oder durch Veränderung der humanen Desmoplakinpolypeptidsequenzen wie Aminosäure-Insertionen, -Substitutionen oder-Deletionen daraus abgeleitet sind.

Die Polypeptidsequenz des humanen Desmoplakins ist in SEQ ID NO: 1 dargestellt. Eine geeignete Keratin-bindende Domäne (Domäne B) ist die Polypeptidsequenz SEQ ID NO: 1 Position 2193 bis 2481 sowie deren funktionelle Äquivalente. Eine weitere Keratin-bindende Domäne (Domäne C) ist die Polypeptidsequenz SEQ ID NO: 1 Position 2606 bis 2871 sowie deren funktionelle Äquivalente.

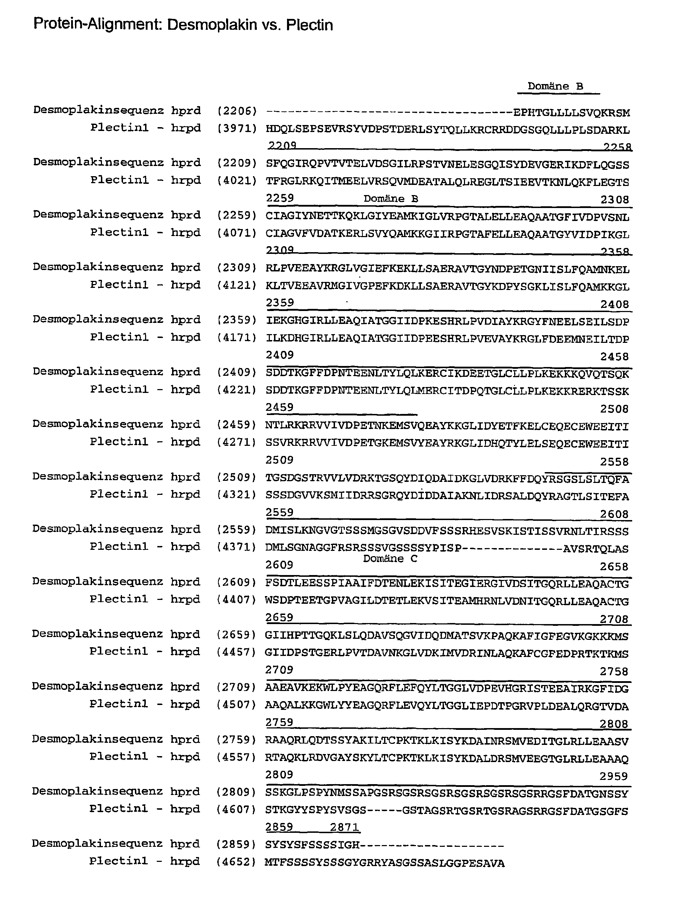

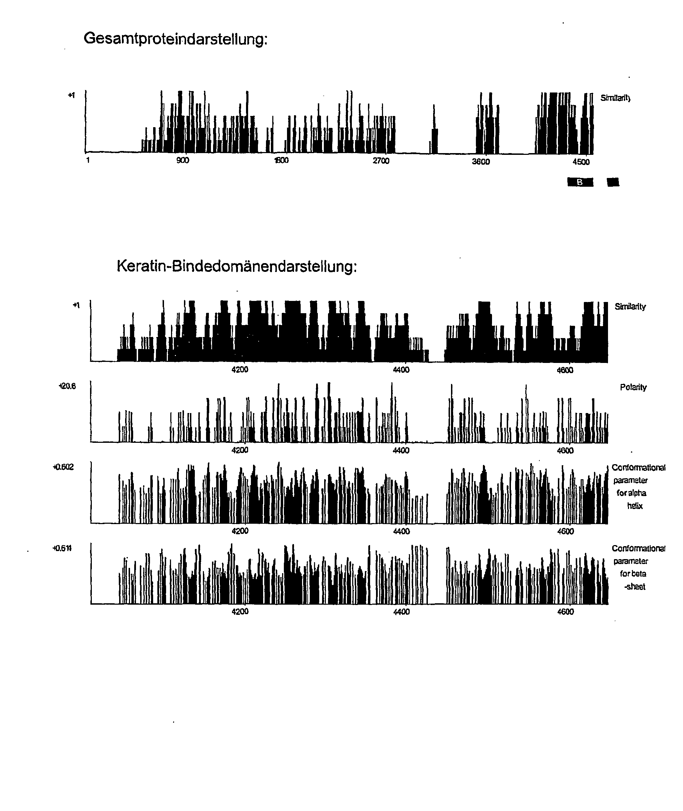

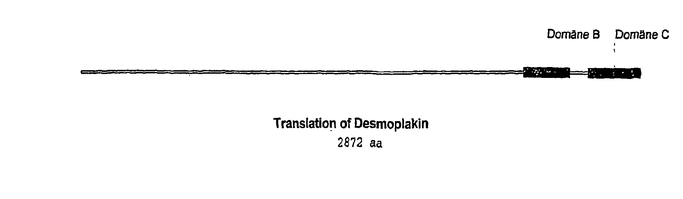

Die Keratin-bindende Domänen sind in Abbildung 1 dargestellt.

Bevorzugte Polypeptidsequenzen (i) umfassen die ober beschriebenen Fragmente einer Aminosäuresequenz gemäß SEQ ID NO: 1.

Erfindungsgemäß mit umfasst sind ebenfalls "funktionale Äquivalente" der konkret offenbarten Polypeptidsequenzen (i) wie oben in Punkt (c) und (d) definiert und die Verwendung dieser in den erfindungsgemäßen Verfahren.

"Funktionale Äquivalente" oder Analoga der konkret offenbarten Polypeptide (i) sind im Rahmen der vorliegenden Erfindung davon verschiedene Polypeptide, welche weiterhin die gewünschte biologische Aktivität, wie z.B. Keratinbindung, besitzen. So versteht man beispielsweise unter "funktionalen Äquivalenten" Polypeptidsequenzen die gemäß einem der in Beispiel 9 oder 10 beschriebenen Bindungstests eine mindestens 10 %ige Bindung zeigen, bevorzugt mindestens 50 %, besonders bevorzugt 75 %, ganz besonders bevorzugt 90 % der Bindung, die ein Polypeptid mit der Domäne B oder der Domäne C von SEQ ID NO: 1 in den Bindungstests gemäß Beispiel 9 oder 10 zeigt.

Beispiele für geeignete Aminosäuresubstitutionen sind folgender Tabelle zu entnehmen:

| Ursprünglicher Rest | Beispiele der Substitution |
|---|---|
| Ala | Ser |
| Arg | Lys |
| Asn | Gln; His |
| Asp | Glu |
| Cys | Ser |
| Gln | Asn |
| Glu | Asp |
| Gly | Pro |
| His | Asn; Gln |
| Ile | Leu; Val |
| Leu | Ile; Val |
| Lys | Arg; Gln; Glu |
| Met | Leu; Ile |
| Phe | Met; Leu; Tyr |
| Ser | Thr |
| Thr | Ser |
| Trp | Tyr |
| Tyr | Trp; Phe |
| Val | Ile; Leu |

Bekannt ist, dass in SEQ ID NO: 1 das an Position 2849 natürlich vorliegende Serin z.B. gegen Glycin ausgetauscht werden kann, um eine Phosphorylierung an dieser Position zu umgehen (Fontao L, Favre B, Riou S, Geerts D, Jaunin F, Saurat JH, Green KJ, Sonnenberg A, Borradori L., Interaction of the bullous pemphigoid antigen 1 (BP230) and desmoplakin with intermediate filaments is mediated by distinct sequences within their COOH terminus., Mol Biol Cell. 2003 May;14(5):1978-92. Epub 2003 Jan 26).

Unter "funktionalen Äquivalenten" versteht man erfindungsgemäß insbesondere auch Muteine, welche in wenigstens einer Sequenzposition der oben genannten Aminosäuresequenzen eine andere als die konkret genannte Aminosäure aufweisen aber trotzdem eine der oben genannten biologischen Aktivitäten besitzen. "Funktionale Äquivalente" umfassen somit die durch eine oder mehrere Aminosäure-Additionen, -Substitutionen, -Deletionen und/oder -Inversionen erhältlichen Muteine, wobei die genannten Veränderungen in jeglicher Sequenzposition auftreten können, solange sie zu einem Mutein mit dem erfindungsgemäßen Eigenschaftsprofil führen.

"Funktionale Äquivalente" im obigen Sinne sind auch "Präkursoren" der beschriebenen Polypeptide sowie "funktionale Derivate" und "Salze" der Polypeptide.

"Präkursoren" sind dabei natürliche oder synthetische Vorstufen der Polypeptide mit oder ohne gewünschte biologische Aktivität.

Unter dem Ausdruck "Salze" versteht man sowohl Salze von Carboxylgruppen als auch Säureadditionssalze von Aminogruppen der erfindungsgemäßen Proteinmoleküle. Salze von Carboxylgruppen können in an sich bekannter Weise hergestellt werden und umfassen anorganische Salze, wie zum Beispiel Natrium-, Calcium-, Ammonium-, Eisen- und Zinksalze, sowie Salze mit organischen Basen, wie zum Beispiel Aminen, wie Triethanolamin, Arginin, Lysin, Piperidin und dergleichen. Säureadditionssalze, wie zum Beispiel Salze mit Mineralsäuren, wie Salzsäure oder Schwefelsäure und Salze mit organischen Säuren, wie Essigsäure und Oxalsäure sind ebenfalls Gegenstand der Erfindung.

"Funktionale Derivate" erfindungsgemäßer Polypeptide können an funktionellen Aminosäure-Seitengruppen oder an deren N- oder C-terminalen Ende mit Hilfe bekannter Techniken ebenfalls hergestellt werden. Derartige Derivate umfassen beispielsweise Ester oder Thioester von Carbonsäuregruppen, Amide von Carbonsäuregruppen, erhältlich durch Umsetzung mit Ammoniak oder mit einem primären oder sekundären Amin; N-Acylderivate freier Aminogruppen, hergestellt durch Umsetzung mit Acylierungsmitteln; N-Alkylderivate freier Aminogruppen, hergestellt durch Umsetzung mit Alkylierungsmitteln; S-Acylderivate freier Mercaptogruppen, hergestellt durch Umsetzung mit Acylierungsmitteln; Thioether durch Umsetzung freier Mercaptogruppen mit Alkylierungsmitteln; Disulfide durch Umsetzung freier Mercaptogruppen z.B. mit Thiolen; O-Acylderivate freier Hydroxygruppen, hergestellt durch Umsetzung mit Acylierungsmitteln; oder Ether durch Umsetzung freier Hydroxylgruppen mit Alkylierungsmitteln.

"Funktionale Äquivalente" umfassen natürlich auch Polypeptide, welche aus anderen Organismen zugänglich sind, sowie natürlich vorkommende Varianten. Beispielsweise lassen sich durch Sequenzvergleich Bereiche homologer Sequenzregionen festlegen und in Anlehnung an die konkreten Vorgaben der Erfindung äquivalente Enzyme ermitteln.

"Funktionale Äquivalente" umfassen ebenfalls Fragmente, vorzugsweise einzelne Domänen oder Sequenzmotive, der erfindungsgemäßen Polypeptide, welche z.B. die gewünschte biologische Funktion aufweisen.

"Funktionale Äquivalente" sind außerdem Fusionsproteine, welche eine der oben genannten Polypeptidsequenzen oder davon abgeleitete funktionale Äquivalente und wenigstens eine weitere, davon funktionell verschiedene, heterologe Sequenz in funktioneller N- oder C-terminaler Verknüpfung (d.h. ohne gegenseitigen wesentliche funktionelle Beeinträchtigung der Fusionsproteinteile) aufweisen. Nichtlimitierende Beispiele für derartige heterologe Sequenzen sind z.B. Signal peptide oder Enzyme.

Erfindungsgemäß mit umfasste "funktionale Äquivalente" sind Homologe zu den konkret offenbarten Proteinen. Diese besitzen wenigstens 50 %, vorzugsweise wenigstens 75 %, insbesondere wenigstens 85 %, wie z.B. 90 %, 95 % oder 99 %, Homologie zu einer der konkret offenbarten Aminosäuresequenzen, berechnet nach dem Algorithmus von Pearson und Lipman, Proc. Natl. Acad. Sci. (USA) 85 (8), 1988, 2444-2448. Eine prozentuale Homologie eines erfindungsgemäßen homologen Polypeptids bedeutet insbesondere prozentuale Identität der Aminosäurereste bezogen auf die Gesamtlänge einer der hierin konkret beschriebenen Aminosäuresequenzen.

Aus WO0055320 ist ein Polypeptid (SEQ ID NO:604) bekannt, das mit Pankreaskrebsassoziiert wird. Diese Polypeptidsequenz weist eine hohen Sequenzidentität (63,7%-98,9%) zu den Sequenzbereichen von SEQ ID NO:1 mit den Positionen 2193-2481 und 2606-2871 auf. Es werden in WO0055320 jedoch keine konkreten kosmetische Zusammensetzungen ausführbar offenbart.

Im Falle einer möglichen Proteinglykosylierung umfassen erfindungsgemäße "funktionale Äquivalente" Proteine des oben bezeichneten Typs in deglykosylierter bzw. glykosylierter Form sowie durch Veränderung des Glykosylierungsmusters erhältliche abgewandelte Formen.

Im Falle einer möglichen Proteinphosphorylierung umfassen erfindungsgemäße "funktionale Äquivalente" Proteine des oben bezeichneten Typs in dephosphorylierter bzw. phosphorylierter Form sowie durch Veränderung des Phosphorylierungsmusters erhältliche abgewandelte Formen.

Homologe der erfindungsgemäßen Polypeptide (i) können durch Mutagenese erzeugt werden, z.B. durch Punktmutation oder Verkürzung des Proteins.

Homologe des erfindungsgemäßen Polypeptide können durch Screening kombinatorischer Banken von Mutanten, wie z.B. Verkürzungsmutanten, identifiziert werden. Beispielsweise kann eine Bank von Protein-Varianten durch kombinatorische Mutagenese auf Nukleinsäureebene erzeugt werden, wie z.B. durch enzymatisches Ligieren eines Gemisches synthetischer Oligonukleotide. Es gibt eine Vielzahl von Verfahren, die zur Herstellung von Banken potentieller Homologer aus einer degenerierten Oligonukleotidsequenz verwendet werden können. Die chemische Synthese einer degenerierten Gensequenz kann in einem DNA-Syntheseautomaten durchgeführt werden, und das synthetische Gen kann dann in einen geeigneten Expressionsvektor ligiert werden. Die Verwendung eines degenerierten Gensatzes ermöglicht die Bereitstellung sämtlicher Sequenzen in einem Gemisch, die den gewünschten Satz an potentiellen Proteinsequenzen kodieren. Verfahren zur Synthese degenerierter Oligonukleotide sind dem Fachmann bekannt (z.B. Narang, S.A. (1983) Tetrahedron 39:3; Itakura et al. (1984) Annu. Rev. Biochem. 53:323; Itakura et al., (1984) Science 198:1056; Ike et al. (1983) Nucleic Acids Res. 11:477).

Im Stand der Technik sind mehrere Techniken zum Screening von Genprodukten kombinatorischer Banken, die durch Punktmutationen oder Verkürzung hergestellt worden sind, und zum Screening von cDNA-Banken auf Genprodukte mit einer ausgewählten Eigenschaft bekannt. Diese Techniken lassen sich an das schnelle Screening der Genbanken anpassen, die durch kombinatorische Mutagenese erfindungsgemäßer Homologer erzeugt worden sind. Die am häufigsten verwendeten Techniken zum Screening großer Genbanken, die einer Analyse mit hohem Durchsatz unterliegen, umfassen das Klonieren der Genbank in replizierbare Expressionsvektoren, Transformieren der geeigneten Zellen mit der resultierenden Vektorenbank und Exprimieren der kombinatorischen Gene unter Bedingungen, unter denen der Nachweis der gewünschten Aktivität die Isolation des Vektors, der das Gen kodiert, dessen Produkt nachgewiesen wurde, erleichtert. Recursive-Ensemble-Mutagenese (REM), eine Technik, die die Häufigkeit funktioneller Mutanten in den Banken vergrößert, kann in Kombination mit den Screeningtests verwendet werden, um Homologe zu identifizieren (Arkin und Yourvan (1992) PNAS 89:7811-7815; Delgrave et al. (1993) Protein Engineering 6(3):327-331).

Erfindungsgemäß sind solche Polypeptidsequenzen (i) umfasst, die mindestens eine der folgenden Polypeptidsequenzen umfassen,
a) die Polypeptidsequenz SEQ ID NO: 1 Position 2193 bis 2481 (Domäne B)
b) die Polypeptidsequenz SEQ ID NO: 1 Position 2606 bis 2871 (Domäne C)
c) eine Polypeptidsequenz, die gegenüber (a) in bis zu 60 % der Aminosäuren verändert ist,
d) eine Polypeptidsequenz, die gegenüber (b) in bis zu 50 % der Aminosäuren verändert ist,
mit der Massgabe, dass die Keratin-Bindung der Polypeptidsequenz (c) oder (d) mindestens 10 % des Wertes beträgt, den die Polypeptidsequenz (a) oder (b) aufweist, gemessen in dem Test gemäß Beispiel 9 oder 10. Mit Domäne B bzw. C sind hier die oben beschriebenen Keratinbindedomänen des humanen Desmoplakins (SEQ ID NO: 1) gemeint. Mit Veränderung von Aminosäuren sind hiermit Aminosäure-Substitutionen, -Insertionen und -Deletionen oder beliebige Kombinationen aus diesen drei Möglichkeiten gemeint.

Bevorzugt werden Polypeptidsequenzen (i) verwendet, die für den gewünschten Organismus eine hochspezifische Affinität besitzen. Für Anwendungen in der Hautkosmetik werden demzufolge Polypeptidsequenzen (i) bevorzugt eingesetzt, die zu dem humanen Hautkeratin eine besonders hohe Affinität haben. Für Anwendungen in der Haarkosmetik werden solche Polypeptidsequenzen bevorzugt, die zu humanem Haarkeratin eine besonders hohe Affinität haben.

Für Anwendungen auf dem Haustiergebiet werden, neben den beschriebenen Polypeptidsequenzen (SEQ ID NO: 1), entsprechend solche Polypeptidsequenzen (i) bevorzugt, die zu dem entsprechenden Keratin, beispielsweise Hundekeratin oder Katzenkeratin eine besonders hohe Affinität besitzen.

Es können aber auch mehr als eine Polypeptidsequenz (i) in dem erfindungsgemäßen Effektormolekül verwendet werden, beispielsweise eine Sequenz (i), die eine hohe Bindungsaffinität zu humanem Hautkeratin besitzt, in Verbindung mit einer Sequenz (i), die eine hohe Affinität zu humanem Haarkeratin besitzt. Es können auch mehrere Kopien der gleichen Polypeptidsequenz (i) hintereinander geschaltet werden, um beispielsweise eine höhere Bindung zu erzielen.

Geeignete Keratin-bindende Polypeptidsequenzen (i) sind bekannt. Beispielsweise enthalten Desmoplakine und Plectine Keratin-bindende Domänen (Fontao L, Favre B, Riou S, Geerts D, Jaunin F, Saurat JH, Green KJ, Sonnenberg A, Borradori L., Interaction of the bullous pemphigoid antigen 1 (BP230) and desmoplakin with intermediate filaments is mediated by distinct sequences within their COOH terminus., Mol Biol Cell. 2003 May;14(5):1978-92. Epub 2003 Jan 26; Hopkinson SB, Jones JC., The N terminus of the transmembrane protein BP180 interacts with the N-terminal domain of BP230, thereby mediating keratin cytoskeleton anchorage to the cell surface at the site of the hemidesmosome, Mol Biol Cell. 2000 Jan;11(1):277-86).

Durch Alignments solcher bekannter Proteinsequenzen, beispielsweise mit einem Computerprogramm wie Vector NTI 8 (Version vom 25. September 2002) der Firma InforMax Inc. können solche Bereiche kartiert und identifiziert werden.

Weitere geeignete Polypeptidsequenzen (i) mit einer guten Bindung zu humanem Keratin sind Sequenzbereiche, die in einem Alignment hohe Homologie bzw. Sequenzidentität aufweisen und als Konsensussequenzen der Keratindindedomänen aufgefasst werden können.

Besonders bevorzugt sind unter diesen Sequenzbereichen die folgenden:
Domäne B (KBD-B): Polypeptidsequenz SEQ ID NO: 1 Position 2193 bis 2448
Domäne B (KBD-B): Polypeptidsequenz SEQ ID NO: 1 Position 2209 bis 2448
Domäne C (KBD-C): Polypeptidsequenz SEQ ID NO: 1 Position 2606 bis 2871
Domäne C (KBD-C): Polypeptidsequenz SEQ ID NO: 1 Position 2616 bis 2871
Domäne C (KBD-C): Polypeptidsequenz SEQ ID NO: 1 Position 2616 bis 2811
Domäne C (KBD-C): Polypeptidsequenz SEQ ID NO: 1 Position 2606 bis 2871

Bekannt ist, dass in SEQ ID NO: 1 das an Position 2849 natürlich vorliegende Serin z.B. gegen Glycin ausgetauscht werden kann, um eine Phosphorylierung an dieser Position zu umgehen und damit eine Bindung der Domäne C an das entsprechende Keratin zu gewährleisten (Fontao L, Favre B, Riou S, Geerts D, Jaunin F, Saurat JH, Green KJ, Sonnenberg A, Borradori L., Interaction of the bullous pemphigoid antigen 1 (BP230) and desmoplakin with intermediate filaments is mediated by distinct sequences within their COOH terminus., Mol Biol Cell. 2003 May;14(5):1978-92. Epub 2003 Jan 26).

Wenn es gewünscht wird, dass die Polypeptidsequenzen (i) zu einem Keratin aus einem nicht-humanen Organismus eine besonders gute Bindung aufweisen, werden als geeignete Sequenzmotive bevorzugt solche aus dem Keratin-bindenden Protein, z.B. Desmoplakin oder Plectin, des entsprechenden Organismus ausgewählt.

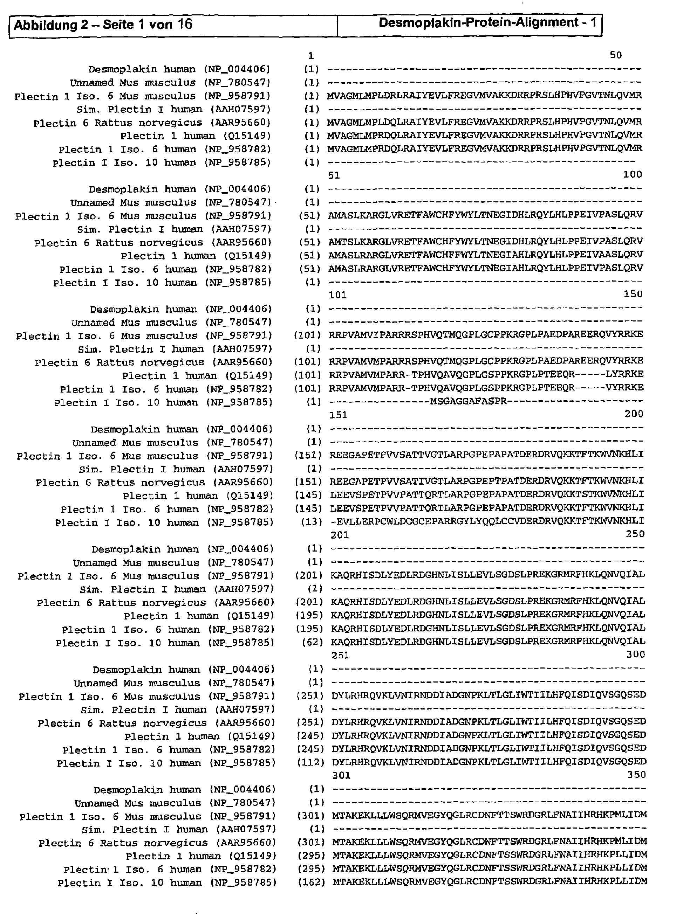

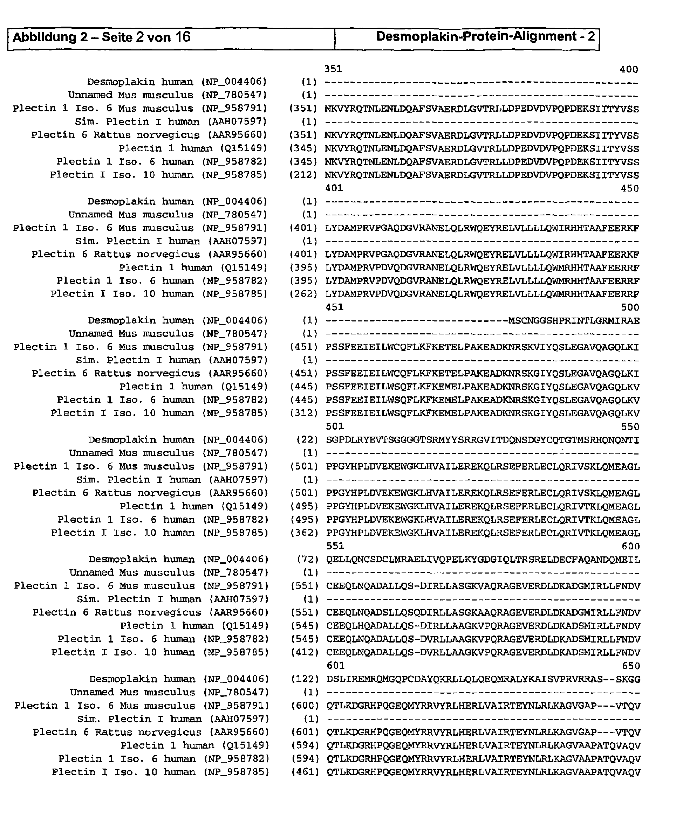

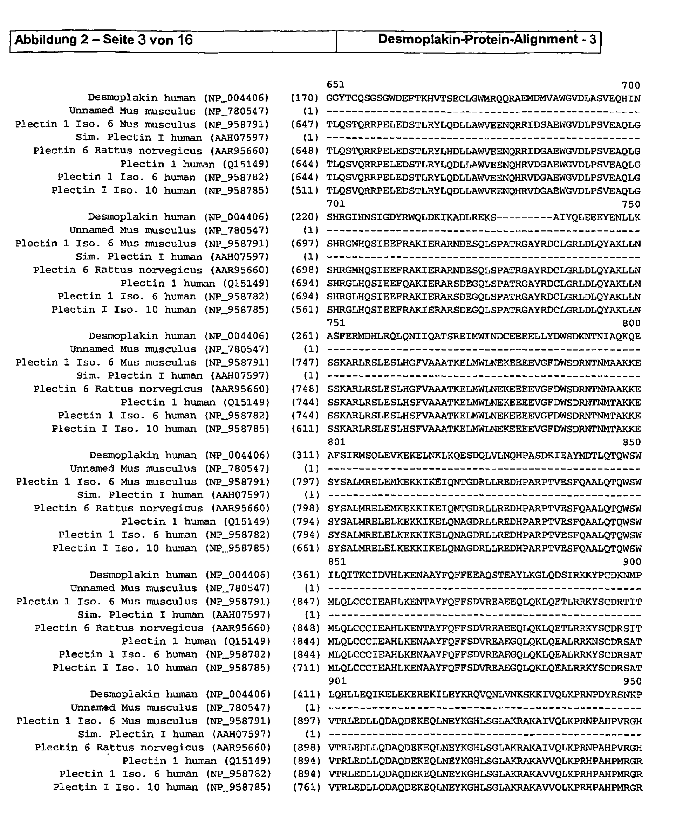

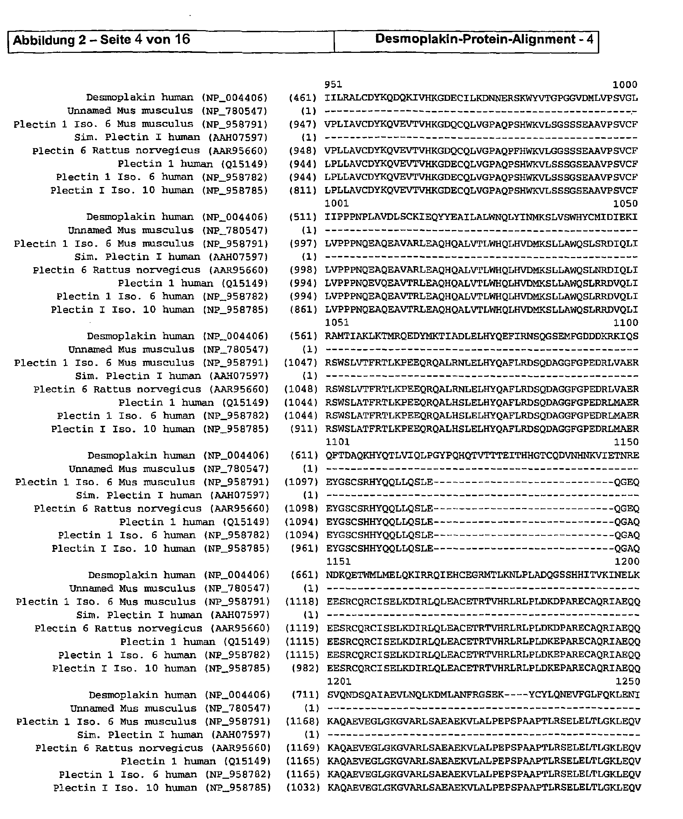

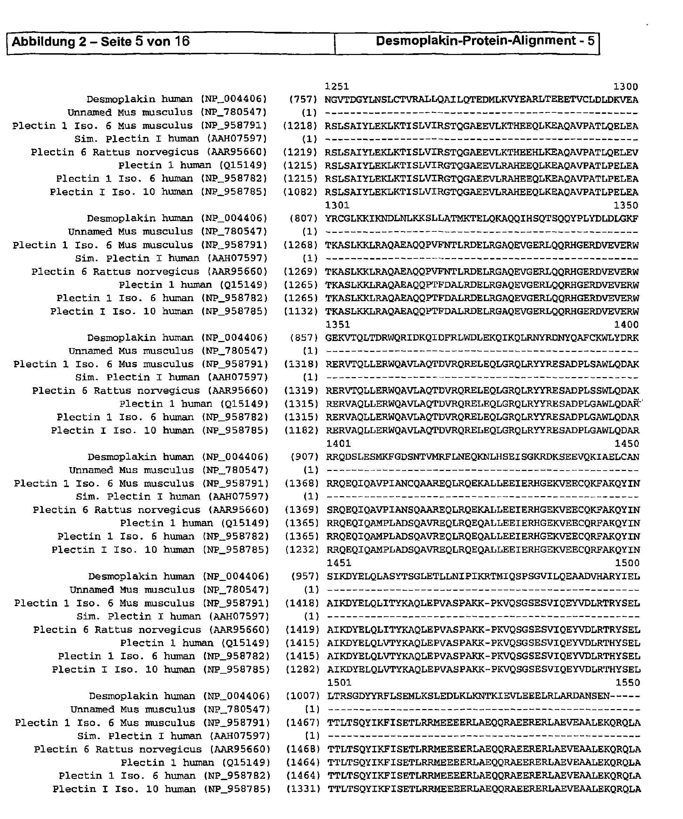

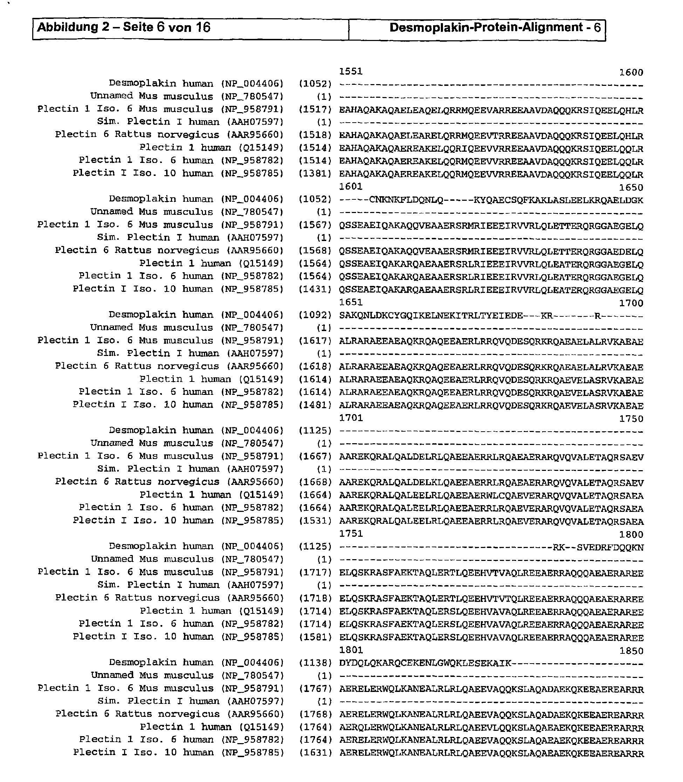

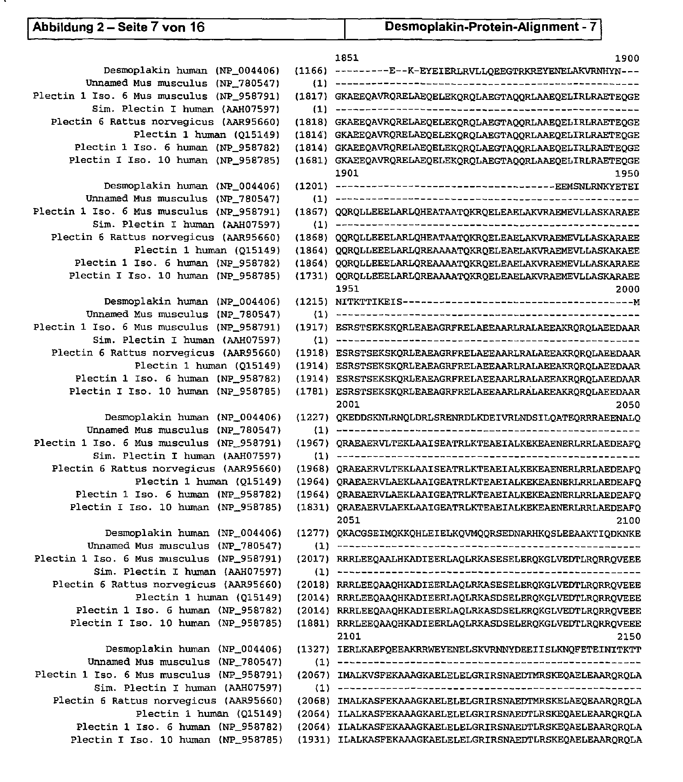

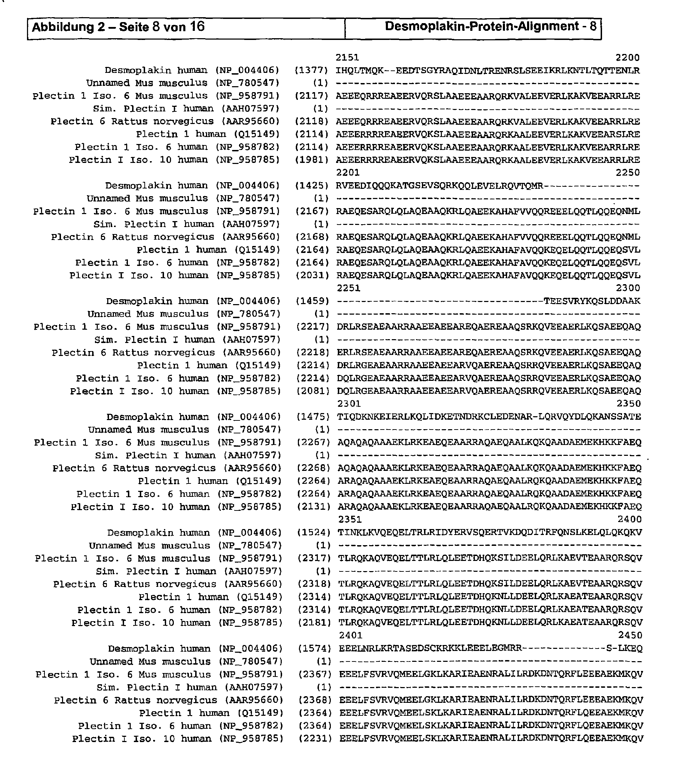

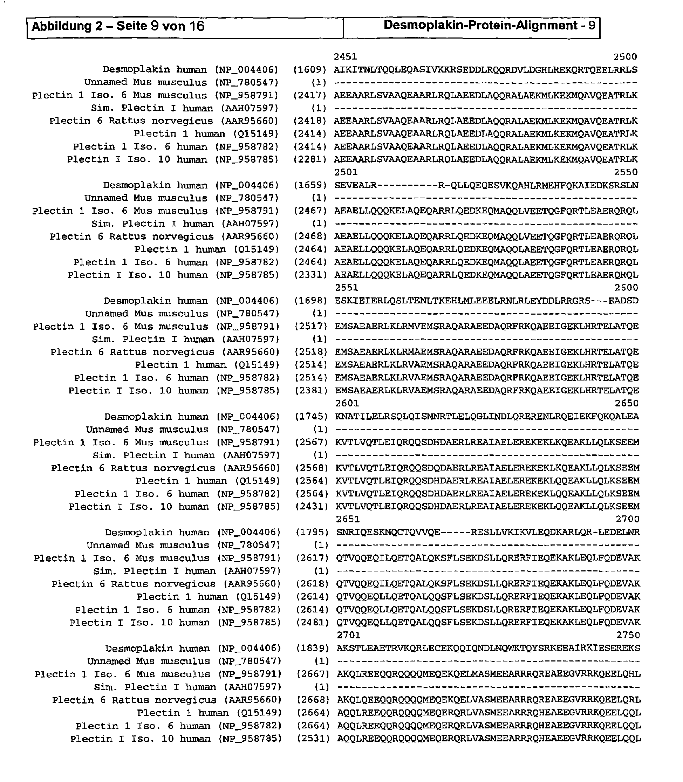

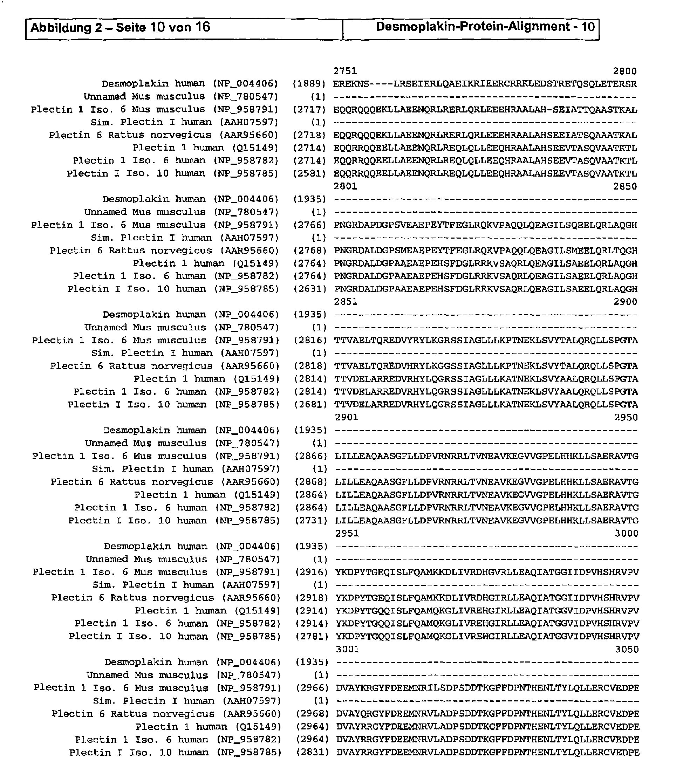

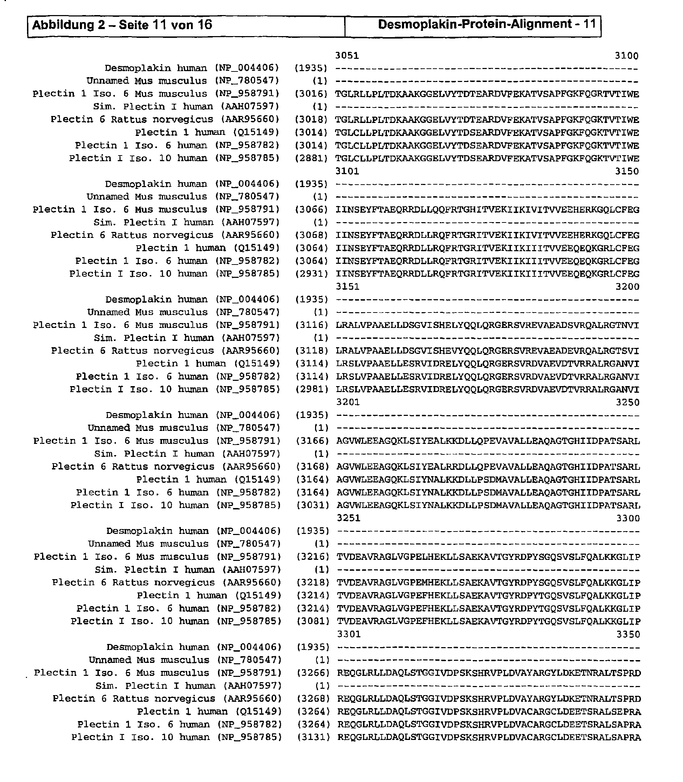

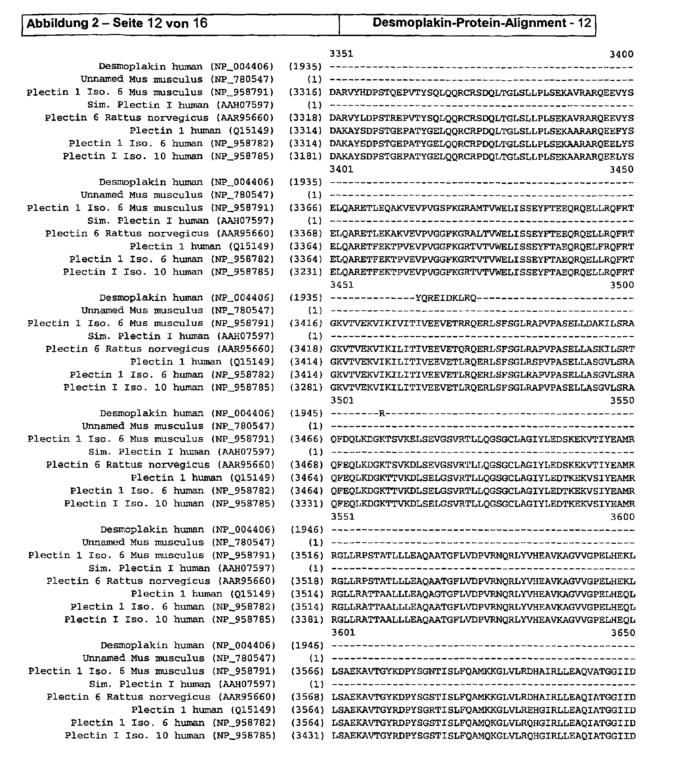

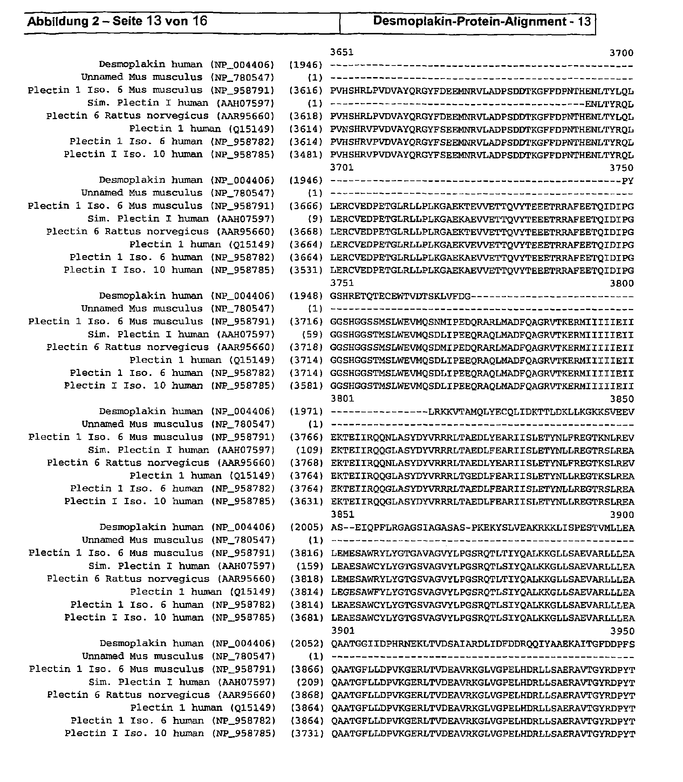

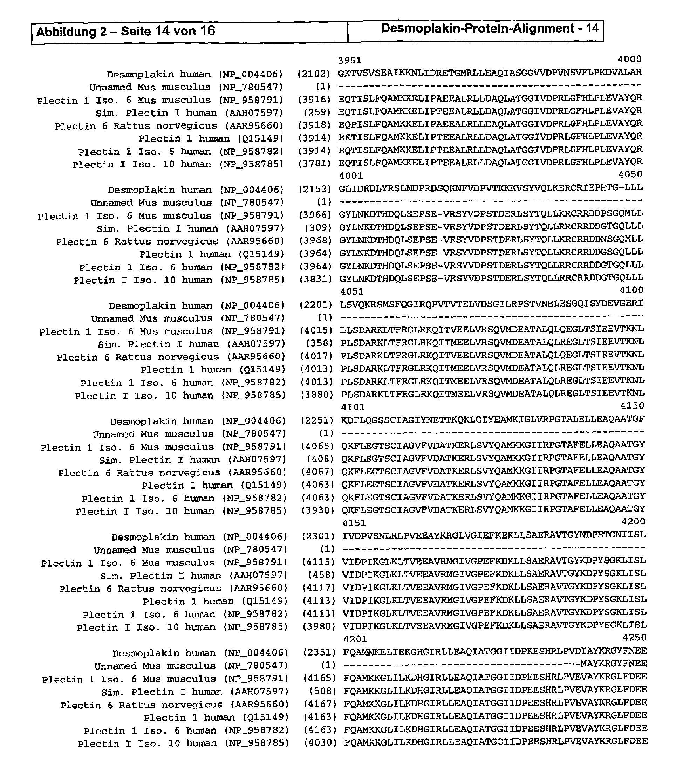

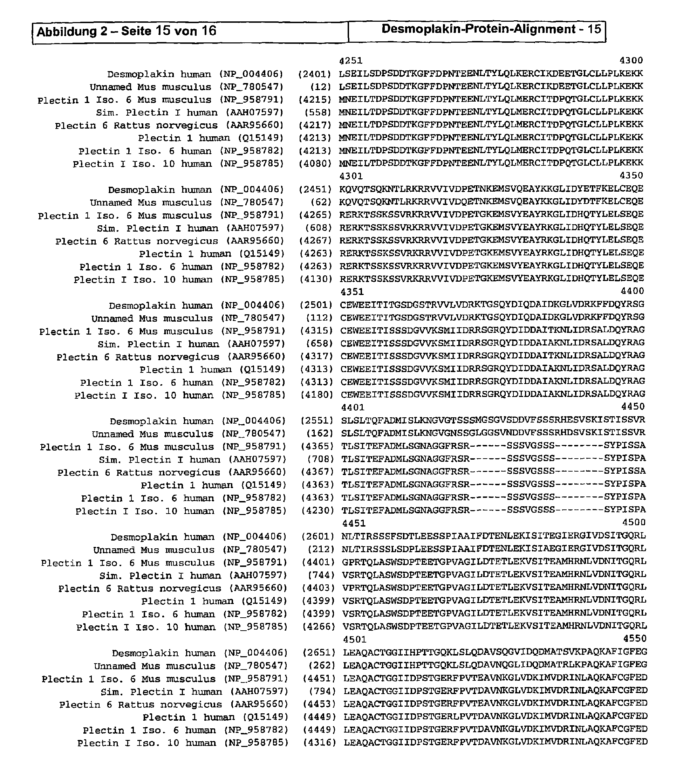

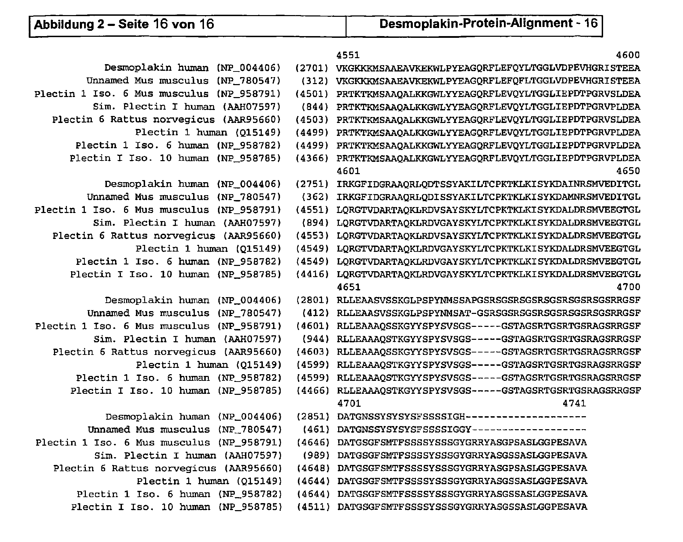

Abb. 2 zeigt ein Alignment von Keratin-bindenden Molekülen.

Die erfindungsgemäßen Keratin-bindenden Polypeptide (i) können auch - falls gewünscht - wieder leicht vom Keratin getrennt werden. Hierzu kann beispielsweise eine Spülung mit Keratin eingesetzt werden, wodurch die Keratin-bindenden Polypeptide (i) aus ihrer bestehenden Bindung zum Keratin verdrängt werden und mit dem Keratin aus der Spülung abgesättigt werden. Alternativ ist auch eine Spülung mit einem hohen Anteil an Detergenz (z.B. SDS) zum Abwaschen möglich.

Die erfindungsgemäßen Keratin-bindenden Polypeptide (i) besitzen ein weites Anwendungsgebiet in der Humankosmetik, insbesondere der Haut-, Nagel- und Haarpflege, der Tierpflege, der Lederpflege und Lederbearbeitung.

Bevorzugt werden die erfindungsgemäßen Keratin-bindenden Polypeptide (i) für die Hautkosmetik angewendet. Sie erlauben eine hohe Konzentration und lange Wirkdauer von hautpflegenden oder hautschützenden Effektorstoffen.

Geeignete Hilfs- und Zusatzstoffe für die Herstellung von haarkosmetischen, nagelkosmetischen oder hautkosmetischen Zubereitungen sind dem Fachmann geläufig und können aus Handbüchern der Kosmetik, beispielsweise Schrader, Grundlagen und Rezepturen der Kosmetika, Hüthig Verlag, Heidelberg, 1989, ISBN 3-7785-1491-1, entnommen werden.

Bei den erfindungsgemäßen kosmetischen Mitteln kann es sich um hautkosmetische, nagelkossmetische, haarkosmetische, dermatologische, hygienische oder pharmazeutische Mittel handeln.

Vorzugsweise liegen die erfindungsgemäßen Mittel in Form eines Gels, Schaums, Sprays, einer Salbe, Creme, Emulsion, Suspension, Lotion, Milch oder Paste vor. Gewünschtenfalls können auch Liposomen oder Mikrosphären eingesetzt werden.

Die erfindungsgemäßen kosmetisch oder pharmazeutisch aktiven Mittel können zusätzlich kosmetisch und/oder dermatologisch aktive Wirkstoffe sowie Hilfsstoffe enthalten.

Vorzugsweise enthalten die erfindungsgemäßen kosmetischen Mittel wenigstens eine wie vorstehend definierte Keratin-bindende Polypeptidsequenz (i), und wenigstens einen davon verschiedenen Bestandteil, der ausgewählt ist unter kosmetisch aktiven Wirkstoffen, Emulgatoren, Tensiden, Konservierungsmitteln, Parfümölen, Verdickern, Haarpolymeren, Haar-und Hautconditionern, Pfropfpolymeren, wasserlöslichen oder dispergierbaren silikonhaltigen Polymeren, Lichtschutzmitteln, Bleichmitteln, Gelbildnern, Pflegemitteln, Färbemitteln, Tönungsmitteln, Bräunungsmitteln, Farbstoffen, Pigmenten, Konsistenzgebern, Feuchthaltemitteln, Rückfettern, Collagen, Eiweißhydrolysaten, Lipiden, Antioxidantien, Entschäumern, Antistatika, Emollienzien und Weichmachern. Die Keratin-bindenden Polypeptidwirkstoffe können auch in verkapselter Form in den kosmetischen Zubereitungen enthalten sein.

Vorteilhafterweise werden die Antioxidantien gewählt aus der Gruppe, bestehend aus Aminosäuren (z.B. Glycin, Histidin, Tyrosin, Tryptophan) und deren Derivate, Imidazole (z.B. Urocaninsäure) und deren Derivate, Peptide wie D,L-Camosin, D-Camosin, L-Camosin und deren Derivate (z.B. Anserin), Carotinoide, Carotine (z.B. β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thiorodoxin, Glutathion, Cystein, Cystin, Cystamin und deren Glycosyl-, N-Acetyl-, Methyl-, Ethyl-, Propyl-, Amyl-, Butyl-, und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximine, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und deren Derivate (z.B. Natriumascorbat, Ascorbylpalmitat, Mg-Ascorbylphosphat, Ascorbylacetat), Tocopherol und Derivate (z.B. Vitamin-E-Acetat, Tocotrienol), Vitamin A und Derivate (Vitamin-A-Palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄), Selen und dessen Derivate (z.B. Selenmethionin), Stilbene und deren Derivate (z.B. Stilbenoxid, Trans-Stilbenoxid).

Übliche Verdickungsmittel in derartigen Formulierungen sind vernetzte Polyacrylsäuren und deren Derivate, Polysaccharide und deren Derivate, wie Xanthangum, Agar-Agar, Alginate oder Tylosen, Cellulosederivate, z.B. Carboxymethylcellulose oder Hydroxycarboxymethylcellulose, Fettalkohole, Monoglyceride und Fettsäuren, Polyvinylalkohol und Polyvinylpyrrolidon. Bevorzugt werden nichtionische Verdicker eingesetzt.

Geeignete kosmetisch und/oder dermatologisch aktive Wirkstoffe sind z.B. färbende Wirkstoffe, Haut-und Haarpigmentierungsmittel, Tönungsmittel, Bräunungsmittel, Bleichmittel, Keratin-härtende Stoffe, antimikrobielle Wirkstoffe, Lichtfilterwirkstoffe, Repellentwirkstoffe, hyperemisierend wirkende Stoffe, keratolytisch und keratoplastisch wirkende Stoffe, Antischuppenwirkstoffe, Antiphlogistika, keratinisierend wirkende Stoffe, antioxidativ bzw. als Radikalfänger aktive Wirkstoffe, hautbefeuchtende oder -feuchthaltende Stoffe, rückfettende Wirkstoffe, antierythimatös oder antiallergisch aktive Wirkstoffe, verzweigte Fettsäuren wie 18-Methyleicosansäure, und Mischungen davon.

Künstlich hautbräunende Wirkstoffe, die geeignet sind, die Haut ohne natürliche oder künstliche Bestrahlung mit UV-Strahlen zu bräunen, sind z.B. Dihydroxyaceton, Alloxan und Walnussschaienextrakt. Geeignete Keratin-härtende Stoffe sind in der Regel Wirkstoffe, wie sie auch in Antitranspirantien eingesetzt werden, wie z.B. Kaliumaluminiumsulfat, Aluminiumhydroxychlorid, Aluminiumlactat, etc.

Antimikrobielle Wirkstoffe werden eingesetzt, um Mikroorganismen zu zerstören bzw. ihr Wachstum zu hemmen und dienen somit sowohl als Konservierungsmittel als auch als desodorierend wirkender Stoff, welcher die Entstehung oder die Intensität von Körpergeruch vermindert. Dazu zählen z.B. übliche, dem Fachmann bekannte Konservierungsmittel, wie p-Hydroxybenzoesäureester, Imidazolidinyl-Hamstoff, Formaldehyd, Sorbinsäure, Benzoesäure, Salicylsäure, etc. Derartige desodorierend wirkende Stoffe sind z.B. Zinkricinoleat, Triclosan, Undecylensäurealkylolamide, Citronensäuretriethylester, Chlorhexidin etc.

Als geeignete Konservierungsmittel, die mit ihrer E-Nummer nachfolgend aufgeführt sind, sind erfindungsgemäß vorteilhaft zu verwenden.

| | | | |
|---|---|---|---|
| E 200 | Sorbinsäure | E 227 | Calciumhydrogensulfit |
| E 201 | Natriumsorbat | E 228 | Kaliumhydrogensulfit) |
| E 202 | Kaliumsorbat | E 230 | Biphenyl (Diphenyl) |
| E 203 | Calciumsorbat | E 231 | Orthophenylphenol |
| E 210 | Benzoesäure | E 232 | Natriumorthophenylphenolat |
| E 211 | Natriumbenzoat | E 233 | Thiabendazol |
| E 212 | Kaliumbenzoat | E 235 | Natamycin |
| E 213 | Calciumbenzoat | E 236 | Ameisensäure |
| E 214 | p-Hydroxybenzoesäureethylester | E 237 | Natriumformiat |
| E 215 | p-Hydroxybenzoesäureethylester-Na-Salz | E 238 | Calciumformiat |
| E 216 | p-Hydroxybenzoesäure-n-propylester | E 239 | Hexamethylentetramin |
| E 217 | p-Hydroxybenzoesäure-n-propylester-Na-Salz | E 249 | Kaliumnitrit |
| E 218 | p-Hydroxybenzoesäuremethylester | E 250 | Natriumnitrit |
| E 219 | p-Hydroxybenzoesäuremethylester-Na-Salz | E 251 | Natriumnitrat |
| E 220 | Schwefeldioxid | E 252 | Kaliumnitrat |
| E 221 | Natriumsulfit | E 280 | Propionsäure |
| E 222 | Natriumyhdrogensulfit | E 281 | Natriumpropionat |
| E 223 | Natriumdisulfit | E 282 | Calciumpropionat |
| E 224 | Kaliumdisulfit | E 283 | Kaliumpropionat |
| E 226 | Calciumsulfit | E 290 | Kohlendioxid |

Ferner sind erfindungsgemäß in der Kosmetik gebräuchliche Konservierungsmittel oder Konservierungshilfsstoffe Dibromdicyanobutan (2-Brom-2-brommethylglutarodinitril), 3-lod-2-propinylbutylcarbamat, 2-Brom-2-nitro-propan-1,3-diol, Imidazolidinylharnstoff, 5-Chlor-2-methyl-4-isothiazolin-3-on, 2-Chloracetamid, Benzalkoniumchlorid, Benzylalkohol geeignet. + Formaldehydabspalter.

Ferner sind Phenylhydroxyalkylether, insbesondere die unter der Bezeichnung Phenoxyethanol bekannte Verbindung aufgrund ihrer bakteriziden und fungiziden Wirkungen auf eine Anzahl von Mikroorganismen als Konservierungsmittel geeignet.

Auch andere keimhemmende Mittel sind ebenfalls geeignet, in die erfindungsgemäßen Zubereitungen eingearbeitet zu werden. Vorteilhafte Substanzen sind zum Beispiel 2,4,4'-Trichlor-2'-hydroxydiphenylether (Irgasan), 1,6-Di-(4-chlorphenylbiguanido)-hexan (Chlorhexidin), 3,4,4'-Trichlorcarbanilid, quaternäre Ammoniumverbindungen, Nelkenöl, Minzöl, Thymianöl, Triethylcitrat, Farnesol (3,7,11-Trimethyl-2,6,10-dodecatriën-1-ol) sowie die in den Patentoffenlegungsschriften DE-37 40186, DE-39 38 140, DE-42 04 321, DE-42 29 707, DE-43 09 372, DE-44 11 664, DE-195 41 967, DE-1 95 43 695, DE-195 43 696, DE-195 47 160, DE-196 02 108, DE-196 02 110, DE-196 02 111, DE-196 31 003, DE-196 31 004 und DE-196 34 019 und den Patentschriften DE-42 29 737, DE-42 37 081, DE-43 24 219, DE-44 29 467, DE-44 23 410 und DE-195 16 705 beschriebenen Wirkstoffe bzw. Wirkstoffkombinationen. Auch Natriumhydrogencarbonat ist vorteilhaft zu verwenden. Ebenso können auch ntimikrobielle Polypeptide eingesetzt werden.

Geeignete Lichtfilterwirkstoffe sind Stoffe, die UV-Strahlen im UV-B- und/oder UV-A-Bereich absorbieren. Geeignete UV-Filter sind z.B. 2,4,6-Triaryl-1,3,5- triazine, bei denen die Arylgruppen jeweils wenigstens einen Substituenten tragen können, der vorzugsweise ausgewählt ist unter Hydroxy, Alkoxy, speziell Methoxy, Alkoxycarbonyl, speziell Methoxycarbonyl und Ethoxycarbonyl und Mischungen davon. Geeignet sind weiterhin p-Aminobenzoesäureester, Zimtsäureester, Benzophenone, Campherderivate sowie UV-Strahlen abhaltende Pigmente, wie Titandioxid, Talkum und Zinkoxid.

Als UV-Filtersubstanzen kommen beliebige UV-A- und UV-B-Filtersubstanzen in Betracht. Beispielsweise sind zu nennen:

| Nr. | Stoff | CAS-Nr. (=Säure) |
|---|---|---|
| 1 | 4-Aminobenzoesäure | 150-13-0 |
| 2 | 3-(4'-Trimethylammonium)-benzylidenboman-2-on-methylsulfat | 52793-97-2 |
| 3 | 3,3,5-Trimethyl-cyclohexyl-salicylat (Homosalatum) | 118-56-9 |
| 4 | 2-Hydroxy-4-methoxy-benzophenon (Oxybenzonum) | 131-57-7 |
| 5 | 2-Phenylbenzimidazol-5-sulfonsäure und ihre Kalium-, Natrium- u. Triethanolaminsalze | 27503-81-7 |
| 6 | 3,3'-(1,4-Phenylendimethin)-bis(7,7-dimethyl-2-oxobicyclo[2.2.1]heptan-1-methansulfonsäure) und ihre Salze | 90457-82-2 |
| 7 | 4-Bis(polyethoxy)amino-benzoesäurepolyethoxy-ethylester | 113010-52-9 |
| 8 | 4-Dimethylamino-benzoesäure-2-ethylhexylester | 21245-02-3 |
| 9 | Salicylsäure-2-ethylhexylester | 118-60-5 |
| 10 | 4-Methoxy-zimtsäure-2-isoamylester | 71617-10-2 |
| 11 | 4-Methoxy-zimtsäure-2-ethylhexylester | 5466-77-3 |
| 12 | 2-Hydroxy-4-methoxy-benzophenon-5-sulfonsäure-(Sulisobenzonum) und das Natriumsalz | 4065-45-6 |
| 13 | 3-(4'-Sulfobenzyliden)-bornan-2-on und Salze | 58030-58-6 |
| 14 | 3-Benzylidenbornan-2-on | 16087-24-8 |
| 15 | 1-(4'-Isopropylphenyl)-3-phenylpropan-1,3-dion | 63260-25-9 |
| 16 | 4-Isopropylbenzylsalicylat | 94134-93-7 |
| 17 | 3-Imidazol-4-yl-acrylsäure und ihr Ethylester | 104-98-3 |
| 18 | 2-Cyano-3,3-diphenylacrylsäureethylester | 5232-99-5 |
| 19 | 2-Cyano-3,3-diphenylacrylsäure-2'-ethylhexylester | 6197-30-4 |
| 20 | Menthyl-o-aminobenzoat oder: 5-Methyl-2-(1-methylethyl)-2-aminobenzoat | 134-09-8 |
| 21 | Glyceryl p-aminobenzoat oder: 4-Aminobenzoesäure-1-glyceryl-ester | 136-44-7 |
| 22 | 2,2'-Dihydroxy-4-methoxybenzophenon (Dioxybenzone) | 131-53-3 |
| 23 | 2-Hydroxy-4-methoxy-4-methylbenzophenon (Mexenon) | 1641-17-4 |
| 24 | Triethanolamin Salicylat | 2174-16-5 |
| 25 | Dimethoxyphenylglyoxalsäure oder: 3,4-dimethoxy-phenyl-glyoxal-saures Natrium | 4732-70-1 |
| 26 | 3-(4'Sulfobenzyliden)-bornan-2-on und seine Salze | 56039-58-8 |
| 27 | 4-tert.-Butyl-4'-methoxy-dibenzoylmethan | 70356-09-1 |
| 28 | 2,2',4,4'-Tetrahydroxybenzophenon | 131-55-5 |
| 29 | 2,2'-Methylen-bis-[6-(2H-benzotriazol-2-yl)-4-(1,1,3,3,-tetramethylbutyl)phenol] | 103597-45-1 |
| 30 | 2,2'-(1,4-Phenylen)-bis-1H-benzimidazol-4,6-disulfonsäure, Na-Salz | 180898-37-7 |
| 31 | 2,4-bis-[4-(2-Ethylhexyloxy)-2-hydroxy]phenyl-6-(4-methoxyphenyl)-(1,3,5)-triazin | 187393-00-6 |
| 32 | 3-(4-Methylbenzyliden)-campher | 36861-47-9 |
| 33 | 4-Bis(polyethoxy)paraaminobenzoesäurepolyethoxyethylester | 113010-52-9 |
| 34 | 2,4-Dihydroxybenzophenon | 131-56-6 |
| 35 | 2,2'-Dihydroxy-4,4'-dimethoxybenzophenon-5,5'-dinatriumsulfonat | 3121-60-6 |
| 36 | Benzoesäure, 2-[4-(diethylamino)-2-hydroxybenzoyl]-, hexylester | 302776-68-7 |
| 37 | 2-(2H-Benzotriazol-2-yl)-4-methyl-6-[2-methyl-3-[1,3,3,3-tetramethyl-1-[(trimethylsilyl)oxy]disiloxanyl]propyl]phenol | 155633-54-8 |
| 38 | 1,1-[(2,2'-Dimethylpropoxy)carbonyl]-4,4-diphenyl-1,3-butadien | 363602-15-7 |

Die erfindungsgemäßen kosmetischen und dermatologischen Zubereitungen können vorteilhafterweise außerdem UV-Strahlen abhaltende anorganische Pigmente auf Basis von Metalloxiden und/oder anderen in Wasser schwerlöslichen oder unlöslichen Metallverbindungen, ausgewählt aus der Gruppe der Oxide des Zinks (ZnO), Titan (TiO₂), Eisens (z.B. Fe₂O₃), Zirkoniums (ZrO₂), Siliciums (SiO₂), Mangans (z.B. MnO), Aluminiums (Al₂O₃), Cers (z.B. Ce₂O₃), Mischoxiden der entsprechenden Metalle sowie Abmischungen aus solchen Oxiden enthalten.

Die anorganischen Pigmente können dabei in gecoateter Form vorliegen, d.h. dass sie oberflächlich behandelt sind. Diese Oberflächenbehandlung kann beispielsweise darin bestehen, dass die Pigmente nach an sich bekannter Weise, wie in DE-A-33 14 742 beschrieben, mit einer dünnen hydrophoben Schicht versehen sind.

Geeignete Repellentwirkstoffe sind Verbindungen, die in der Lage sind, bestimmte Tiere, insbesondere Insekten, vom Menschen abzuhalten oder zu vertreiben. Dazu gehört z.B. 2-Ethyl-1, 3-hexandiol, N, N-Diethyl-m-toluamid etc. Geeignete hyperemisierend wirkende Stoffe, welche die Durchblutung der Haut anregen, sind z.B. ätherische Öle, wie Latschenkieferextrakt, Lavendelextrakt, Rosmarinextrakt, Wacholderbeerextrakt, Rosskastanienextrakt, Birkenblätterextrakt, Heublumenextrakt, Ethylacetat, Campher, Menthol, Pfefferminzöl, Rosmarinextrakt, Eukalyptusöl, etc. Geeignete keratolytisch und keratoplastisch wirkende Stoffe sind z.B. Salicylsäure, Kalziumthioglykolat, Thioglykolsäure und ihre Salze, Schwefel, etc. Geeignete Antischuppen-Wirkstoffe sind z.B. Schwefel, Schwefelpolyethylenglykolsorbitanmonooleat, Schwefelricinolpolyethoxylat, Zinkpyrithion, Aluminiumpyrithion, etc. Geeignete Antiphlogistika, die Hautreizungen entgegenwirken, sind z.B. Allantoin, Bisabolol, Dragosantol, Kamillenextrakt, Panthenol, etc.

Die erfindungsgemäßen kosmetischen Mittel können als kosmetischen und/oder pharmazeutischen Wirkstoff (wie auch gegebenenfalls als Hilfsstoff) wenigstens ein kosmetisch oder pharmazeutisch akzeptables Polymer enthalten, das sich von den Polymeren unterscheidet, die den erfindungsgemäß eingesetzten Polyelektrolytkomplex bilden. Dazu zählen ganz allgemein kationische, amphotere und neutrale Polymere.

Geeignete Polymere sind z.B. kationische Polymere mit der Bezeichnung Polyquaternium nach INCl, z.B. Copolymere aus Vinylpyrrolidon/N-Vinylimidazoliumsalzen (Luviquat FC, Luviquat HM, Luviquat MS, Luviquat&commat, Care), Copolymere aus N-Vinylpyrrolidon/Dimethylaminoethylmethacrylat, quatemisiert mit Diethylsulfat (Luviquat PQ 11), Copolymere aus N-Vinylcaprolactam/N-Vinylpyrrolidon/N-Vinylimidazoliumsalzen (Luviquat E Hold), kationische Cellulosederivate (Polyquaternium-4 und -10), Acrylamidocopolymere (Polyquaternium-7) und Chitosan.

Geeignete kationische (quaternisierte) Polymere sind auch Merquat (Polymer auf Basis von Dimethyldiallylammoniumchlorid), Gafquat (quaternäre Polymere, die durch Reaktion von Polyvinylpyrrolidon mit quaternären Ammoniumverbindungen entstehen), Polymer JR (Hydroxyethylcellulose mit kationischen Gruppen) und kationische Polymere auf pflanzlicher Basis, z.B. Guarpolymere, wie die Jaguar-Marken der Firma Rhodia.

Weitere geeignete Polymere sind auch neutrale Polymere, wie Polyvinylpyrrolidone, Copolymere aus N-Vinylpyrrolidon und Vinylacetat und/oder Vinylpropionat, Polysiloxane, Polyvinylcaprolactam und andere Copolymere mit N-Vinylpyrrolidon, Polyethylenimine und deren Salze, Polyvinylamine und deren Salze, Cellulosederivate, Polyasparaginsäuresalze und Derivate. Dazu zählt beispielsweise Luviflex 0 Swing (teilverseiftes Copolymerisat von Polyvinylacetat und Polyethylenglykol, Firma BASF).

Geeignete Polymere sind auch nichtionische, wasserlösliche bzw. wasserdispergierbare Polymere oder Oligomere, wie Polyvinylcaprolactam, z.B. Luviskol 0 Plus (BASF), oder Polyvinylpyrrolidon und deren Copolymere, insbesondere mit Vinylestern, wie Vinylacetat, z.B. Luviskol 0 VA 37 (BASF), Polyamide, z.B. auf Basis von Itaconsäure und aliphatischen Diaminen, wie sie z.B. in der DE-A-43 33 238 beschrieben sind.

Geeignete Polymere sind auch amphotere oder zwitterionische Polymere, wie die unter den Bezeichnungen Amphomer (National Starch) erhältlichen Octylacrylamid / Methylmethacrylat / tert.-Butylaminoethylmethacrylat-Hydroxypropylmethacrylat-Copolymere sowie zwitterionische Polymere, wie sie beispielsweise in den deutschen Patentanmeldungen DE39 29 973, DE 21 50 557, DE28 17 369 und DE 3708 451 offenbart sind. Acrylamidopropyltrimethylammoniumchlorid/Acrylsäure-bzw. -Methacrylsäure-Copolymerisate und deren Alkali-und Ammoniumsalze sind bevorzugte zwitterionische Polymere. Weiterhin geeignete zwitterionische Polymere sind Methacroylethylbetain/Methacrylat-Copolymere, die unter der Bezeichnung Amersette (AMERCHOL) im Handel erhältlich sind, und Copolymere aus Hydroxyethylmethacrylat, Methylmethacrylat, N, N-Dimethylaminoethylmethacrylat und Acrylsäure (Jordapon (D)).

Geeignete Polymere sind auch nichtionische, siloxanhaltige, wasserlösliche oder - dispergierbare Polymere, z.B. Polyethersiloxane, wie Tegopren 0 (Firma Goldschmidt) oder Besi&commat (Firma Wacker).

Die Formulierungsgrundlage erfindungsgemäßer pharmazeutischer Mittel enthält bevorzugt pharmazeutisch akzeptable Hilfsstoffe. Pharmazeutisch akzeptabel sind die im Bereich der Pharmazie, der Lebensmitteltechnologie und angrenzenden Gebieten bekanntermassen verwendbaren Hilfsstoffe, insbesondere die in einschlägigen Arzneibüchern (z.B. DAB Ph. Eur. BP NF) gelisteten sowie andere Hilfsstoffe, deren Eigenschaften einer physiologischen Anwendung nicht entgegenstehen.

Geeignete Hilfsstoffe können sein: Gleitmittel, Netzmittel, emulgierende und suspendierende Mittel, konservierende Mittel, Antioxidantien, Antireizstoffe, Chelatbildner, Emulsionsstabilisatoren, Filmbildner, Gelbildner, Geruchsmaskierungsmittel, Harze, Hydrokolloide, Lösemittel, Lösungsvermittler, Neutralisierungsmittel, Permeationsbeschleuniger, Pigmente, quaternäre Ammoniumverbindungen, Rückfettungs- und Überfettungsmittel, Salben-, Creme- oder Öl-Grundstoffe, Siliconderivate, Stabilisatoren, Sterilantien, Treibmittel, Trocknungsmittel, Trübungsmittel, Verdickungsmittel, Wachse, Weichmacher, Weissöl. Eine diesbezügliche Ausgestaltung beruht auf fachmännischem Wissen, wie sie beispielsweise in Fiedler, H. P. Lexikon der Hilfsstoffe für Pharmazie, Kosmetik und angrenzende Gebiete, 4. Aufl., Aulendorf: ECV-Editio-Kantor-Verlag, 1996, dargestellt sind.

Zur Herstellung der erfindungsgemäßen dermatologischen Mittel können die Wirkstoffe mit einem geeigneten Hilfsstoff (Exzipient) vermischt oder verdünnt werden. Exzipienten können feste, halb feste oder flüssige Materialien sein, die als Vehikel, Träger oder Medium für den Wirkstoff dienen können. Die Zumischung weiterer Hilfsstoffe erfolgt gewünschtenfalls in der dem Fachmann bekannten Weise. Weiterhin sind die Polymere und Dispersionen geeignet als Hilfsmittel in der Pharmazie, bevorzugt als oder in Beschichtungsmittel(n) oder Bindemittel(n) für feste Arzneiformen. Sie können auch in Cremes und als Tablettenüberzugsmittel und Tablettenbindemittel verwendet werden.

Nach einer bevorzugten Ausführungsform handelt es sich bei den erfindungsgemäßen Mitteln um ein Hautreinigungsmittel.

Bevorzugte Hautreinigungsmittel sind Seifen von flüssiger bis gelförmiger Konsistenz, wie Transparentseifen, Luxusseifen, Deoseifen, Cremeseifen, Babyseifen, Hautschutzseifen, Abrasiveseifen und Syndets, pasteuse Seifen, Schmierseifen und Waschpasten, Peelingseifen, Feuchtigkeitstücher, flüssige Wasch-, Dusch- und Badepräparate, wie Waschlotionen, Duschbäder und -gele, Schaumbäder, Ölbäder und Scrub-Präparate, Rasierschäume, -lotionen und -cremes.

Nach einer weiteren bevorzugten Ausführungsform handelt es sich bei den erfindungsgemäßen Mitteln um kosmetische Mittel zur Pflege und zum Schutz der Haut und Haar, Nagelpflegemittel oder Zubereitungen für die dekorative Kosmetik.

Geeignete hautkosmetische Mittel sind z.B. Gesichtswässer, Gesichtsmasken, Deodorantien und andere kosmetische Lotionen. Mittel für die Verwendung in der dekorativen Kosmetik umfassen beispielsweise Abdeckstifte, Theaterfarben, Mascara und Lidschatten, Lippenstifte, Kajalstifte, Eyeliner, Rouges, Puder und Augenbrauenstifte.

Ausserdem können die Polypeptidsequenzen (i) verwendet werden in Nose-Strips zur Porenreinigung, in Antiaknemitteln, Repellents, Rasiermitteln, After- und Pre Shave Pflegemittel, After Sun Pflegemittel, Haarentfernungsmitteln, Haarfärbenitteln, Intimpflegemitteln, Fusspflegemitteln sowie in der Babypflege.

Bei den erfindungsgemäßen Hautpflegemitteln handelt es sich insbesondere um W/O- oder O/W-Hautcremes, Tag- und Nachtcremes, Augencremes, Gesichtscremes, Antifaltencremes, Sonnenschutzcremes, Feuchthaltecremes, Bleichcremes, Selbstbräunungscremes, Vitamincremes, Hautlotionen, Pflegelotionen und Feuchthaltelotionen.

Hautkosmetische und dermatologische Mittel auf Basis der zuvor beschriebenen Polyelektrolytkomplexe zeigen vorteilhafte Wirkungen. Die Polymere können unter anderem zur Feuchthaltung und Konditionierung der Haut und zur Verbesserung des Hautgefühls beitragen. Die Polymere können auch als Verdicker in den Formulierungen wirken. Durch Zusatz der erfindungsgemäßen Polymere kann in bestimmten Formulierungen eine erhebliche Verbesserung der Hautverträglichkeit erreicht werden.

Hautkosmetische und dermatologische Mittel enthalten vorzugsweise wenigstens eine Polypeptidsequenz (i) in einem Anteil von etwa 0,001 bis 30 Gew.-%, vorzugsweise 0,01 bis 20 Gew.-%, ganz besonders bevorzugt 0,1 bis 12 Gew.-%, bezogen auf das Gesamtgewicht des Mittels.

Besonders Lichtschutzmittel auf Basis der Polypeptidsequenzen (i) besitzen die Eigenschaft, die Verweilzeit der UV-absorbierenden Inhaltsstoffe im Vergleich zu gängigen Hilfsmitteln wie Polyvinylpyrrolidon zu erhöhen.

Je nach Anwendungsgebiet können die erfindungsgemäßen Mittel in einer zur Hautpflege geeigneten Form, wie z.B. als Creme, Schaum, Gel, Stift, Mousse, Milch, Spray (Pumpspray oder treibmittelhaltiger Spray) oder Lotion appliziert werden.

Die hautkosmetischen Zubereitungen können neben den Polypeptidsequenzen (i) und geeigneten Trägern noch weitere in der Hautkosmetik übliche Wirkstoffe und Hilfsstoffe, wie zuvor beschrieben, enthalten. Dazu zählen vorzugsweise Emulgatoren, Konservierungsmittel, Parfümöle, kosmetische Wirkstoffe wie Phytantriol, Vitamin A, E und C, Retinol, Bisabolol, Panthenol, Lichtschutzmittel, Bleichmittel, Färbemittel, Tönungsmittel, Bräunungsmittel, Collagen, Eiweisshydrolysate, Stabilisatoren, pH-Wert-Regulatoren, Farbstoffe, Salze, Verdicker, Gelbildner, Konsistenzgeber, Silicone, Feuchthaltemittel, Rückfetter und weitere übliche Additive.

Bevorzugte Öl- und Fettkomponenten der hautkosmetischen und dermatologischen Mittel sind die zuvor genannten mineralischen und synthetischen Öle, wie z.B. Paraffine, Siliconöle und aliphatische Kohlenwasserstoffe mit mehr als 8 Kohlenstoffatomen, tierische und pflanzliche Öle, wie z.B. Sonnenblumenöl, Kokosöl, Avocadoöl, Olivenöl, Lanolin, oder Wachse, Fettsäuren, Fettsäureester, wie z.B. Triglyceride von C6-C30-Fettsäuren, Wachsester, wie z.B. Jojobaöl, Fettalkohole, Vaseline, hydriertes Lanolin und acetyliertes Lanolin sowie Mischungen davon.

Man kann die erfindungsgemäßen Polypeptidsequenzen (i) auch mit herkömmlichen Polymeren abmischen, falls spezielle Eigenschaften eingestellt werden sollen.

Zur Einstellung bestimmter Eigenschaften wie z.B. Verbesserung des Anfassgefühls, des Spreitverhaltens, der Wasserresistenz und/oder der Bindung von Wirk- und Hilfsstoffen, wie Pigmenten, können die hautkosmetischen und dermatologischen Zubereitungen zusätzlich auch konditionierende Substanzen auf Basis von Siliconverbindungen enthalten.

Geeignete Siliconverbindungen sind beispielsweise Polyalkylsiloxane, Polyarylsiloxane, Polyarylalkylsiloxane, Polyethersiloxane oder Siliconharze.

Die Herstellung der kosmetischen oder dermatologischen Zubereitungen erfolgt nach üblichen, dem Fachmann bekannten Verfahren.

Bevorzugt liegen die kosmetischen und dermatologischen Mittel in Form von Emulsionen insbesondere als Wasser-in-Öl (W/O)- oder Öl-in-Wasser (O/W)-Emulsionen vor.

Es ist aber auch möglich, andere Formulierungsarten zu wählen, beispielsweise, Gele, Öle, Öleogele, multiple Emulsionen, beispielsweise in Form von W/O/W- oder O/W/O-Emulsionen, wasserfreie Salben bzw. Salbengrundlagen, usw. Auch emulgatorfreie Formulierungen wie Hydrodispersionen, Hydrogele oder eine Pickering-Emulsion sind vorteilhafte Ausführungsformen.

Die Herstellung von Emulsionen erfolgt nach bekannten Methoden. Die Emulsionen enthalten neben wenigstens einer Polypeptidsequenz (i) in der Regel übliche Bestandteile, wie Fettalkohole, Fettsäureester und insbesondere Fettsäuretriglyceride, Fettsäuren, Lanolin und Derivate davon, natürliche oder synthetische Öle oder Wachse und Emulgatoren in Anwesenheit von Wasser. Die Auswahl der Emulsionstyp-spezifischen Zusätze und die Herstellung geeigneter Emulsionen ist beispielsweise beschrieben in Schrader, Grundlagen und Rezepturen der Kosmetika, Hüthig Buch Verlag, Heidelberg, 2. Auflage, 1989, dritter Teil, worauf hiermit ausdrücklich Bezug genommen wird.

Eine geeignete Emulsion als W/O-Emulsion, z.B. für eine Hautcreme etc., enthält im Allgemeinen eine wässrige Phase, die mittels eines geeigneten Emulgatorsystems in einer Öl- oder Fettphase emulgiert ist. Zur Bereitstellung der wässrigen Phase kann ein Polyelektrolytkomplex eingesetzt werden.

Bevorzugte Fettkomponenten, welche in der Fettphase der Emulsionen enthalten sein können, sind: Kohlenwasserstofföle, wie Paraffinöl, Purcellinöl, Perhydrosqualen und Lösungen mikrokristalliner Wachse in diesen Ölen; tierische oder pflanzliche Öle, wie Süssmandelöl, Avocadoöl, Calophylumöl, Lanolin und Derivate davon, Ricinusöl, Sesamöl, Olivenöl, Jojobaöl, Karite-Öl, Hoplostethus-Öl, mineralische Öle, deren Destillationsbeginn unter Atmosphärendruck bei ca. 250°C und deren Destillationsendpunkt bei 410°C liegt, wie z.B. Vaselinöl, Ester gesättigter oder ungesättigter Fettsäuren, wie Alkylmyristate, z.B. i-Propyl-, Butyl- oder Cetylmyristat, Hexadecylstearat, Ethyl- oder i-Propylpalmitat, Octan- oder Decansäuretriglyceride und Cetylricinoleat.

Die Fettphase kann auch in anderen Ölen lösliche Siliconöle, wie Dimethylpolysiloxan, Methylphenylpolysiloxan und das Siliconglykol-Copolymer, Fettsäuren und Fettalkohole enthalten.

Neben den Polypeptidsequenzen (i) können auch Wachse verwendet werden, wie z.B. Carnaubawachs, Candilillawachs, Bienenwachs, mikrokristallines Wachs, Ozokeritwachs und Ca-, Mg- und Al-Oleate, -Myristate, -Linoleate und -Stearate.

Weiterhin kann eine erfindungsgemäße Emulsion als O/W-Emulsion vorliegen. Eine derartige Emulsion enthält üblicherweise eine Ölphase, Emulgatoren, die die Ölphase in der Wasserphase stabilisieren, und eine wässrige Phase, die üblicherweise verdickt vorliegt. Als Emulgatoren kommen vorzugsweise O/W-Emulgatoren, wie Polyglycerinester, Sorbitanester oder teilveresterte Glyceride, in Betracht.

Nach einer weiteren bevorzugten Ausführungsform handelt es sich bei den erfindungsgemäßen Mitteln um ein Duschgel, eine Shampoo-Formulierung oder ein Badepräparat.

Solche Formulierungen enthalten wenigstens einen Polypeptidsequenz (i) sowie üblicherweise anionische Tenside als Basistenside und amphotere und/oder nichtionische Tenside als Cofenside. Weitere geeignete Wirkstoffe und/oder Hilfsstoffe sind im allgemeinen ausgewählt unter Lipiden, Parfümölen, Farbstoffen, organischen Säuren, Konservierungsstoffen und Antioxidantien sowie Verdickern/Gelbildnern, Hautkonditioniermitteln und Feuchthaltemitteln.

Diese Formulierungen enthalten vorzugsweise 2 bis 50 Gew.-%, bevorzugt 5 bis 40 Gew.-%, besonders bevorzugt 8 bis 30 Gew.-% Tenside, bezogen auf das Gesamtgewicht der Formulierung.

In den Wasch-, Dusch- und Badepräparaten können alle in Körperreinigungsmitteln üblicherweise eingesetzten anionische, neutrale, amphotere oder kationische Tenside verwendet werden.

Geeignete anionische Tenside sind beispielsweise Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisothionate, Alkylphosphate, Alkyletherphosphate, Alkylethercarboxylate, Alpha-Olefinsulfonate, insbesondere die Alkali- und Erdalkalimetallsalze, z.B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid- oder Propylenoxideinheiten, bevorzugt 1 bis 3 Ethylenoxideinheiten im Molekül aufweisen.

Dazu zählen z.B. Natriumlaurylsulfat, Ammoniumtaurytsulfat, Natriumlaurylethersulfat, Ammoniumlaurylethersulfat, Natriumlaurylsarkosinat, Natriumoleylsuccinat, Ammoniumlaurylsulfosuccinat, Natriumdodecylbenzolsulfonat, Triethanolamindodecylbenzolsulfonat.

Geeignete amphotere Tenside sind z.B. Alkylbetaine, Alkylamidopropylbetaine, Alkylsulfobetaine, Alkylglycinate, Alkylcarboxyglycinate, Alkylamphoacetate oder - propionate, Alkylamphodiacetate oder -dipropionate.

Beispielsweise können Cocodimethylsulfopropylbetain, Laurylbetain, Cocamidopropylbetain oder Natriumcocamphopropionat eingesetzt werden.

Als nichtionische Tenside sind beispielsweise geeignet die Umsetzungsprodukte von aliphatischen Alkoholen oder Alkylphenolen mit 6 bis 20 C-Atomen in der Alkylkette, die linear oder verzweigt sein kann, mit Ethylenoxid und/oder Propylenoxid. Die Menge Alkylenoxid beträgt ca. 6 bis 60 Mole auf ein Mol Alkohol. Ferner sind Alkylaminoxide, Mono-oder Dialkylalkanolamide, Fettsäureester von Polyethylenglykolen, ethoxylierte Fettsäureamide, Alkylpolyglycoside oder Sorbitanetherester geeignet.

Ausserdem können die Wasch-, Dusch- und Badepräparate übliche kationische Tenside enthalten, wie z.B. quaternäre Ammoniumverbindungen, beispielsweise Cetyltrimethylammoniumchlorid.

Weiterhin können die Duschgel-/Shampoo-Formulierungen Verdicker, wie z.B. Kochsalz, PEG-55, Propylenglykol-Oleat, PEG-120-Methylglucosedioleat und andere, sowie Konservierungsmittel, weitere Wirk- und Hilfsstoffe und Wasser enthalten.

Nach einer weiteren bevorzugten Ausführungsform handelt es sich bei den erfindungsgemäßen Mitteln um ein Haarbehandlungsmittel.

Erfindungsgemäße Haarbehandlungsmittel enthalten vorzugsweise wenigstens eine Polypeptidsequenz (i) in einer Menge im Bereich von etwa 0,01 bis 30 Gew.-%, bevorzugt 0,5 bis 20 Gew.-%, bezogen auf das Gesamtgewicht des Mittels.

Vorzugsweise liegen die erfindungsgemäßen Haarbehandlungsmittel in Form eines Schaumfestigers, Haarmousses, Haargels, Shampoos, Haarsprays, Haarschaums, Spitzenfluids, Egalisierungsmittels für Dauerwellen, Haarfärbe- und -bleichmittels oder "Hot-Oil-Treatments" vor. Je nach Anwendungsgebiet können die haarkosmetischen Zubereitungen als (Aerosol-) Spray, (Aerosol-) Schaum, Gel, Gelspray, Creme, Lotion oder Wachs appliziert werden. Haarsprays umfassen dabei sowohl Aerosolsprays als auch Pumpsprays ohne Treibgas. Haarschäume umfassen sowohl Aerosolschäume wie auch Pumpschäume ohne Treibgas. Haarsprays und Haarschäume umfassen vorzugsweise überwiegend oder ausschliesslich wasserlösliche oder wasserdispergierbare Komponenten. Sind die in den erfindungsgemäßen Haarsprays und Haarschäumen eingesetzten Verbindungen wasserdispergierbar, können sie in Form von wässrigen Mikrodispersionen mit Teilchendurchmessern von üblicherweise 1 bis 350 nm, bevorzugt 1 bis 250 nm, zur Anwendung gebracht werden. Die Feststoffgehalte dieser Präparate liegen dabei üblicherweise in einem Bereich von etwa 0,5 bis 20 Gew.-%. Diese Mikrodispersionen benötigen in der Regel keine Emulgatoren oder Tenside zu ihrer Stabilisierung.

Die erfindungsgemäßen haarkosmetischen Formulierungen enthalten in einer bevorzugten Ausführungsform a) 0,01 bis 30 Gew.-% wenigstens einer Polypeptidsequenz (i) b) 20 bis 99,95 Gew.-% Wasser und/oder Alkohol, c) 0 bis 50 Gew.-% wenigstens eines Treibgases, d) 0 bis 5 Gew.-% wenigstens eines Emulgators, e) 0 bis 3 Gew.-% wenigstens eines Verdickers, sowie bis zu 25 Gew.-% weitere Bestandteile.

Unter Alkohol sind alle in der Kosmetik üblichen Alkohole zu verstehen, z.B. Ethanol, Isopropanol, n-Propanol.

Unter weiteren Bestandteilen sind die in der Kosmetik üblichen Zusätze zu verstehen, beispielsweise Treibmittel, Entschäumer, grenzflächenaktive Verbindungen, d.h. Tenside, Emulgatoren, Schaumbildner und Solubilisatoren. Die eingesetzten grenzflächenaktiven Verbindungen können anionisch, kationisch, amphoter oder neutral sein. Weitere übliche Bestandteile können ferner sein z.B. Konservierungsmittel, Parfümöle, Trübungsmittel, Wirkstoffe, UV-Filter, Pflegestoffe wie Panthenol, Collagen, Vitamine, Eiweisshydrolysate, Alpha- und Beta-Hydroxycarbonsäuren, Stabilisatoren, pH-Wert-Regulatoren, Farbstoffe, Viskositätsregulierer, Gelbildner, Salze, Feuchthaltemittel, Rückfetter, Komplexbildner und weitere übliche Additive.

Weiterhin zählen hierzu alle in der Kosmetik bekannten Styling- und Conditioner-Polymere, die in Kombination mit den erfindungsgemäßen Polypeptidsequenzen (i) eingesetzt werden können, falls ganz spezielle Eigenschaften eingestellt werden sollen.

Als herkömmliche Haarkosmetik-Polymere eignen sich beispielsweise die zuvor genannten kationischen, anionischen, neutralen, nichtionischen und amphoteren Polymere, auf die hier Bezug genommen wird.

Zur Einstellung bestimmter Eigenschaften können die Zubereitungen zusätzlich auch konditionierende Substanzen auf Basis von Silikonverbindungen enthalten. Geeignete Silikonverbindungen sind beispielsweise Polyalkylsiloxane, Polyarylsiloxane, Polyarylalkylsiloxane, Polyethersiloxane, Silikonharze oder Dimethicon Copolyole (CTFA) und aminofunktionelle Silikonverbindungen wie Amodimethicone (CTFA).

Die erfindungsgemäßen Polymerisate eignen sich insbesondere als Festigungsmittel in Haarstyling-Zubereitungen, insbesondere Haarsprays (Aerosolsprays und Pumpsprays ohne Treibgas) und Haarschäume (Aerosolschäume und Pumpschäume ohne Treibgas).

In einer bevorzugten Ausführungsform enthalten Spray-Zubereitungen a) 0,01 bis 30 Gew.-% wenigstens einer Polypeptidsequenz (i), b) 20 bis 99,9 Gew.-% Wasser und/oder Alkohol, c) 0 bis 70 Gew.-% wenigstens eines Treibmittel, d) 0 bis 20 Gew.-% weitere Bestandteile.

Treibmittel sind die für Haarsprays oder Aerosolschäume üblich verwendeten Treibmittel. Bevorzugt sind Gemische aus Propan/Butan, Pentan, Dimethylether, 1,1-Difluorethan (HFC-152 a), Kohlendioxid, Stickstoff oder Druckluft.

Eine erfindungsgemäß bevorzugte Formulierung für Aerosolhaarschäume enthält a) 0,01 bis 30 Gew.-% wenigstens einer Polypeptidsequenz (i), b) 55 bis 99,8 Gew.-% Wasser und/oder Alkohol, c) 5 bis 20 Gew.-% eines Treibmittel, d) 0,1 bis 5 Gew.-% eines Emulgators, e) 0 bis 10 Gew.-% weitere Bestandteile.

Als Emulgatoren können alle in Haarschäumen üblicherweise eingesetzten Emulgatoren verwendet werden. Geeignete Emulgatoren können nichtionisch, kationisch bzw. anionisch oder amphoter sein.

Beispiele für nichtionische Emulgatoren (INCl-Nomenklatur) sind Laurethe, z.B. Laureth-4 ; Cetethe, z.B. Cetheth-1, Polyethylenglycolcetylether, Cetearethe, z.B. Cetheareth-25, Polyglycolfettsäureglyceride, hydroxyliertes Lecithin, Lactylester von Fettsäuren, Alkylpolyglycoside.

Beispiele für kationische Emulgatoren sind Cetyldimethyl-2-hydroxyethylammonium-dihydrogenphosphat, Cetyltrimoniumchlorid, Cetyltrimmoniumbromid, Cocotrimoniummethylsulfat, Quatemium-1 bis x (INCl).

Anionische Emulgatoren können beispielsweise ausgewählt werden aus der Gruppe der Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisethionate, Alkylphosphate, Alkyletherphosphate, Alkylethercarboxylate, Alpha-Olefinsulfonate, insbesondere die Alkali- und Erdalkalimetallsalze, z.B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid oder Propylenoxid-Einheiten, bevorzugt 1 bis 3 Ethylenoxid-Einheiten im Molekül aufweisen.

Eine erfindungsgemäß für Styling-Gele geeignete Zubereitung kann beispielsweise wie folgt zusammengesetzt sein: a) 0,01 bis 30 Gew.-% wenigstens eines Polypeptidsequenz (i), b) 80 bis 99,85 Gew.-% Wasser und/oder Alkohol, c) 0 bis 3 Gew.-%, bevorzugt 0,05 bis 2 Gew.-%, eines Gelbildners, d) 0 bis 20 Gew.-% weitere Bestandteile.

Im allgemeinen wirken die erfindungsgemäß eingesetzten Polypeptidsequenzen (i) bereits "selbstverdickend", so dass in vielen Fällen bei der Herstellung von Gelen auf den Einsatz von Gelbildnern verzichtet werden kann. Ihr Einsatz kann jedoch von Vorteil sein, um spezielle rheologische oder andere anwendungstechnische Eigenschaften der Gele einzustellen. Als Gelbildner können alle in der Kosmetik üblichen Gelbildner eingesetzt werden. Hierzu zählen leicht vernetzte Polyacrylsäure, beispielsweise Carbomer (INCl), Cellulosederivate, z.B. Hydroxypropylcellulose, Hydroxyethylcellulose, kationisch modifizierte Cellulosen, Polysaccharide, z.B. Xanthangummi, Capryl/Caprin-Triglycerid, Natriumacrylat-Copolymere, Polyquatemium-32 (und) Paraffinum Liquidum (INCl), Natriumacrylat-Copolymere (und) Paraffinum Liquidum (und) PPG-1 Trideceth-6, Acrylamidopropyltrimoniumchlorid / Acrylamid-Copolymere, Steareth-10-Allylether, Acrylat-Copolymere, Polyquaternium-37 (und) Paraffinum Liquidum (und) PPG-1 Trideceth-6, Polyquatemium 37 (und) Propylenglycoldicapratdicaprylat (und) PPG-1 Trideceth-6, Polyquaternium-7, Polyquatemium-44.

Die erfindungsgemäßen Polypeptidsequenzen (i) können in kosmetischen Zubereitungen als Konditioniermittel eingesetzt werden.

Eine die erfindungsgemäßen Polypeptidsequenzen (i) enthaltende Zubereitung kann bevorzugt in Shampooformulierungen als Festigungs- und/oder Konditioniermittel eingesetzt werden. Bevorzugte Shampooformulierungen enthalten a) 0,01 bis 30 Gew.-% wenigstens einer Polypeptidsequenz (i), b) 25 bis 94,95 Gew.-% Wasser, c) 5 bis 50 Gew.-% Tenside, c) 0 bis 5 Gew.-% eines weiteren Konditioniermittels, d) 0 bis 10 Gew.-% weitere kosmetische Bestandteile.

In den Shampooformulierungen können alle in Shampoos üblicherweise eingesetzte anionische, neutrale, amphotere oder kationische Tenside verwendet werden.

Geeignete anionische Tenside sind beispielsweise Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisothionate, Alkylphosphate, Alkyletherphosphate, Alkylethercarboxylate, Alpha-Olefinsulfonate, insbesondere die Alkali- und Erdalkalimetallsalze, z.B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid- oder Propylenoxid-Einheiten, bevorzugt 1 bis 3 Ethylenoxid-Einheiten im Molekül aufweisen.

Geeignet sind zum Beispiel Natriumlaurylsulfat, Ammoniumlaurysulfat, Natriumlaurylethersulfat, Ammoniumlaurylethersulfat, Natriumlauroylsarkosinat, Natriumoleylsuccinat, Ammoniumlaurylsulfosuccinat, Natriumdodecylbenzolsulfonat, Triethanolamindodecylbenzolsulfonat.

Geeignete amphotere Tenside sind zum Beispiel Alkylbetaine, Alkylamidopropylbetaine, Alkylsulfobetaine, Alkylglycinate, Alkylcarboxyglycinate, Alkylamphoacetate oder - propionate, Alkylamphodiacetate oder -dipropionate.

Beispielsweise können Cocodimethylsulfopropylbetain, Laurylbetain, Cocamidopropylbetain oder Natriumcocamphopropionat eingesetzt werden.

Als nichtionische Tenside sind beispielsweise geeignet die Umsetzungsprodukte von aliphatischen Alkoholen oder Alkylphenolen mit 6 bis 20 C-Atomen in der Alkylkette, die linear oder verzweigt sein kann, mit Ethylenoxid und/oder Propylenoxid. Die Menge Alkylenoxid beträgt ca. 6 bis 60 Mole auf ein Mol Alkohol. Ferner sind Alkylaminoxide, Mono- oder Dialkylalkanolamide, Fettsäureester von Polyethylenglykolen, Alkylpolyglykoside oder Sorbitanetherester geeignet.

Ausserdem können die Shampooformulierungen übliche kationische Tenside enthalten, wie z.B. quaternäre Ammoniumverbindungen, beispielsweise Cetyltrimethylammoniumchlorid.

In den Shampooformulierungen können zur Erzielung bestimmter Effekte übliche Konditioniermittel in Kombination mit den Polypeptidsequenzen (i) eingesetzt werden.

Hierzu zählen beispielsweise die zuvor genannten kationischen Polymere mit der Bezeichnung Polyquaternium nach INCl, insbesondere Copolymere aus Vinylpyrrolidon/ N-Vinylimidazoliumsalzen (Luviquat FC, Luviquat&commat, HM, Luviquat MS, Luviquat Care), Copolymere aus N-VinylpyrrolidonlDimethylaminoethylmethacrylat, quatemisiert mit Diethylsulfat (Luviquat D PQ 11), Copolymere aus N-Vinylcaprolactam/N-Vinylpyrrolidon/N-Vinylimidazoliumsalzen (Luviquat D Hold), kationische Cellulosederivate (Polyquaternium-4 und -10), Acrylamidcopolymere (Polyquaternium-7). Ferner können Eiweißhydrolysate verwendet werden, sowie konditionierende Substanzen auf Basis von Silikonverbindungen, beispielsweise Polyalkylsiloxane, Polyarylsiloxane, Polyarylalkylsiloxane, Polyethersiloxane oder Silikonharze. Weitere geeignete Silikonverbindungen sind Dimethicon Copolyole (CTFA) und aminofunktionelle Silikonverbindungen wie Amodimethicone (CTFA). Ferner können kationische Guarderivate wie Guarhydroxypropyltrimoniumchlorid (INCl) verwendet werden.

Ein weiterer Gegenstand der Erfindung sind Keratin-bindende Effektormoleküle bestehend aus
(i) mindestens einer Polypeptidsequenz (i), die eine Bindungsaffinität zu einem Keratin besitzt,
(ii) einem Effektormolekül, das natürlicherweise nicht mit der Polypeptidsequenz (i) verknüpft ist.

### Geeignete Polypeptidsequenzen (i) sind oben beschrieben

Eine besonders vorteilhafte Ausgestaltung sind Keratin-bindende Effektormoleküle wobei die Polypeptidsequenz (i) mindestens eine der folgenden Polypeptidsequenzen umfasst,
i. die Polypeptidsequenz (Domäne B )
ii. die Polypeptidsequenz (Domäne C)
iii. eine Polypeptidsequenz, die gegenüber (a) in bis zu 70% der Aminosäuren verändert ist,
iv. eine Polypeptidsequenz, die gegenüber (b) in bis zu 70% der Aminosäuren verändert ist,
mit der Massgabe, dass die Keratin-Bindung der Polypeptidsequenz (c) oder (d) mindestens 10 % des Wertes beträgt, den die Polypeptidsequenz (a) oder (b) aufweist, gemessen in dem Test gemäß Beispiel 9 oder 10. Mit Domäne B bzw. C sind hier die oben und auf den folgenden Seiten beschriebenen Keratinbindedomänen des humanen Desmoplakins (SEQ ID NO:1) gemeint. Mit Veränderung von Aminosäuren sind hiermit Aminosäuresubstitutionen, -insertionen und -deletionen oder beliebige Kombinationen aus diesen drei Möglichkeiten gemeint.

Bevorzugt werden Polypeptidsequenzen (i) verwendet, die für den gewünschten Organismus eine hochspezifische Affinität besitzen. Für Anwendungen in der Hautkosmetik werden demzufolge Polypeptidsequenzen (i) bevorzugt eingesetzt, die zu dem humanen Hautkeratin eine besonders hohe Affinität haben. Für Anwendungen in der Haarkosmetik werden solche Polypeptidsequenzen bevorzugt, die zu humanem Haarkeratin eine besonders hohe Affinität haben.

Für Anwendungen auf dem Haustiergebiet werden entsprechend solche Polypeptidsequenzen (i) bevorzugt, die zu dem entsprechenden Keratin, beispielsweise Hundekeratin oder Katzenkeratin eine besonders hohe Affinität besitzen.

Es können aber auch mehr als eine Polypeptidsequenz (i) in dem erfindungsgemässen Effektormolekül verwendet werden, beispielsweise eine Sequenz (i),die eine hohe Bindungsaffinität zu humanem Hautkeratin besitzt, in Verbindung mit einer Sequenz (i); die eine hohe Affinität zu humanem Haarkeratin besitzt. Es können auch mehrere Kopien der gleichen Polypeptidsequenz (i) hintereinander geschaltet werden, um beispielsweise eine höhere Bindung zu erzielen.

Geeignete Keratin-bindende Polypeptidsequenzen (i) sind bekannt. Beispielsweise enthalten Desmoplakine und Plectine Keratin-bindende Domänen. (Fontao L, Favre B, Riou S, Geerts D, Jaunin F, Saurat JH, Green KJ, Sonnenberg A, Borradori L., Interaction of the bullous pemphigoid antigen 1 (BP230) and desmoplakin with intermediate filaments is mediated by distinct sequences within their COOH terminus., Mol Biol Cell. 2003 May;14(5):1978-92. Epub 2003 Jan 26; Hopkinson SB, Jones JC., The N terminus of the transmembrane protein BP180 interacts with the N-terminal domain of BP230, thereby mediating keratin cytoskeleton anchorage to the cell surface at the site of the hemidesmosome, Mol Biol Cell. 2000 Jan;11(1):277-86).

Durch Alignments solcher bekannter Proteinsequenzen, beispielsweise mit einem Computerprogramm wie Vector NTI 8 (Version vom 25. September 2002) der Firma InforMax Inc. können solche Bereiche kartiert und identifiziert werden.

Weitere geeignete Polypeptidsequenzen (i) mit einer guten Bindung zu humanem Keratin sind Sequenzbereiche, die in einem Alignment hohe Homologie bzw. Sequenzidentität aufweisen und als Konsensussequenzen der Keratindindedomänen aufgefasst werden können.

Besonders bevorzugt sind unter diesen Sequenzbereichen die folgenden:
Domäne B (KBD-B): Polypeptidsequenz SEQ ID NO: 1 Position 2193 bis 2448
Domäne B (KBD-B): Polypeptidsequenz SEQ ID NO: 1 Position 2209 bis 2448
Domäne C (KBD-C): Polypeptidsequenz SEQ ID NO: 1 Position 2606 bis 2871
Domäne C (KBD-C): Polypeptidsequenz SEQ ID NO: 1 Position 2616 bis 2871
Domäne C (KBD-C): Polypeptidsequenz SEQ ID NO: 1 Position 2616 bis 2811
Domäne C (KBD-C): Polypeptidsequenz SEQ ID NO: 1 Position 2606 bis 2871

Wenn es gewünscht wird, dass die Polypeptidsequenzen (i) zu einem Keratin aus einem nicht-humanen Organismus eine besonders gute Bindung aufweisen, werden als geeignete Sequenzmotive bevorzugt solche aus dem Keratin-bindenden Protein, z.B. Desmoplakin oder Plectin, des entsprechenden Organismus ausgewählt.

Abb. 2 zeigt ein Alignment von Keratin-bindenden Molekülen.

### Effektormoleküle (ii)

Als Effektormoleküle (ii) werden im folgenden Moleküle verstanden, die eine bestimmte, vorhersehbare Wirkung aufweisen. Dies können entweder proteinartige Moleküle sein, wie Enzyme oder auch nicht-proteinogene Moleküle wie Farbstoffe, Lichtschutz-mittel, Vitamine, Provitamine, Antioxidantien und Fettsäuren, Conditioner oder Metallionen- enthaltende Verbindungen.

Unter den proteinartigen Effektormolekülen sind Enzyme und Antikörper bevorzugt. Unter den Enzymen sind folgende als Effektormoleküle (ii) bevorzugt: Oxidasen, Peroxidasen, Proteasen, Glucanasen, Mutanase, Tyrosinasen, Laccasen, metallbindende Enzyme, Lactoperoxidase, Lysozym, Amyloglycosidase, Glucoseoxidase, Superoxid-dismutase, Photolyase, T4 Endonuklease, Katalase, Thioredoxin, Thioredoxin-Reduktase.
Bei den proteinartigen aber nicht enzymatisch wirkenden Effektormolekülen sind folgende als Effektormoleküle (ii) bevorzugt: Antimikrobielle Peptide, Seidenproteine, Hydrophobine, Kollagen, Carotenoid bindende Proteine, Schwermetalle bindende Proteine, Odorantien bindende Proteine.

Gut geeignet als proteinartige Effektormoleküle (ii) sind auch Hydrolysate von Proteinen aus pflanzlichen und tierischen Quellen, beispielsweise Hydrolysate von Proteinen marinen Ursprungs oder Seidenhydrolysate.

Unter den nicht-proteinartigen Effektormolekülen (ii) sind Farbstoffe, beispielsweise Lebensmittelfarbstoffe, semipermanente Farbstoffe, Reaktiv- oder Oxidationsfarbstoffe, bevorzugt. Bei den Oxidationsfarbstoffen ist es bevorzugt, eine Komponente als Effektormolekül (ii) mit der Keratin-bindenden Polypeptidsequenz (i) zu verknüpfen und anschliessend am Wirkort, d.h. nach Bindung am Haar, oxidativ mit der zweiten Farbstoffkomponente zu kuppeln. Bevorzugt ist es bei Oxidationsfarbstoffen ferner, die Kupplung der Farbkomponenten vor der Verknüpfung mit der Polypeptidsequenz (i) auszuführen.

Die Reaktivfarbstoffe können weiterhin bevorzugt als eine Komponente als Effektormolekül (ii) mit der Keratin-bindenden Polypeptidsequenz (i) verknüpft sein und anschließend an das Haar gebunden werden. Weiterhin können solche Farbstoffe die als Effektormolekül (ii) mit der Keratin-bindenden Polypeptidsequenz (i) verknüpft durch Bindung an Nagel oder Haut in der dekorativen Kosmetik eingesetzt werden.
Als Farbstoffe sind alle gängigen Haarfarbstoffe für die erfindungsgemässen Moleküle geeignet. Geeignete Farbstoffe sind dem Fachmann aus Handbüchern der Kosmetik beispielsweise Schrader, Grundlagen und Rezepturen der Kosmetika, Hüthig Verlag, Heidelberg, 1989, ISBN 3-7785-1491-1 bekannt.

Besonders vorteilhafte Farbstoffe sind die in der folgenden Liste genannten. Die Colour Index Nummern (CIN) sind dem Rowe Colour Index, 3. Auflage, Society of Dyers and Colourists, Bradford, England, 1971 entnommen.

| Chemische oder sonstige Bezeichnung | CIN | Farbe |
|---|---|---|
| Pigment Green | 10006 | grün |
| Acid Green 1 | 10020 | grün |
| 2,4-Dinitrohydroxynaphthalin-7-sulfosäure | 10316 | gelb |
| Pigment Yellow 1 | 11680 | gelb |
| Pigment Yellow 3 | 11710 | gelb |
| Pigment Orange 1 | 11725 | orange |
| 2,4-Dihydroxyazobenzol | 11920 | orange |
| Solvent Red 3 | 12010 | rot |
| 1-(2'-Chlor-4'-nitro-1'-phenylazo)-2-hydroxynaphthalin | 12085 | rot |
| Pigment Red 3 | 12120 | rot |
| Ceresrot; Sudanrot; Fettrot G | 12150 | rot |
| Pigment Red 112 | 12370 | rot |
| Pigment Red 7 | 12420 | rot |
| Pigment Brown 1 | 12480 | braun |
| 4-(2'-Methoxy-5'-sulfosäurediethylamid-1'-phenylazo)-3-hydroxy-5"-chloro-2",4"-dimethoxy-2-naphthoesäureanilid | 12490 | rot |
| Disperse Yellow 16 | 12700 | gelb |
| 1-(4-Sulfo-1-phenylazo)-4-amino-benzol-5-sulfosäure | 13015 | gelb |
| 2,4-Dihydroxy-azobenzol-4'-sulfosäure | 14270 | orange |
| 2-(2,4-Dimethylphenylazo-5-sulfosäure)-1-hydroxynaphthalin-4-sulfosäure | 14700 | rot |
| 2-(4-Sulfo-1-naphthylazo)-1-naphthol-4-sulfosäure | 14720 | rot |
| 2-(6-Sulfo-2,4-xylylazo)-1-naphthol-5-sulfosäure | 14815 | rot |
| 1-(4'-Sulfophenylazo)-2-hydroxynaphthalin | 15510 | orange |
| 1-(2-Sulfosäure-4-chlor-5-carbonsäure-1-phenylazo)-2-hydroxynaphthalin | 15525 | rot |
| 1-(3-Methyl-phenylazo-4-sulfosäure)-2-hydroxynaphthalin | 15580 | rot |
| 1-(4',(8')-Sulfosäurenaphthylazo)-2-hydroxynaphthalin | 15620 | rot |
| 2-Hydroxy-1,2'-azonaphthalin-1'-sulfosäure | 15630 | rot |
| 3-Hydroxy-4-phenylazo-2-naphthylcarbonsäure | 15800 | rot |
| 1-(2-Sulfo-4-methyl-1-phenylazo)-2-naphthylcarbonsäure | 15850 | rot |
| 1-(2-Sulfo-4-methyl-5-chlor-1-phenylazo)-2-hydroxy-naphthalin-3-carbonsäure | 15865 | rot |
| 1-(2-Sulfo-1-naphthylazo)-2-hydroxynaphthalin-3-carbonsäure | 15880 | rot |
| 1-(3-Sulfo-1-phenylazo)-2-naphthol-6-sulfosäure | 15980 | orange |
| 1-(4-Sulfo-1-phenylazo)-2-naphthol-6-sulfosäure | 15985 | gelb |
| Allura Red | 16035 | rot |
| 1-(4-Sulfo-1-naphthylazo)-2-naphthol-3,6-disulfosäure | 16185 | rot |
| Acid Orange 10 | 16230 | orange |
| 1-(4-Sulfo-1-naphthylazo)-2-naphthol-6,8-disulfosäure | 16255 | rot |
| 1-(4-Sulfo-1-naphthylazo)-2-naphthol-3,6,8-trisulfosäure | 16290 | rot |
| 8-Amino-2-phenylazo-1-naphthol-3,6-disulfosäure | 17200 | rot |
| Acid Red 1 | 18050 | rot |
| Acid Red 155 | 18130 | rot |
| Acid Yellow 121 | 18690 | gelb |
| Acid Red 180 | 18736 | rot |
| Acid Yellow 11 | 18820 | gelb |
| Acid Yellow 17 | 18965 | gelb |
| 4-(4-Sulfo-1-phenylazo)-1-(4-sulfophenyl)-5-hydroxy-pyrazolon-3-carbonsäure | 19140 | gelb |
| Pigment Yellow 16 | 20040 | gelb |
| 2,6-(4'-Sulfo-2",4"-dimethyl)-bis-phenylazo)-1,3-dihydroxybenzol | 20170 | orange |
| Acid Black 1 | 20470 | schwarz |
| Pigment Yellow 13 | 21100 | gelb |
| Pigment Yellow 83 | 21108 | gelb |
| Solvent Yellow | 21230 | gelb |
| Acid Red 163 | 24790 | rot |
| Acid Red 73 | 27290 | rot |
| 2-[4'-(4"-Sulfo-1"-phenylazo)-7'-suffo-1'-naphthylazo]-1-hydroxy-7-aminonaphthalin-3,6-disulfosäure | 27755 | schwarz |
| 4'-[(4"-Sulfo-1"-phenylazo)-7'-sulfo-1'-naphthylazo]-1-hydroxy-8-acetyl-amino-naphthalin-3,5-disulfosäure | 28440 | schwarz |
| Direct Orange 34, 39, 44, 46, 60 | 40215 | orange |
| Food Yellow | 40800 | orange |
| trans-β-Apo-8'-Carotinaldehyd (C₃₀) | 40820 | orange |
| trans-Apo-8'-Carotinsäure(C₃₀)-ethylester | 40825 | orange |
| Canthaxanthin | 40850 | orange |
| Acid Blue 1 | 42045 | blau |
| 2,4-Disulfo-5-hydroxy-4'-4"-bis-(diethylamino)-triphenyl-carbinol | 42051 | blau |
| 4-[(-4-N-Ethyl-p-sulfobenzylamino)-phenyl-(4-hydroxy-2-sulfophenyl)-(methylen)-1-(N-ethylN-p-sulfobenzyl)-2,5-cyclohexadienimin] | 42053 | grün |
| Acid Blue 7 | 42080 | blau |
| (N-Ethyl-p-sulfobenzyl-amino)-phenyl-(2-sulfophenyl)-methylen-(N-ethyl-N-p-sulfo-benzyl)-□^{2,5}-cyclohexadienimin | 42090 | blau |
| Acid Green 9 | 42100 | grün |
| Diethyl-di-sulfobenzyl-di-4-amino-2-chlor-di-2-methyl-fuchsonimmonium | 42170 | grün |
| Basic Violet 14 | 42510 | violett |
| Basic Violet 2 | 42520 | violett |
| 2'-Methyl-4'-(N-ethyl-N-m-sulfobenzyl)-amino-4"-(N-diethyl)-amino-2-methyl-N-ethylN-m-sulfobenzyl-fuchsonimmonium | 42735 | blau |
| 4'-(N-Dimethyl)-amino-4"-(N-phenyl)-amino-naphtho-N-dimethyl-fuchsonimmonium | 44045 | blau |
| 2-Hydroxy-3,6-disulfo-4,4'-bis-dimethylaminonaphthofuchsonimmonium | 44090 | grün |
| Acid Red 52 | 45100 | rot |
| 3-(2'-Methylphenylamino)-6-(2'-methyl-4'-sulfophenylamino)-9-(2"-carboxyphenyl)-xantheniumsalz | 45190 | violett |
| Acid Red 50 | 45220 | rot |
| Phenyl-2-oxyfluoron-2-carbonsäure | 45350 | gelb |
| 4,5-Dibromfluorescein | 45370 | orange |
| 2,4,5,7-Tetrabromfluorescein | 45380 | rot |
| Solvent Dye | 45396 | orange |
| Acid Red 98 | 45405 | rot |
| 3',4',5',6'-Tetrachlor-2,4,5,7-tetrabrom-fluorescein | 45410 | rot |
| 4,5-Diiodfluorescein | 45425 | rot |
| 2,4,5,7-Tetraiodfluorescein | 45430 | rot |
| Chinophthalon | 47000 | gelb |
| Chinophthalon-disulfosäure | 47005 | gelb |
| Acid Violet 50 | 50325 | violett |
| Acid Black 2 | 50420 | schwarz |
| Pigment Violet 23 | 51319 | violett |
| 1,2-Dioxyanthrachinon, Calcium-Aluminiumkomplex | 58000 | rot |
| 3-Oxypyren-5,8,10-sulfosäure | 59040 | grün |
| 1-Hydroxy-4-N-phenyl-aminoanthrachinon | 60724 | violett |
| 1-Hydroxy-4-(4'-methylphenylamino)-anthrachinon | 60725 | violett |
| Acid Violet 23 | 60730 | violett |
| 1,4-Di(4'-methyl-phenylamino)-anthrachinon | 61565 | grün |
| 1,4-Bis-(o-sulfo-p-toluidino)-anthrachinon | 61570 | grün |
| Acid Blue 80 | 61585 | blau |
| Acid Blue 62 | 62045 | blau |
| N,N'-Dihydro-1,2,1',2'-anthrachinonazin | 69800 | blau |
| Vat Blue 6; Pigment Blue 64 | 69825 | blau |
| Vat Orange 7 | 71105 | orange |
| Indigo | 73000 | blau |
| Indigo-disulfosäure | 73015 | blau |
| 4,4'-Dimethyl-6,6'-dichlorthioindigo | 73360 | rot |
| 5,5'-Dichlor-7,7'-dimethylthioindigo | 73385 | violett |
| Quinacridone Violet 19 | 73900 | violett |
| Pigment Red 122 | 73915 | rot |
| Pigment Blue 16 | 74100 | blau |
| Phthalocyanine | 74160 | blau |
| Direct Blue 86 | 74180 | blau |
| Chlorierte Phthalocyanine | 74260 | grün |
| Natural Yellow 6,19; Natural Red 1 | 75100 | gelb |
| Bixin, Nor-Bixin | 75120 | orange |
| Lycopin | 75125 | gelb |
| trans-alpha-, beta- bzw. gamma-Carotin | 75130 | orange |
| Keto- und/oder Hydroxylderivate des Carotins | 75135 | gelb |
| Guanin oder Perlglanzmittel | 75170 | weiß |
| 1,7-Bis-(4-hydroxy-3-methoxyphenyl)1,6-heptadien-3,5-dion | 75300 | gelb |
| Komplexsalz (Na, Al, Ca) der Karminsäure | 75470 | rot |
| Chlorophyll a und b; Kupferverbindungen der Chlorophylle und Chlorophylline | 75810 | grün |
| Aluminium | 77000 | weiß |
| Tonerdehydrat | 77002 | weiß |
| Wasserhaltige Aluminiumsilikate | 77004 | weiß |
| Ultramarin | 77007 | blau |
| Pigment Red 101 und 102 | 77015 | rot |
| Bariumsulfat | 77120 | weiß |
| Bismutoxychlorid und seine Gemische mit Glimmer | 77163 | weiß |
| Calciumcarbonat | 77220 | weiß |
| Calciumsulfat | 77231 | weiß |
| Kohlenstoff | 77266 | schwarz |
| Pigment Black 9 | 77267 | schwarz |
| Carbo medicinalis vegetabilis | 77268 | schwarz |
| Chromoxid | 77288 | grün |
| Chromoxid, wasserhaltig | 77289 | grün |
| Pigment Blue 28, Pigment Green 14 | 77346 | grün |
| Pigment Metal 2 | 77400 | braun |
| Gold | 77480 | braun |
| Eisenoxide und -hydoxide | 77489 | orange |
| Eisenoxid | 77491 | rot |
| Eisenoxidhydrat | 77492 | gelb |
| Eisenoxid | 77499 | schwarz |
| Mischungen aus Eisen(II)- und Eisen(III)-hexacyanoferrat | 77510 | blau |
| Pigment White 18 | 77713 | weiß |
| Manganammoniumdiphosphat | 77742 | violett |
| Manganphosphat; Mn₃(PO₄)₂ □ 7 H20 | 77745 | rot |
| Silber | 77820 | weiß |
| Titandioxid und seine Gemische mit Glimmer | 77891 | weiß |
| Zinkoxid | 77947 | weiß |
| 6,7-Dimethyl-9-(1'-D-ribityl)-isoalloxazin, Lactoflavin | | gelb |
| Zuckerkulör | | braun |
| Capsanthin, Capsorubin | | orange |
| Betanin | | rot |
| Benzopyryliumsalze, Anthocyane | | rot |
| Aluminium-, Zink-, Magnesium- und Calcium-stearat | | weiß |
| Bromthymolblau | | blau |
| Bromkresolgrün | | grün |
| Acid Red 195 | | -ot |

Gut geeignet können auch Lebensmittelfarbstoffe als Haarfarbstoffe geeignet sein.

Die oben genannten Farbstoffe können auch als Effektormoleküle(ii) an einer haut- oder nagelbindenden Polypeptidsequenz (i) für die Haut- oder Nagel-Colourierung z.B. in Tattoos, eingesetzt werden.

Die erfindungsgemäßen Effektormolekül (ii) welche mit der Keratin-bindenden Polypeptidsequenz (i) verknüpft sind auch - falls gewünscht - wieder leicht vom Keratin in Haut-, Haar oder Nagel getrennt werden. Hierzu kann beispielsweise eine Spülung mit Keratin eingesetzt werden, wodurch Effektormolekül (ii) welche mit der Keratin-bindenden Polypeptidsequenz (i) verknüpft sind aus ihrer bestehenden Bindung zum Keratin verdrängt werden und mit dem Keratin aus der Spülung abgesättigt werden. Alternativ ist auch eine Spülung mit einem hohen Anteil an Detergenz (z.B. SDS) zum Abwaschen möglich.
Weitere bevorzugte Effektormoleküle (ii) sind Fettsäuren, insbesondere gesättigte Fettsäuren, die eine Alkylverzweigung tragen, besonders bevorzugt verzweigte Eicosansäuren, wie 18-Methyl-Eicosansäure.

Weitere bevorzugte Effektormoleküle (ii) sind Carotinoide. Unter Carotinoide sind erfindungsgemäß folgende Verbindungen sowie deren veresterte oder glykosylierte Derivate zu verstehen: β-Carotin, Lycopin, Lutein, Astaxanthin, Zeaxanthin, Cryptoxanthin, Citranaxanthin, Canthaxanthin, Bixin, β-Apo-4-carotinal, β-Apo-8-carotinal, β-Apo-8-carotinsäureester, Neurosporen, Echinenon, Adonirubin, Violaxanthin, Torulen, Torularhodin, einzeln oder als Mischung. Bevorzugt verwendete Carotinoide sind β-Carotin, Lycopin, Lutein, Astaxanthin, Zeaxanthin, Citranaxanthin und Canthaxanthin.

Weitere bevorzugte Effektormoleküle (ii) sind Vitamine, insbesondere Vitamin A und deren Ester.

Unter Retinoide sind im Rahmen der vorliegenden Erfindung Vitamin A Alkohol (Retinol) und seine Derivate wie Vitamin A Aldehyd (Retinal), Vitamin A Säure (Retinsäure) und Vitamin A Ester (z.B. Retinylacetat, Retinylpropionat und Retinylpalmitat) gemeint. Der Begriff Retinsäure umfasst dabei sowohl all-trans Retinsäure als auch 13-cis Retinsäure. Die Begriffe Retinol und Retinal umfassen bevorzugt die all-trans Verbindungen. Als bevorzugtes Retinoid verwendet man für die erfindungsgemäßen Suspensionen all-trans-Retinol, im folgenden als Retinol bezeichnet.

Weitere bevorzugte Effektormoleküle (ii) sind Vitamine, Provitamine und Vitaminvorstufen aus den Gruppen A, C, E und F, insbesondere 3,4-Didehydroretinol, β-Carotin (Provitamin des Vitamin A), Ascorbinsäure (Vitamin C), sowie die Palmitinsäureester, Glucoside oder Phosphate der Ascorbinsäure, Tocopherole, insbesondere α-Tocopherol sowie seine Ester, z.B. das Acetat, das Nicotinat, das Phosphat und das Succinat; weiterhin Vitamin F, worunter essentielle Fettsäuren, besonders Linolsäure, Linolensäure und Arachidonsäure, verstanden werden.

Zu den erfindungsgemäß bevorzugt einzusetzenden Vitaminen, Provitaminen oder Vitaminvorstufen der Vitamin B-Gruppe oder deren Derivaten sowie den Derivaten von 2-Furanon gehören unter anderem:
Vitamin B₁ , Trivialname Thiamin, chemische Bezeichnung 3-[(4'-Amino-2'-methyl-5'-pyrimidinyl) methyl]-5-(2-hydroxyethyl)-4-methylthiazoliumchlorid.

Vitamin B₂, Trivialname Riboflavin, chemische Bezeichung 7,8-Dimethyl-10-(1-D-ribityl)-benzo[g]pteridin-2,4(3H,10H)-dion. In freier Form kommt Riboflavin z. B. in Molke vor, andere Riboflavin-Derivate lassen sich aus Bakterien und Hefen isolieren. Ein erfindungsgemäß ebenfalls geeignetes Stereoisomeres des Riboflavin ist das aus Fischmehl oder Leber isolierbare Lyxoflavin, das statt des D-Ribityl einen D-Arabityl-Rest trägt.

Vitamin B₃. Unter dieser Bezeichnung werden häufig die Verbindungen Nicotinsäure und Nicotinsäureamid (Niacinamid) geführt. Erfindungsgemäß bevorzugt ist das Nicotinsäureamid.

Vitamin B₅ (Pantothensäure und Panthenol). Bevorzugt wird Panthenol eingesetzt. Erfindungsgemäß einsetzbare Derivate des Panthenols sind insbesondere die Ester und Ether des Panthenols sowie kationisch derivatisierte Panthenole. In einer weiteren bevorzugten Ausführungsform der Erfindung können zusätzlich zu Pantothensäure oder Panthenol auch Derivate des 2-Furanon eingesetzt werden. Besonders bevorzugte Derivate sind die auch im Handel erhältlichen Substanzen Dihydro-3 hydroxy-4,4-dimethyl-2(3H)-furanon mit dem Trivialnamen Pantolacton (Merck), 4 Hydroxymethyl-γbutyrolacton (Merck), 3,3-Dimethyl-2-hydroxy-γ-butyrolacton (Aldrich) und 2,5- Dihydro-5-methoxy-2-furanon (Merck), wobei ausdrücklich alle Stereoisomeren eingeschlossen sind.

Vorteilhafterweise verleihen diese Verbindungen den erfindungsgemässen Keratin-bindenden Effektormolekülen feuchtigkeitsspendende sowie hautberuhigende Eigenschaften.

Vitamin B₆, wobei man hierunter keine einheitliche Substanz, sondern die unter den Trivialnamen Pyridoxin, Pyridoxamin und Pyridoxal bekannten Derivate des 5 Hydroxymethyl-2-methylpyridin-3-ols versteht.

Vitamin B₇ (Biotin), auch als Vitamin H oder "Hautvitamin" bezeichnet. Bei Biotin handelt es sich um (3aS,4S, 6aR)-2-Oxohexahydrothienol[3,4-d]imidazol-4-valeriansäure.

Panthenol, Pantolacton, Nicotinsäureamid sowie Biotin sind erfindungsgemäß ganz besonders bevorzugt.

Erfindungsgemäß können geeignete Derivate (Salze, Ester, Zucker, Nukleotide, Nukleoside, Peptide und Lipide). Als lipophile, öllösliche Antioxidantien aus dieser Gruppe sind Tocopherol und dessen Derivate, Gallussäureester, Flavonoide und Carotinoide sowie Butylhydroxytoluollanisol bevorzugt. Als wasserlösliche Antioxidantien sind Aminosäuren, z. B. Tyrosin und Cystein und deren Derivate sowie Gerbstoffe, insbesondere solche pflanzlichen Ursprungs bevorzugt.

Triterpene, insbesondere Triterpensäuren wie Ursolsäure, Rosmarinsäure, Betulinsäure, Boswelliasäure und Bryonolsäure.

Ein weiteres bevorzugtes Effektormolekül (ii) ist Liponsäure und geeignete Derivate (Salze, Ester, Zucker, Nukleotide, Nukleoside, Peptide und Lipide).

Weitere bevorzugte Effektormoleküle (ii) sind UV-Lichtschutzfilter. Darunter sind organische Substanzen zu verstehen, die in der Lage sind, ultraviolette Strahlen zu absorbieren und die aufgenommene Energie in Form längerwelliger Strahlung, z.B. Wärme, wieder abzugeben. Die organischen Substanzen können öllöslich oder wasserlöslich sein.

Als öllösliche UV-B-Filter können z.B. folgende Substanzen verwendet werden:

3-Benzylidencampher und dessen Derivate, z.B. 3-(4-Methylbenzyliden)campher;

4-Aminobenzoesäurederivate, vorzugsweise 4-(Dimethylamino)benzoesäure-2-ethylhexylester, 4-( Dimethylamino)benzoesäure-2-octylester und 4-(Dimethylamino)-benzoesäureamylester;

Ester der Zimtsäure, vorzugsweise 4-Methoxyzimtsäure-2-ethylhexylester, 4 Methoxyzimtsäurepropylester, 4-Methoxyzimtsäureisoamylester, 4 Methoxyzimtsäureisopentylester, 2-Cyano-3-phenyl-zimtsäure-2-ethylhexylester (Octocrylene);

Ester der Salicylsäure, vorzugsweise Salicylsäure-2-ethylhexylester, Salicylsäure-4 isopropylbenzylester, Salicylsäurehomomenthylester;

Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;

Ester der Benzalmalonsäure, vorzugsweise 4-Methoxybenzmalonsäuredi-2-ethylhexylester;

Triazinderivate, wie z.B. 2,4,6-Trianilino-(p-carbo-2'-ethyl-1'-hexyloxy)-1,3,5-triazin (Octyltriazone) und Dioctyl Butamido Triazon (Uvasorb^{®} HEB):
Propan-1,3-dione, wie z.B. 1 -(4-tert. Butylphenyl)-3-(4'-methoxyphenyl)propan-1,3-dion.

Als wasserlösliche Substanzen kommen in Frage:

2-Phenylbenzimidazol-5-sulfonsäure und deren Alkali-, Erdalkali-, Ammonium-, Alkylammonium-, Alkanolammonium- und Glucammoniumsalze;

Sulfonsäurederivate von Benzophenonen , vorzugsweise 2-Hydroxy-4-methoxybenzo-phenon-5-sulfonsäure und ihre Salze;

Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Besonders bevorzugt ist die Verwendung von Estern der Zimtsäure, vorzugsweise 4-Methoxyzimtsäure-2-ethylhexylester, 4-Methoxyzimtsäureisopentylester, 2-Cyano-3-phenyl-zimtsäure-2-ethylhexylester (Octocrylene).

Des weiteren ist die Verwendung von Derivaten des Benzophenons, insbesondere 2-Hydroxy-4-methoxybenzophenon, 2-Hydroxy-4-methoxy-4'-methylbenzophenon, 2,2'-Dihydroxy-4-methoxybenzophenon sowie der Einsatz von Propan-1,3-dionen, wie z.B. 1-(4-tert. Butylphenyl)-3-(4-'methoxyphenyl)propan-1,3-dion bevorzugt.

Als typische UV-A-Filter kommen in Frage:
Derivate des Benzoylmethans, wie beispielsweise 1-(4'-tert.Butylphenyl)-3-(4'-methoxyphenyl)propan-1,3-dion, 4-tert.-Butyl-4'-methoxydibenzoylmethan oder 1-Phenyl-3-(4'-isopropylphenyl)-propan-1,3-dion;
Amino-hydroxy-substituierte Derivate von Benzophenonen wie z.B. N,N-Diethylamino-hydroxybenzoyl-n-hexylbenzoat.

Die UV-A und UV-B-Filter können selbstverständlich auch in Mischungen eingesetzt werden.

Geeignete UV-Filtersubstanzen sind in der folgenden Tabelle genannt.

| Nr. | Stoff | CAS-Nr. (=Säure) |
|---|---|---|
| 1 | 4-Aminobenzoesäure | 150-13-0 |
| 2 | 3-(4'-Trimethylammonium)-benzylidenbornan-2-on-methylsulfat | 52793-97-2 |
| 3 | 3,3,5-Trimethyl-cyclohexyl-salicylat (Homosalatum) | 118-56-9 |
| 4 | 2-Hydroxy-4-methoxy-benzophenon (Oxybenzonum) | 131-57-7 |
| 5 | 2-Phenylbenzimidazol-5-sulfonsäure und ihre Kalium-, Na-trium- u. Triethanolaminsalze | 27503-81-7 |
| 6 | 3,3'-(1,4-Phenylendimethin)-bis(7,7-dimethyl-2-oxobicyclo[2.2.1]heptan-1-methansulfonsäure) und ihre Salze | 90457-82-2 |
| 7 | 4-Bis(polyethoxy)amino-benzoesäurepolyethoxy-ethylester | 113010-52-9 |
| 8 | 4-Dimethylamino-benzoesäure-2-ethylhexylester | 21245-02-3 |
| 9 | Salicylsäure-2-ethylhexylester | 118-60-5 |
| 10 | 4-Methoxy-zimtsäure-2-isoamylester | 71617-10-2 |
| 11 | 4-Methoxy-zimtsäure-2-ethylhexylester | 5466-77-3 |
| 12 | 2-Hydroxy-4-methoxy-benzophenon-5-sulfonsäure-(Sulisobenzonum) und das Natriumsalz | 4065-45-6 |
| 13 | 3-(4'-Sulfobenzyliden)-bornan-2-on und Salze | 58030-58-6 |
| 14 | 3-Benzylidenbornan-2-on | 16087-24-8 |
| 15 | 1-(4'-Isopropylphenyl)-3-phenylpropan-1,3-dion | 63260-25-9 |
| 16 | 4-Isopropylbenzylsalicylat | 94134-93-7 |
| 17 | 3-Imidazol-4-yl-acrylsäure und ihr Ethylester | 104-98-3 |
| 18 | 2-Cyano-3,3-diphenylacrylsäureethylester | 5232-99-5 |
| 19 | 2-Cyano-3,3-diphenylacrylsäure-2'-ethylhexylester | 6197-30-4 |
| 20 | Menthyl-o-aminobenzoat oder: 5-Methyl-2-(1-methylethyl)-2-aminobenzoat | 134-09-8 |
| 21 | Glyceryl p-aminobenzoat oder: 4-Aminobenzoesäure-1-glyceryl-ester | 136-44-7 |
| 22 | 2,2'-Dihydroxy-4-methoxybenzophenon (Dioxybenzone) | 131-53-3 |
| 23 | 2-Hydroxy-4-methoxy-4-methylbenzophenon (Mexenon) | 1641-17-4 |
| 24 | Triethanolamin Salicylat | 2174-16-5 |
| 25 | Dimethoxyphenylglyoxalsäure oder: 3,4-dimethoxy-phenyl-glyoxal-saures Natrium | 4732-70-1 |
| 26 | 3-(4'Sulfobenzyliden)-bornan-2-on und seine Salze | 56039-58-8 |
| 27 | 4-tert.-Butyl-4'-methoxy-dibenzoylmethan | 70356-09-1 |
| 28 | 2,2',4,4'-Tetrahydroxybenzophenon | 131-55-5 |
| 29 | 2,2'-Methylen-bis-[6-(2H-benzotriazol-2-yl)-4-(1,1,3,3,-tetramethylbutyl)phenol] | 103597-45-1 |
| 30 | 2,2'-(1,4-Phenylen)-bis-1H-benzimidazol-4,6-disulfonsäure, Na-Salz | 180898-37-7 |
| 31 | 2,4-bis-[4-(2-Ethylhexyloxy)-2-hydroxy]phenyl-6-(4-methoxyphenyl)-(1,3,5)-triazin | 187393-00-6 |
| 32 | 3-(4-Methylbenzyliden)-campher | 36861-47-9 |
| 33 | 4-Bis(polyethoxy)paraaminobenzoesäurepolyethoxyethylester | 113010-52-9 |
| 34 | 2,4-Dihydroxybenzophenon | 131-56-6 |
| 35 | 2,2'-Dihydroxy-4,4'-dimethoxybenzophenon-5,5'-dinatriumsulfonat | 3121-60-6 |
| 36 | Benzoesäure, 2-[4-(diethylamino)-2-hydroxybenzoyl]-, hexylester | 302776-68-7 |
| 37 | 2-(2H-Benzotriazol-2-yl)-4-methyl-6-[2-methyl-3-[1,3,3,3-tetramethyl-1-[(trimethylsilyl)oxy]disiloxanyl]propyl]phenol | 155633-54-8 |
| 38 | 1,1-[(2,2'-Dimethylpropoxy)carbonyl]-4,4-diphenyl-1,3-butadien | 363602-15-7 |

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Superoxid-Dismutase, Katalase, Tocopherole (Vitamin E) und Ascorbinsäure (Vitamin C).

Eine weitere Gruppe sind Anti-irritantien, die eine entzündungshemmende Wirkung auf durch UV-Licht geschädigte Haut besitzten. Solche Stoffe sind beispielsweise Bisabolol, Phytol und Phytantriol.

### Verknüpfung der Effektormoleküle (ii) mit der Keratin-bindenden Polypeptidsequenz (i)

Die Effektormoleküle (ii) sind mit einer Polypeptidsequenz (i), die eine Bindungsaffinität zu einem Keratin besitzt, verbunden. Die Verbindung zwischen (i) und (ii) kann sowohl eine kovalente Bindung sein als auch auf ionischen oder van-der Waals Wechselwirkungen beruhen.

Bevorzugt ist eine kovalente Verknüpfung. Diese kann beispielsweise über die Seitenketten der Polypeptidsequenz (i) erfolgen, insbesondere über Aminofunktionen oder Hydroxyfunktionen oder Carboxylatfunktionen oder Thiolfunktionen. Bevorzugt ist eine Verknüfung über die Aminofunktionen von einem oder mehreren Lysinresten, einer oder mehrere Thiolgruppen von Cysteinresten oder über die N-terminale oder C-terminale Funktion des Polypeptids (i). Außer den in der Polypeptidsequenz (i) vorkommenden Aminosäurefunktionen können auch Aminosäuren mit geeigneten Funktionen (z.B. Cysteine, Lysine, Aspartate, Glutamate) an die Sequenz angefügt werden, oder Aminosäuren der Polypeptidsequenz (i) durch solche Aminosäurefunktionen substituiert werden.

Die Verknüpfung der Effektormoleküle (ii) mit der Polypeptidsequenz (i) kann entweder direkt, d.h. als kovalente Verknüpfung zweier in (i) und (ii) bereits vorhandener chemischer Funktionen erfolgen, beispielsweise eine Aminofunktion von (i) wird mit einer Carboxylatfunktion von (ii) zum Säureamid verknüpft. Die Verknüpfung kann aber auch über einen sogenannten Linker, d.h. einem mindestens bifunktionellen Molekül erfolgen, der mit einer Funktion mit (i) eine Bindung eingeht und mit einer oder mehreren anderen Funktionen mit (ii) verknüpft wird.

Falls das Effektormolekül (ii) ebenfalls aus einer Polypeptidsequenz besteht, kann die Verknüpfung von (i) und (ii) als ein sogenanntes Fusionsprotein erfolgen, d.h. eine durchgängige Polypeptidsequenz, die aus den beiden Teilsequenzen (i) und (ii) besteht.

Es können zwischen (i) und (ii) auch noch sogenannte Spacerelemente eingebaut werden, beispielsweise Polypeptidsequenzen, die eine potentielle Spaltstelle für eine Protease, Lipase, Esterase, Phosphatase, Hydrolase besitzen oder Oligo- und Polypeptidsequenzen, die eine leichte Aufreinigung des Fusionsproteins gestatten, beispielsweise sogenannte His-tags, d.h. Oligohistidinreste.

Weiterhin können die Spacerelemete aus , Alkylketten, Ethylenglykol und Polyethylenglykolen zusammengesetzt sein.

Besonders bevorzugt sind Linker- und/oder Spacerelemente, die eine potentielle Spaltstelle für eine Protease, Lipase, Esterase, Phosphatase, Hydrolase besitzen, d.h. enzymatisch spaltbar sind.
Beispiele für enzymatisch spaltbare Linker, die bei den erfindungsgemässen Molekülen eingesetzt werden können, sind beispielsweise in WO 98/01406 genannt.

Besonders bevorzugt sind Linker und Spacer die thermospaltbar, photospaltbar sind. Entsprechende chemische Strukturen sind dem Fachmann bekannt und werden zwischen die Molekülteile (i) und (ii) integriert.

Die Verknüpfung im Falle eines nicht-proteinartigen Effektormoleküls mit der Polypeptidsequenz (i) erfolgt bevorzugt durch funktionalisierbare Reste (Seitengruppen, C- oder N-Terminus) am Polypeptid (i), die mit einer chemischen Funktion des Effektormoleküls eine kovalente Verbindung eingehen.

Bevorzugt ist hier die Bindungsknüpfung über eine Amino-, Thiol- oder Hydroxyfunktion des Polypeptids (i), die beispielsweise mit einer Carboxylfunktion des Effektormoleküls (ii), ggf. nach Aktivierung, eine entsprechende Amid-, Thioester oder Esterbindung eingehen können.

Eine weitere bevorzugte Verknüpfung der Polypeptidsequenz (i) mit einem Effektormolekül (ii) ist die Verwendung eines massgeschneiderten Linkers. Ein solcher Linker hat zwei oder mehr sogenannte Ankergruppen mit denen er die Polypeptidsequenz (i) und ein oder mehrere Effektormoleküle (ii) verknüpfen kann. Beispielsweise kann eine Ankergruppe für (i) eine Thiolfunktion sein, mittels derer der Linker mit einem Cysteinrest des Polypeptids (i) eine Disulfidbindung eingehen kann. Eine Ankergruppe für (ii) kann beispielsweise eine Carboxylfunktion sein, mittels derer der Linker mit einer Hydroxylfunktion des Effektormoleküls (ii) eine Esterbindung eingehen kann.

Die Verwendung solcher massgeschneiderter Linker erlaubt die genaue Anpassung der Verknüpfung an das gewünschte Effektormolekül. Ausserdem ist es dadurch möglich, mehrere Effektormoleküle mit einer Polypeptidsequenz (i) definiert zu verknüpfen.

Der verwendete Linker richtet sich nach der zu koppelnden Funktionalität. Geeignet sind z.B. Moleküle, die zu Polypeptiden (i) koppeln mittels Sulfhydryl-reaktiven Gruppen, z.B. Maleimide, Pydridyldisulfide, α -Haloacetyle, Vinylsulfone, Sulfatoalkylsulfone (bevorzugt Sulfatoethylsulfone) und zu Effektormolekülen (ii) mittels
- Sulfhydryl-reaktiven Gruppen (z.B. Maleimide, Pydridyldisulfide, α -Haloacetyle, Vinylsulfone, Sulfatoalkylsulfone (bevorzugt Sulfatoethylsulfone)
- Amin-reaktive Gruppen (z.B. Succinimidylester, Carbodiimde, Hydroxymethyl-Phosphin, Imidoester, PFP-Ester etc.)
- Zucker bzw. oxidierte Zucker-reaktive Gruppen (z.B. Hydrazide etc.)
- Carboxy-reaktive Gruppen (z.B. Carbodiimide etc.)
- Hydroxyl-reaktive Gruppen (z.B. Isocyanate etc.)
- Thymin-reaktive Gruppen (z.B. Psoralen etc.)
- unselektive Gruppen (z.B. Arylazide etc.)
- photoaktivierbare Gruppen (z.B. Perfluorphenylazid etc.)
- Metallkomplexierenden Gruppen (z.B. EDTA, Hexahis, Ferritin) Antikörper und -fragmente (z.B. single-chain antibodies, F(ab)-Fragmente von Antikörpern, katalytische Antikörper).

Alternativ kann eine direkte Kopplung zwischen Wirk-/Effektstoff und der Keratin-Bindedomäne z.B. mittels Carbodiimide, Glutardialdehyd, den oben genannten oder anderen, dem Fachmann bekannten Crosslinkem durchgeführt werden.

Die erfindungsgemässen Keratin-bindenden Effektormoleküle können auch - falls gewünscht - wieder leicht vom Keratin getrennt werden. Hierzu kann beispielsweise eine Spülung mit Keratin eingesetzt werden, wodurch die Keratin-bindenden Effektormoleküle aus ihrer bestehenden Bindung zum Keratin verdrängt werden und mit dem Keratin aus der Spülunglösung abgesättigt werden. Damit ist eine reversible Anhaftung von einer Vielzahl von Effektormolekülen an Keratin möglich. Alternativ ist auch eine Spülung mit einem hohen Anteil an Detergenz (z.B. SDS) zum Abwaschen möglich.

Die erfindungsgemässen Keratin-bindenden Effektormoleküle besitzen ein weites Anwendungsgebiet in der Humankosmetik, insbesondere der Haut- und Haarpflege, der Tierpflege, der Lederpflege und Lederbearbeitung.

Bevorzugt werden die erfindungsgemässen Keratin-bindenden Effektormoleküle für die Haut-, Nagel und Haarkosmetik angewendet. Sie erlauben eine hohe Konzentration und lange Wirkdauer von haut-, nagel- und haarpflegenden oder haut-, nagel-, und haarschützenden Effektorstoffen.

Geeignete Hilfs- und Zusatzstoffe für die Herstellung von haarkosmetischen oder hautkosmetischen Zubereitungen sind dem Fachmann geläufig und können aus Handbüchern der Kosmetik, beispielsweise Schrader, Grundlagen und Rezepturen der Kosmetika, Hüthig Verlag, Heidelberg, 1989, ISBN 3-7785-1491-1 entnommen werden.

Nach einer weiteren Ausführungsform dient diese haarkosmetische oder hautkosmetische Zubereitung der Pflege oder dem Schutz der Haut oder Haars und liegt in Form einer Emulsion, einer Dispersion, einer Suspension, einer wässrigen Tensidzubereitung, einer Milch, einer Lotion, einer Creme, eines Balsams, einer Salbe, eines Gels, eines Granulats, eines Puders, eines Stiftpräparates, wie z.B. eines Lippenstifts, eines Schaums, eines Aerosols oder eines Sprays vor. Solche Formulierungen sind gut geeignet für topische Zubereitungen. Als Emulsionen kommen Öl-in-Wasser-Emulsionen und Wasser-in-Öl-Emulsionen oder Mikroemulsionen in Frage.

Im Regelfall wird die haarkosmetische oder hautkosmetische Zubereitung zur Applikation auf der Haut (topisch) oder Haar verwendet. Unter topischen Zubereitungen sind dabei solche Zubereitungen zu verstehen, die dazu geeignet sind, die Wirkstoffe in feiner Verteilung und bevorzugt in einer durch die Haut resorbierbaren Form auf die Haut aufzubringen. Hierfür eignen sich z.B. wässrige und wässrig-alkoholische Lösungen, Sprays, Schäume, Schaumaerosole, Salben, wässrige Gele, Emulsionen vom O/W- oder W/O-Typ, Mikroemulsionen oder kosmetische Stiftpräparate.

Nach einer bevorzugten Ausführungsform des erfindungsgemäßen kosmetischen Mittels enthält das Mittel einen Träger. Bevorzugt als Träger ist Wasser, ein Gas, eine Wasser-basierte Flüssigkeit, ein Öl, ein Gel, eine Emulsion oder Mikroemulsion, eine Dispersion oder eine Mischung davon. Die genannten Träger zeigen eine gute Hautverträglichkeit. Besonders vorteilhaft für topische Zubereitungen sind wässrige Gele, Emulsionen oder Mikroemulsionen.

Als Emulgatoren können nichtionogene Tenside, zwitterionische Tenside, ampholytische Tenside oder anionische Emulgatoren verwendet werden. Die Emulgatoren können in der erfindungsgemäßen Zusammensetzung in Mengen von 0,1 bis 10, vorzugsweise 1 bis 5 Gew.-%, bezogen auf die Zusammensetzung, enthalten sein.

Als nichtionogenes Tensid kann beispielsweise ein Tensid aus mindestens einer der folgenden Gruppen verwendet werden:
Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
C_{12/18}-Fettsäuremono- und -diester von Anlagerungsprodukten von 1 bis 30 Mol Ethylenoxid an Glycerin;
Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
Alkylmono- und -oligoglycoside mit 8 bis 22 Kohlenstoffatomen im Alkylrest und deren ethoxylierte Analoga;
Anlagerungsprodukte von 15 bis 60 Mol Ethylenoxid an Ricinusöl und/oder gehärtetes Ricinusöl;
Polyol- und insbesondere Polyglycerinester, wie z.B. Polyglycerinpolyricinoleat, Polygl ycerinpoly-12-hydroxystearat oder Polyglycerindimerat. Ebenfalls geeignet sind Gemische von Verbindungen aus mehreren dieser Substanzklassen;
Anlagerungsprodukte von 2 bis 15 Mol Ethylenoxid an Ricinusöl und/oder gehärtetes Ricinusöl;
Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipenta-erythrit, Zuckeralkohole (z. B. Sorbit), Alkylglucoside (z.B. Methylglucosid, Butylglucosid, Lauryl-glucosid) sowie Polyglucoside (z.B. Cellulose);
Mono-, Di- und Trialkylphosphate sowie Mono-, Di- und/oder Tri-PEG-alkylphosphate und deren Salze;
Wollwachsalkohole;
Polysiloxan-Polyalkyl-Polyether-Copolymere bzw. entsprechende Derivate;
Mischester aus Pentaerythrit, Fettsäuren, Citronensäure und Fettalkohol gemäß DE PS 1165574 und/oder Mischester von Fettsäuren mit 6 bis 22 Kohlenstoffatomen, Methylglucose und Polyolen, vorzugsweise Glycerin oder Polyglycerin sowie
Polyalkylenglycole;
Betaine.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- oder eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethyl-ammoniumglycinat, N-Acylamino-propyl-N,N dimethylammoniumglycinate, beispielsweise das Kokosacylaminopropyldimethylammonium-glycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethyl-carboxymethylglycinat. Besonders bevorzugt ist das unter der CTFA Bezeichnung Cocamidopropyl Betaine bekannte Fettsäureamid-Derivat.

Ebenfalls geeignete Emulgatoren sind ampholytische Tenside. Unter ampholytischen Tensiden werden solche oberflächenaktive Verbindungen verstanden, die außer einer C_{8,18}-Alkyl- oder -Acylgruppe im Molekül mindestens eine freie Aminogruppe und mindestens eine -COOH- oder -S0₃H-Gruppe enthalten und zur Ausbildung innerer Salze befähigt sind. Beispiele für geeignete ampholytische Tenside sind N-Alkylglycine, N-Alkylpropionsäuren , N-Alkylamino-buttersäuren, N Alkyliminodipropionsäuren, N-Hydroxyethyl-N-alkylamido-propylglycine, N-Alkyltaurine, N Alkylsarcosine, 2-Alkylaminopropionsäuren und Alkylaminoessigsäuren mit jeweils etwa 8 bis 18 C-Atomen in der Alkylgruppe.

Besonders bevorzugte ampholytische Tenside sind das N-Kokosalkyl-aminopropionat, Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methyl-quaternierte Difettsäuretriethanolaminester-Salze, besonders bevorzugt sind. Des weiteren können als anionische Emulgatoren Alkylethersulfate, Monoglyceridsulfate, Fettsäuresulfate, Sulfosuccinate und/oder Ethercarbonsäuren eingesetzt werden.

Als Ölkörper kommen Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettaikohoien, Ester von verzweigten C₆-C₁₃-Carbonsäuren mit linearen C₆-C₂₂-Fettalkoholen, Ester von linearen C₆-C₂₂-Fettsäuren mit verzweigten Alkoholen, insbesondere 2-Ethylhexanol, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-, Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare C₆-C₂₂-Fettalkoholcarbonate, Guerbetcarbonate, Ester der Benzoesäure mit linearen und/oder verzweigten C₆-C₂₂-Alkoholen (z.B. Finsolv^{®} TN), Dialkylether, Ringöffnungsprodukte von epoxidierten Fettsäureestern mit Polyolen, Siliconöle und/oder aliphatische bzw. naphthenische Kohlenwasserstoffe in Betracht. Als Ölkörper können ferner auch Silicoriverbindungen eingesetzt werden, beispielsweise Dimethylpolysiloxane, Methylphenylpolysiloxane, cyclische Silicone sowie amino-, fettsäure-, alkohol-, polyether-, epoxy-, fluor-, alkyl- und/oder glykosidmodifizierte Siliconverbindungen, die bei Raumtemperatur sowohl flüssig als auch harzförmig vorliegen können. Die Ölkörper können in den erfindungsgemäßen Mitteln in Mengen von 1 bis 90, vorzugsweise 5 bis 80, und insbesondere 10 bis 50 Gew.-%, bezogen auf die Zusammensetzung enthalten sein.

Durch Kopplung von entsprechenden Verbindungen an ein Keratin-bindendes Polypeptid (i) kann die Wirkdauer auf der Haut signifikant verlängert werden. Die Kopplung erfolgt wie oben beschrieben, Formulierung und Applikation erfolgt nach dem Fachmann bekannten Methoden. Insbesondere für Deodorantien sind geeignete Effektormoleküle (ii) : Parfumöle, Cyclodextrine, Ionenaustauscher, Zink-Ricinoleat, keimhemmende/bakteriostatische Verbindungen (z.B. DCMX, Irgasan DP 300, TCC ).

Für Antitranspirantien geeignet sind: Tannine, und Zink-/Aluminiumsalze.

Ein weiteres Anwendungsgebiet für die erfindungsgemässen Substanzen ist der therapeutische oder prophylaktische Einsatz bei bestimmten Erkrankungen der Haut und der Schleimhäute. Besonders im Mund-, Rachen- und Nasenraum ist es von Vorteil, Wirkstoffe zur Therapie/Prophylaxe über eine Keratin-Bindedomäne verstärkt und länger andauernd zu binden. Anwendungsgebiete hierfür sind insbesondere:
- virale Erkrankungen (z.B. Herpes, Coxsackie, Varicella zoster, Cytomegalovirus etc)
- bakterielle Erkrankungen (z.B. TB, Syphilis etc.)
- pilzliche Erkrankungen (z.B. Candida, Cryptococcus, Histoplasmosis, Aspergillus, Mucormycosis etc.)
- Tumorerkrankungen (z.B. Melanome, Adenome etc.)
- Autoimmunerkrankungen (z.B. PEMPHIGUS VULGARIS, BULLOUS PEMPHIGOID, SYSTEMIC LUPUS ERYTHEMATOSIS etc.)
- Sonnenbrand
- parasitärer Befall (z.B. Zecken, Milben, Flöhe etc.)
- Insektenkontakt (z.B. blutsaugende Insekten wie Anopheles etc.)
   Die zur Therapie oder Prophylaxe geeigneten Substanzen (z.B. Corticoide, immunsupprimierende Verbindungen, Antibiotika, Antimykotika, anti-virale Verbindungen, Insektenrepellent etc.) können über die oben beschriebenen Linker (je nach zu koppelnder Funktionalität ein zu optimierender Linker) an die Keratin-bindenden Polypeptide (i) gekoppelt werden.

### Beispiel 1: Expressionsvektoren und Produktionsstämme

Es wurden verschiedene Expressionsvektoren für die Expression der Keratin-bindenden Domänen (KBD) getestet. Dabei kamen verschiedene Promotoren (z.B. IPTG-induzierbar, Rhamnose-induzierbar, Arabindose-induzierbar, Methanolinduzierbar, konstitutive Promotoren, u.a.) zum Einsatz. Ebenso wurden Konstrukte getestet, bei denen die KBD als Fusionsproteine exprimiert wurden (z.B. als Fusion mit Thioredoxin, oder eGFP, oder YaaD [B. subtilis, SWISS-PROT: P37527, PDX1], u.a.). Dabei wurden sowohl die beschriebene KBD-B (Keratin-bindende Domäne B), als auch KBD-C (Keratin-bindende Domäne C), sowie die Kombination aus beiden Domänen KBD-BC mit den verschiedenen Expressionssystemen exprimiert. Die erwähnten Vektor-Konstrukte sind nicht limitierend für die Beanspruchung.

Stellvertretend als Beispiel ist die Vektorkarte des IPTG-induzierbaren Vektors pQE30-KBD-B (Abbildung 3), der Methanol-induzierbaren Vektoren pLib15 (Abbildung 4) und pLib16 (Abbildung 5) sowie des induzierbaren Vektors pLib19 (Abbildung 6) angegeben. Analog zu den beschriebenen Vektorkonstruktionen und Expressionen kann auch für KBD-C vorgegangen werden.

Für die Expression der KBD wurden verschiedene Produktionswirte genutzt, wie z.B. E. coli-Stämme (siehe Bsp. 2; z.B. XL10-Gold [Firma Stratagene], BL21-CodonPlus [Firma Stratagene], und andere). Es wurden aber auch andere bakterielle Produktionswirte, wie z.B. Bacillus megaterium oder Bacillus subtilis genutzt. Bei der KBD-Expression in B. megaterium wurde analog zu: Barg, H., Malten, M. & Jahn, D. (2005). Protein and vitamin production in Bacillus megaterium. In Methods in Biotechnology-Micobial Products and Biotransformations (Barredo, J.-L., ed.) vorgegangen. Als pilzliche Produktionsstämme kamen Pichia pastoris (siehe Bsp. 3; z.B. GS115 und KM71 [beide Firma Invitrogen]; und andere) und Aspergillus nidulans (siehe Bsp. 4; z.B. RMS011 [Stringer, MA, Dean, RA, Sewall, TC, Timberlake, WE (1991) Rodletless, a new Aspergillus developmental mutant induced by direct gene activation. Genes Dev 5:1161-1171] und SRF200 [Karos, M, Fischer, R (1999) Molecular characterization of HymA, an evolutionarily highly conserved and highly expressed protein of Aspergillus nidulans. Mol Genet Genomics 260:510-521], und andere) zum Einsatz. Es könnten aber auch andere pilzliche Produktionswirte, wie z.B. Aspergillus niger (KBD-Expression analog zu EP 0635574A1 und/oder WO 98/46772) zur KBD-Expression genutzt werden.

### Beispiel 2: KBD-Expression in E. coli-Stämmen mit IPTG induzierbaren Promotoren, z.B. durch das Expressionsplasmid pQE30-KBD-B

Für die Expression wurden verschiedene Produktionswirte eingesetzt, wie z.B. verschiedene E. coli-Stämme (z.B. XL10-Gold [Firma Stratagene], BL21-CodonPlus [Firma Stratagene], und andere), Bacillus megaterium, Bacillus subtilis u.a. Hier wird - stellvertretend als Beispiel - die Klonierung und Expression von KBD-B durch E. coli, transformiert mit pQE30-KBD-B beschrieben:

### Klonierung von pQE30-KBD-B

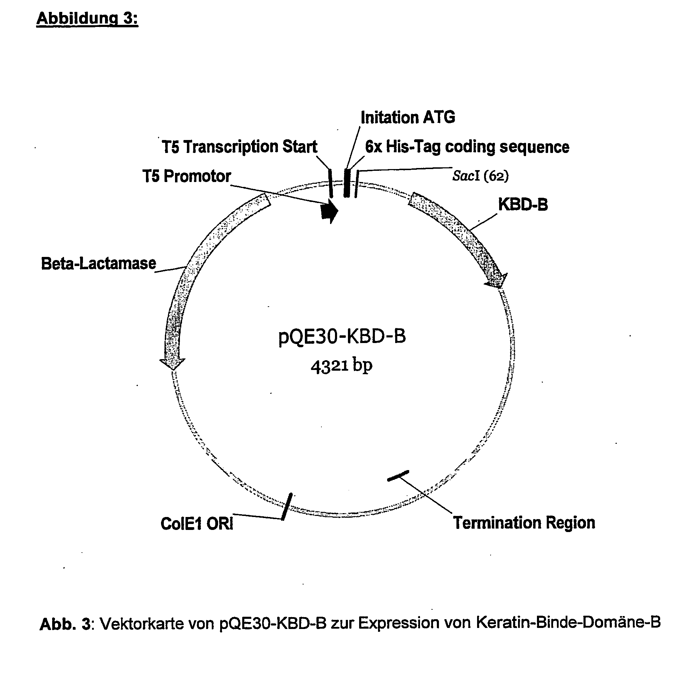

Lambda-MaxiDNA (DNA-Lambda Maxi Kit, Firma Qiagen) wurde aus einer cDNA-Bank von humanen Keratinozyten hergestellt (Firma BD Bioscience, Clontech, Human Keratinocyte cDNA, foreskin, primary culture in log phase, Vektor: λgt11).
Die PCR wurde unter Verwendung der folgenden Oligonukleotide durchgeführt:
   Bag 43 (5'- GGTCAGTTACGTGCAGCTGAAGG -3') und
   Bag 44 (5' GCTGAGGCTGCCGGATCG -3')
Das entstandene etwa 1102 bp große PCR-Produkt wurde aus einem Agarosegel ausgeschnitten und aufgereinigt.
Anschließend wurde mit dem gereinigten PCR-Produkt als Templat eine 2. PCR durchgeführt:
   Verwendete Oligonukleotide:
      Bag 53: (5'- CGCGCCTCGAGCCACATACTGGTCTGC -3') und
      Bag 51 (5'- GCTTAGCTGAGGCTGCCGGATCG -3')
Das entstandene etwa 1073 bp große PCR-Produkt wurde aus einem Agarosegel ausgeschnitten, aufgereinigt und in folgenden Vektor kloniert: pCR2.1-TOPO (Firma Invitrogen).
Der entstehende Vektor pCR2.1-TOPO+KBD-B (5027 bp) wurde anschließend transformiert, amplifiziert in E. coli, dann mit Xhol und EcoRI geschnitten und das entstandene KBD-B-Fragment in pBAD/HisA (Firma Invitrogen; ebenfalls geschnitten mit Xhol und EcoRI) kloniert.
Der neu entstandene Vektor pBAD/HisA+KBD-B (5171 bp) wurde erneut geschnitten mit Sacl und Stul und das entstandene KBD-B-Fragment in pQE30 (Firma Qiagen; geschnitten mit Sacl und Smal) kloniert. Der daraus entstandene Expressionsvektor pQE30-KBD-B (4321 bp; siehe auch Abbildung 3) wurde für die folgenden KBD-B-Expressionen verwendet.

Die durch den Vektor pQE30-KBD-B in E. coli expremierte KBD-B beinhaltete neben der Polypeptidsequenz SEQ ID NO: 1 Position 2193-2481 zusätzlich am N-Terminus die Aminosäuren MRGSHHHHHHGSACEL sowie am C-Terminus die Aminosäuren GVDLQPSLIS.

### Expression von KBD-B durch pQE30-KBD-B in E. coli

Vorkulturen wurden von Platte oder Glycerinkultur mit pQE30-KBD-B transformierten E. coli Stämmen (z.B. XL10-Gold [Firma Stratagene]) angeimpft. Je nach Größe der Hauptkultur wurde in einem Röhrchen oder einem kleinen Kolben mit LB-Medium angeimpft (ca. 1:100).
Antibiotika wurden je nach verwendetem Stamm eingesetzt (für pQE30-KBD-B Ampicillin 100 µg/ml).
Es wurde bei 250 rpm und 37°C inkubiert.
Die Hauptkultur wurde ca. 1:100 mit Vorkultur angeimpft, Hauptkultur: LB-Medium oder geeignetes Minimalmedium mit den jeweiligen Antibiotika. Inkubation bei 250 rpm und 37°C.
Die Induktion erfolgte mit 1 mM IPTG ab einer OD(600nm) von 0,5.
Die Zellen wurden nach 4 h Induktion abzentrifugiert.

In Fermentern wurde analog vorgegangen, jedoch konnte bei sehr viel höheren OD-Einheiten induziert werden und damit die Zell- und Protein-Ausbeute erheblich gesteigert werden.

### Beispiel 3: Intrazelluläre und sekretorische Expression von KBD mittels Pichia pastoris-Stämmen unter Verwendung von Methanol-induzierbaren Promotoren, z.B. durch die Expressionsplasmide pLib 15 und pLib 16 (Schüttelkolben)

Für die KBD-Expression wurden verschiedene Pichia pastoris-Stämme eingesetzt, wie z.B. GS115 und KM71 (Pichia Expression Kit, Version M; Invitrogen Life Technologies).

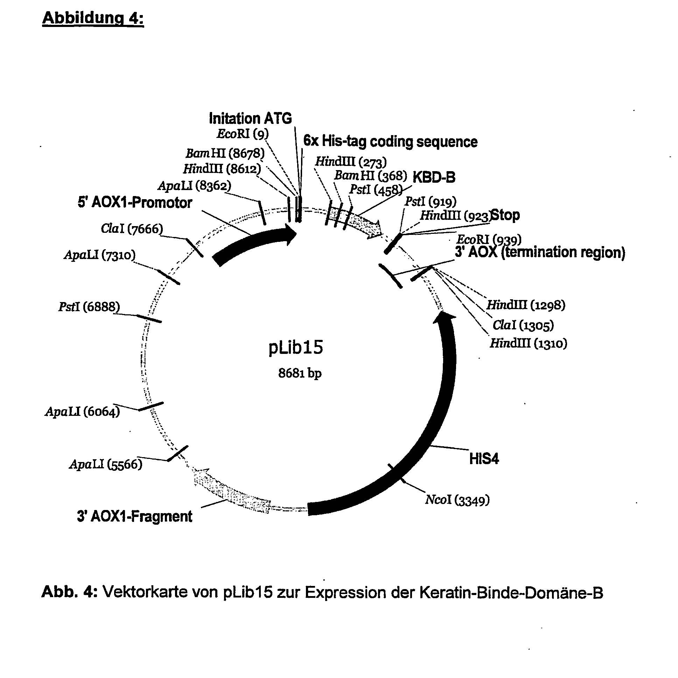

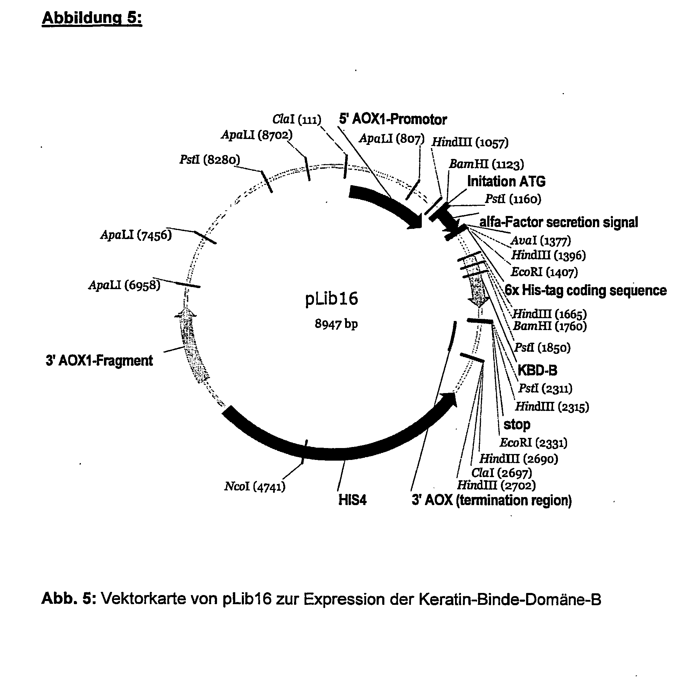

Hier wird - stellvertretend als Beispiel - die Expression von KBD-B durch P. pastoris, transformiert mit pLib15 (intrazelluläre Expression, Vektor siehe Abbildung 4) oder pLib16 (sekretorische Expression, Vektor siehe Abbildung 5) beschrieben.
Zur Konstruktion von pLib15 wurde ein etwa 930 bp großes, KBD-B-kodierendes DNA-Fragment mittels PCR unter Verwendung der Oligonukleotide Lib148 (5'-GCTAAGGAATTCACCATGCATCACCATCACCATCACGAGCCACA-TACTGGTCTGCT-3') und Lib149
(5'-GCTGGAGAATTCTCAGCTAATTAAGCTTGGCTGCA-3') sowie des Vektors pQE30-KBD-B (Beispiel 2, Abb. 3) als Template amplifiziert. Dabei wurden EcoRI-Restriktionsschnittstellen an beiden Enden der PCR-Produkte eingebracht.
Zur Konstruktion von pLib16 wurde ein etwa 930 bp großes, KBD-B-kodierendes DNA-Fragment mittels PCR unter Verwendung der Oligonukleotide Lib149 (5'-GCTGGAGAATTCTCAGCTAATTAAGCTTGGCTGCA-3') und Lib150 (5'-GCTAAGGAATTCCATCACCATCACCATCACGAGCCACATACTGGTCTGCT-3') sowie des Vektors pQE30-KBD-B (Beispiel 2, Abbildung 3) als Template amplifiziert. Dabei wurden EcoRI-Restriktionsschnittstellen an beiden Enden der PCR-Produkte eingebracht.
Das PCR-Produkt, welches mit den Oligonukleotiden Lib148/Lib149 amplifiziert wurde, wurde mit EcoRI verdaut und in den EcoRI-geschnittenen Vektor pPIC3.5 (Pichia Expression Kit, Version M, Firma Invitrogen) ligiert. Die korrekte KBD-B Amplifizierung wurde durch Sequenzierung des aus der Ligation resultierenden Vektors pLib15 (Abbildung 4) überprüft.
Das PCR-Produkt, welches mit den Oligonukleotiden Lib149/Lib150 amplifiziert wurde, wurde mit EcoRI verdaut und in den EcoRI-geschnittenen Vektor pPIC9 (Pichia Expression Kit, Version M, Firma Invitrogen) ligiert. Die korrekte KBD-B Amplifizierung wurde durch Sequenzierung des aus der Ligation resultierenden Vektors pLib16 (Abbildung 5) überprüft.
Elektrokompetente Zellen und Spheroplasten der P. pastoris-Stämme wurden mit den zirkulären und Stul-linearisierten Vektoren pLib15 bzw. pLib16 transformiert.
Die Analyse der Transformanten erfolgte mittels PCR und Southern Blot unter Verwendung chromosomaler DNA.
Zur Vorkultur wurden KBD-B-expremierende P. pastoris-Transformanten von Platte oder Glycerinkultur angeimpft. Je nach Größe der Hauptkultur wurde ein Röhrchen oder ein kleiner Kolben mit MGY-, BMG- oder BMGY-Medium (Pichia-Expression-Kit, Version M, Firma Invitrogen) angeimpft (ca. 1:100).
Die Kultur wurde bei 250-300 rpm und 30°C bis zur OD₆₀₀=2-6 inkubiert.
Die Zellen wurden mit 1500-3000 × g für 5 min bei Raumtemperatur geerntet.
Zur Hauptkultur wurde das geerntete Zellpellet auf eine OD₆₀₀=1 in Methanolenthaltendem mM-, BMM- oder BMMY-Medium (Pichia-Expression-Kit, Version M, Firma Invitrogen) aufgenommen, um die Expression zu induzieren.
Die Inkubation der Hauptkultur erfolgte bei 250-300 rpm und 30°C für 1-96 h.
Die Aufrechterhaltung der Induktion erfolgte alle 24 h durch Zugabe von 100 % Methanol bei einer Methanol-Endkonzentration von 0,5 %.
Bei intrazellulärer Expression erfolgte die Ernte und der Aufschluss der Zellen nach Ende der Hauptkultur mittels eines Menton-Gaulin.
Bei sekretorischer Expression wurde der Kulturüberstand gesammelt und die KBD-B direkt daraus gereinigt.
Die intrazellulär in P. pastoris expremierte KBD-B (pLib15) beinhaltete neben der Polypeptidsequenz SEQ ID NO: 1 Position 2193-2481 zusätzlich am N-Terminus die Aminosäuren MHHHHHH sowie am C-Terminus die Aminosäuren GVDLQPLIS.
Die sekretorisch in P. pastoris expremierte KBD-B (pLib16) beinhaltete vor der Prozessierung neben der Polypeptidsequenz SEQ ID NO: 1 Position 2193-2481 zusätzlich am N-Terminus die Aminosäuren MRFPSIFTAVLFAASSALAAPVNTT-TEDETAQIPAEAVIGYSDLEGDFDVAVLPFSNSTNNGLLFINTTIASIAAKEEGVS-LEKREAEAYVEFHHHHHH sowie am C-Terminus die Aminosäuren GVDLQPLIS. Die mittels P. pastoris sekretierte und prozessierte KBD-B (pLib16) beinhaltete neben der Polypeptidsequenz SEQ ID NO: 1 Position 2193-2481 zusätzlich am N-Terminus die Aminosäuren YVEFHHHHHH sowie am C-Terminus die Aminosäuren GVDLQPLIS.

### Beispiel 4: Expression von KBD mittels Aspergillus nidulans-Stämmen unter Verwendung des induzierbaren alcA-Promotors, z.B. durch das Expressionsplasmid pLib 19 (Schüttelkolben)

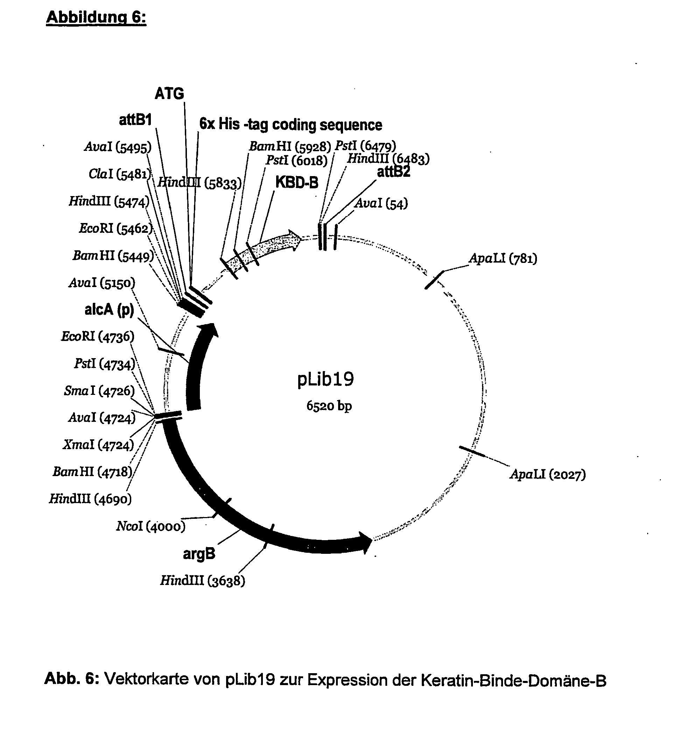

Für die Expression wurden A. nidulans-Wildtypstämme eingesetzt, wie z.B. RMS011 oder SRF200. Hier wird - stellvertretend als Beispiel - die Expression von KBD-B durch A. nidulans, transformiert mit pLib19 (Abbildung 6) beschrieben.
Zur Konstruktion von pLib19 wurde ein etwa 900 bp großes, KBD-B-kodierendes DNA-Fragment mittels PCR unter Verwendung der Oligonukleotide Lib151 (5'-CACCATGCATCACCATCACCATCACGAGCCACATACTGGTCTGCT-3') und Lib152 (5'- GCTAATTAAGCTTGGCTGCA-3') sowie des Vektors pQE30-KBD-B (Beispiel 2, Abbildung 3) als Template amplifiziert. Das PCR-Produkt wurde in den Vektor pENTR/D (pENTR^{™} Directional TOPO^{®} Cloning Kit, Version E, Firma Invitrogen) ligiert. Die korrekte KBD-B Amplifizierung wurde durch Sequenzierung überprüft.
Die Rekombination des KBD-B kodierenden DNA-Fragmentes erfolgte in den Vektor pMT-OvE (Toews MW, Warmbold J, Konzack S, Rischitor P, Veith D, Vienken K, Vinuesa C, Wei H, Fischer R; Establishment of mRFP1 as a fluorescent marker in Aspergillus nidulans and construction of expression vectors for high-throughput protein tagging using recombination in vitro (GATEWAY). (2004) Curr Genet 45: 383-389) unter Verwendung des "Gateway^{®} LR clonase^{™} enzyme mix" (Firma Invitrogen). Dabei entstand der Vektor pLib19 (Abbildung 6).
Protoplasten der A. nidulans Wildtyp-Stämme wurden mit dem zirkulären Vektor pLib19 transformiert. Die Analyse der Transformanten erfolgte mittels PCR und Southern Blot unter Verwendung chromosomaler DNA.
Zur Vorkultur von KBD-B-expremierenden A. nidulans-Transformanten wurden 100 ml Minimalmedium (0,6 % NaNO₃; 0,152 % KH₂PO₄; 0,052 % KCl [pH 6,5]; 0,8 % Glukose; 0,05 % MgSO₄; 1 ml Spurenelementelösung [1 g/l FeSO₄ × 7 H₂O; 8,8 g/l ZnSO₄ × 7 H₂O; 0,4 g/l CuSO₄ × 5 H₂O; 0,15 g/l MnSO₄ × 4 H₂O; 0,1 g/l Na₂B₄O₇ × 10 H₂O; 0,05 g/l (NH₄)₆Mo₇O₂₄ × 4 H₂O], + stammspezifische Supplemente) oder 100 ml Komplettmedium (2 % Malzextrakt; 0,1 % Pepton; 2 % Glukose; + stammspezifische Supplemente) in 500 ml Kolben mit 10⁶-10⁷ Sporen angeimpft und für 16-24 h bei 200-250 rpm und 37°C inkubiert.
Nach der Vorkultur wurde das Pilzmyzel durch Filtration geerntet, mit destilliertem Wasser gewaschen und in Kolben mit 100-500 ml frischem Minimalmedium überführt. In diesem Hauptkulturmedium wurde 0,1 % Fructose statt Glukose als C-Quelle verwendet. Zur Induktion der KBD-Expression wurde dem Medium zusätzlich Ethanol (1 % Endkonzentration) oder Glycerol (50 mM) oder Natriumacetat (50 mM) oder Ethylamin oder Threonin zugegeben. Die erwähnten Zusätze zur Expressionsinduktion sind nicht limitierend für die Beanspruchung. Die Hauptkultur wurde für weitere 5-48 h bei 200-250 rpm und 37°C inkubiert.
Nach Kulturende wurde das Pilzmyzel mit 1500-3000 × g für 5 min bei Raumtemperatur geerntet und mittels eines Menton-Gaulin aufgeschlossen.
Die in A. nidulans expremierte KBD-B (pLib19) beinhaltete neben der Polypeptidsequenz SEQ ID NO: 1 Position 2193-2481 zusätzlich am N-Terminus die Aminosäuren MHHHHHH sowie am C-Terminus die Aminosäuren KGGRAD-PAFLYKVVMIRLLTKPERKLLEGGPGTQLLFPLVRVNCALGVIMVI-AVSCVKLLSAHNSTQHTSRKHKV.

### Beispiel 5: Zell-Aufschluss und Inclusion-Body-Reinigung (pQE30-KBD-B)

Löslich exprimierte KBD konnte nach Reinigung direkt verwendet werden. Unlöslich exprimierte KBD (z.B. in Inclusion Bodies) wurde folgendermaßen gereinigt:
Der Fermenter wurde zentrifugiert, das Pellet in 20 mM Phosphatpuffer pH = 7,4 suspendiert und mittels eines Menton-Gaulin aufgeschlossen.
Der Aufschluss wurde erneut zentrifugiert (15000g), das Pellet hiervon mit 20 mM Phosphat, 500 mM NaCl und 8 M Harnstoff versetzt und so gerührt. (Lösen der Inclusion-Bodies)
Der pH-Wert des Überstandes wurde auf 7,5 eingestellt
Danach wurde nochmals zentrifugiert und der Überstandes auf eine Ni-Chelat Sepharose Säule aufgetragen.

### Beispiel 6: Reinigung von Keratin-Binde-Domäne B über Ni-Chelat-Sepharose

Die Reinigung der KBD konnte durch das angehängte His-Tag über eine Ni-Säule chromatographisch gereinigt werden.

### Säulenmaterial: Ni-Sepharose High Performance Firma Amersham Biosciences Best. Nr.: 17-5268-02

Das Material wurde in eine Säule gepackt (z.B. Durchmesser 2,6 cm, Höhe 5 cm) und mit Puffer A + 4 % Puffer B (entspricht 20mM Imidazol) äquilibriert.

Der Proteinextrakt (siehe z.B. Zell-Aufschluss und Inclusion-Body-Reinigung) wurde mit pH 7,5 über einen Superloop (ÄKTA-System) auf die Säule auftragen (Flow ca. 5 ml/Min).

Nach dem Auftrag wurde mit Puffer A + 20mM Imidazol gewaschen.
Elution erfolgte mit Puffer B (500mM Imidazol in Puffer A).
Das Eluat wurde mittels eines Fraktionssammlers fraktioniert aufgefangen.
Puffer A:
   20 mM Natriumdihydrogenphosphat
   500 mM NaCl
   8M Harnstoff
   pH = 7,40
Puffer B:
   20mM Natriumdihydrogenphosphat
   500mM NaCl
   8M Harnstoff
   500mM Imidazol
   pH = 7,40

### Beispiel 7: Renaturierung von Keratin-Binde-Domäne B

Unlöslich exprimierte Keratin-Binde-Domäne (z.B. aus Inclusion Bodies) kann folgendermaßen renaturiert und damit aktiviert werden:

### Methode 1: diskontinuierliche Dialyse

Zu 6,5 ml KBD-B-Inclusion-Bodies in 8M Harnstoff (Ni-Chelat-Eluat, HiTrap) wurden 6,5 ml Cellytic IB (Firma Sigma, Bestellnr. C5236) und 5 mM DTT gegeben. Danach wurde die zu renaturierende Lösung in einen Dialyseschlauch gefüllt (Firma Spectrum: Spectra Por MWCO:12-14kD).

Ca. 12 Stunden gegen 1 L 6M Harnstofflsg. bei 4°C unter vorsichtigem Rühren dialysieren.

Es wurden 500 ml 25 mM Tris/HCl pH = 7,50 zugegeben und so für 9 Stunden bei 4°C dialysiert. Anschließend Zugabe von weiteren 250 ml des Trispuffers (s.o) und weitere 12 Stunden dialysiert.

Anschließend wurden erneut 500 ml 25 mM Tris/HCl pH = 7,50 zugegeben und so für 9 Stunden bei 4°C dialysiert. Anschließend Zugabe von weiteren 250 ml des Trispuffers (s.o.) und weitere 12 Stunden dialysiert.

Anschließend wurden erneut 500 ml 25 mM Tris/HCl pH = 7,50 zugegeben und so für 9 Stunden bei 4°C dialysiert. Dann wurde der Dialyseschlauch mit dem Dialysat in 2L: 25 mM Tris + 150 mM NaCl pH= 7,50 gegeben. So wurde erneut bei 4°C für 12 Stunden dialysiert.

Anschließend wurde der Inhalt des Dialyseschlauches entnommen.

### Methode 2: kontinuierliche Dialyse

20 ml KBD-B-Inclusion-Bodies in 8 M Harnstoff (Ni-Chelat-Eluat, HiTrap) wurden mit 10 ml Cellytic IB (Firma Sigma, Bestellnr. C5236) und 5 mM DTT versetzt. Danach wurde die Lösung in eine Dialysekammer: Slide-A-Lyzer Dialyses Cassette Firma PIERCE, MWCO: 10 kD. Bestellnr.: 66830, eingefüllt.

Anschließend wurde für ca. 1 Stunde gegen 1 L 6M Harnstofflsg. bei 4°C dialysiert.

Danach wurden über einen Zeitraum von 48 h kontinuierlich 2 I des folgenden Puffers mittels einer Schlauchpumpe zudosiert: 25 mM Tris/HCl pH = 7,5.

Anschließend wurde der Dialyseschlauch mit dem Dialysat in 2 L des Endpuffers gegeben:
25 mM Tris + 150 mM NaCl pH= 7,50 und für ca. 12 Stunden bei 4°C dialysiert.

Anschließend wurde der Inhalt des Dialyseschlauches entnommen.

### Beispiel 8: Bindung an Haut 1 (Qualitativ)

Es wurde ein visueller qualitative Test entwickelt, um zu überprüfen, ob KBD an Haut bindet.

### Verwendete Lösungen:

Blockierungslsg: DIG Wash+Bufferset 1585762 Boehringer MA (10xLsg) in TBS verdünnt
TBS: 20mM Tris; 150mM NaCl pH 7,5
TTBS: TBS + 0,05% Tween20

Der erste Schritt ist der Transfer der äußeren Keratinschicht von der Haut auf einen stabilen Träger. Dazu wurde ein Klarsichtklebestreifen fest auf enthaarte menschliche Haut aufgebracht und wieder entfernt. Der Test kann direkt auf dem Klarsichtklebestreifen durchgeführt werden oder die anhaftende Keratinschicht durch erneutes Aufkleben auf einen Glasobjektträger überführt werden. Der Nachweis von Bindung wurde wie folgt vorgenommen:
zur Inkubation mit den verschiedenen Reagentien, Transfer in ein Falcongefäß
ggf. Zugabe von Ethanol zur Entfettung, Entfernung von Ethanol und Trocknung der Objektträger
1 h bei Raumtemperatur inkubiert mit Blocking Puffer
2x 5 min gewaschen mit TTBS
1x 5 min gewaschen mit TBS
Inkubation mit der zu testenden KBD (gekoppelt an tag - z.B. His₆, HA etc.) bzw. Kontrollprotein in TBS / 0,05% Tween 20 während 2-4 h bei Raumtemperatur
Entfernung des Überstands
3x Waschen mit TBS
1 h bei Raumtemperatur Inkubation mit Monoclonal Anti-polyHistidin (oder spezifischen KBD Rabbit) Antikörper, verdünnt 1:2000 in TBS + 0,01% Blocking
2x 5min gewaschen mit TTBS
1x 5 min gewaschen mit TBS
1 h bei Raumtemperatur Inkubation mit Anti-Mouse IgG Alkalische-Phosphatase-Conjugate, verdünnt 1:5000 in TBS + 0,01 % Blocking
2x 5 min gewaschen mit TTBS
1x 5 min gewaschen mit TBS
Zugabe von Phosphatasesubstrat (NBT-BCIP; Boehringer MA 1Tablette/40 ml Wasser 2,5 min; Stopp: mit Wasser)
Optische Detektion des Farbniederschlages mit bloßem Auge oder im Mikroskop. Ein blauer Farbniederschlag zeigt an, dass KBD an die Haut gebunden hat.

### Beispiel 9: Bindung an Haut 2 (Quantitativ)

Es wurde ein quantitativer Test entwickelt, mit dem sich die Haar/Haut-Bindungsstärke der KBD mit unspezifischen Proteinen vergleichen lässt.

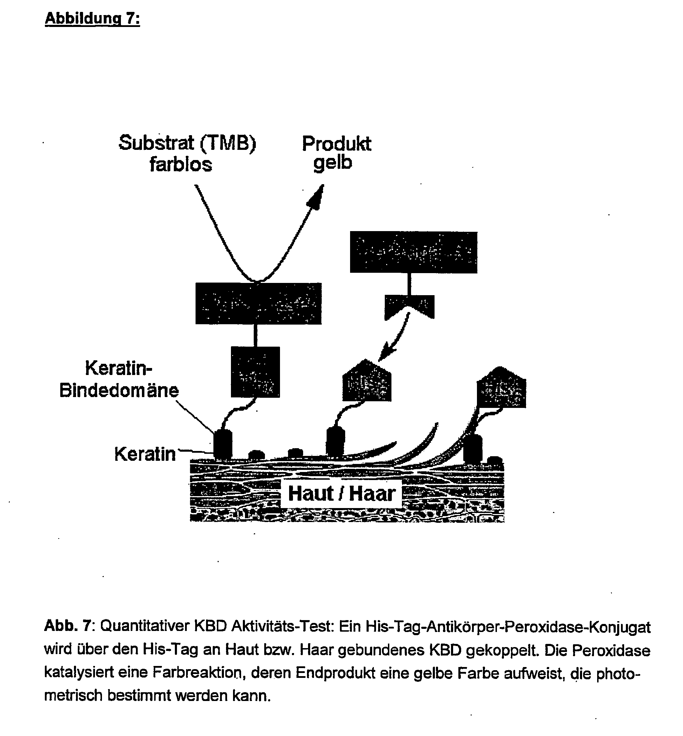

Aus einem aufgetauten trockenem Stück Haut ohne Haare (human oder Schwein), wurde mit einem 5 mm Korkbohrer ein Stück herausgebohrt (bzw. bei einem Oberflächentest ein Stück Haut in ein Falcondeckel eingepasst). Die Hautprobe wurde dann auf eine Dicke von 2-3 mm gebracht um evt. vorhandenes Gewebe zu entfernen. Die Hautprobe wurde anschließend in ein Eppendorfgefäß (Protein-Lowbind) überführt, um den Bindungsnachweis durchzuführen (siehe auch Abbildung 7):
2 x Waschen mit PBS / 0,05 % Tween 20
Zugabe von 1 ml 1 % BSA in PBS und Inkubation während 1 h bei Raumtemperatur, leichte Schwenkbewegungen (900 rpm).
Entfernung des Überstands
Zugabe von 100 µg KBD in PBS+0,05 % Tween 20; Inkubation 2 h bei Raumtemperatur und leichten Schwenkbewegungen (900 rpm).
Entfernung des Überstands
3x Waschen mit PBS / 0,05 % Tween 20
Inkubation mit 1 ml monoklonalen Maus anti-tag-(His6 bzw. HA oder spezifischen KBD)-Antikörper mit Peroxidase Conjugate (1:2000 in PBS / 0,05 % Tween 20) [Monoclonal AntipolyHistidin Peroxidase Conjugate, produced in mouse, lyophilized powder, Firma Sigma] während 2-4 h bei Raumtemperatur, leichte Schwenkbewegung (900 rpm)
3x Waschen mit PBS / 0,05 % Tween 20
Zugabe von Peroxidasesubstrat (1 ml / Eppendorfgefäß; Zusammensetzung s.u.)
Reaktion bis zur Blaufärbung laufen lassen (ca. 1:30 Minuten).
Mit 100 µl 2 M H₂SO₄ die Reaktion abstoppen.
Die Absorption wurde bei 405 nm gemessen
   Peroxidasesubstrat (kurz vorher ansetzten):
   0,1 ml TMB-Lösung (42 mM TMB in DMSO)
   + 10 ml Substratpuffer (0,1 M Natriumacetat pH 4,9)
   + 14,7 µl H₂O₂ 3%ig

### Beispiel 10: Bindung an Haar (Quantitativ)

Um die Bindungsstärke der KBD an Haar auch im Vergleich zu anderen Proteinen nachweisen zu können, wurde ein quantitativer Assay entwickelt (siehe auch Abbildung 7). Bei diesem Test wurde zunächst Haar mit KBD inkubiert und überschüssige KBD abgewaschen. Anschließend wurde eine Antikörper-Peroxidase-Konjugat über das His-Tag der KBD gekoppelt. Nicht gebundene Antikörper-Peroxidase-Konjugat wurde erneut abgewaschen. Das gebundene Antikörper-Peroxidase-Konjugat [Monoclonal AntipolyHistidin Peroxidase Conjugate, produced in mouse, lyophilized powder, Firma Sigma] kann ein farbloses Substrat (TMB) in ein farbiges Produkt umsetzen, das photometrisch bei 405 nm vermessen wurde. Die Stärke der Absorption zeigt die Menge an gebundenem KBD bzw. Vergleichsprotein an. Als Vergleichsprotein wurde z.B. Y-aaD aus B. subtilis gewählt, das ebenfalls - wie es für diesen Test nötig ist - ein His-Tag zur Detektion aufwies. Anstatt des His-Tag können auch andere, spezifische Antikörper konjugiert mit Peroxidase verwendet werden.

5 mg Haare (human) werden in 5 mm lange Stücke geschnitten und in Eppendorfgefäße (Protein-Lowbind) überführt, um den Bindungsnachweis durchzuführen:
Zugabe von 1 ml Ethanol zur Entfettung
Zentrifugation, Entfernung von Ethanol und Waschung der Haare mit H₂O
Zugabe von 1 ml 1% BSA in PBS und Inkubation während 1 h bei Raumtemperatur, leichte Schwenkbewegungen.
Zentrifugation, Entfernung des Überstands
Zugabe der zu testenden Keratinbindedomäne (gekoppelt an tag -z.B. His₆, HA etc.) bzw. Kontrollprotein in 1 ml PBS / 0,05 % Tween 20; Inkubation für 16 h bei 4°C (oder mindestens 2 h bei Raumtemperatur) bei leichten Schwenkbewegungen.
Zentrifugation, Entfernung des Überstands
3x Waschen mit PBS / 0,05 % Tween 20
Inkubation mit 1 ml monoklonalen Maus anti-tag-(His6 bzw. HA)-Antikörper mit Peroxidase-Konjugat (1:2000 in PBS / 0,05 % Tween 20) [Monoclonal AntipolyHistidin Peroxidase Conjugate, produced in mouse, lyophilized powder, Firma Sigma] während 2-4 h bei Raumtemperatur, leichte Schwenkbewegung 3 x Waschen mit PBS / 0,05 % Tween 20
Zugabe von Peroxidasesubstrat (1 ml / Eppendorfgefäß)
Reaktion bis zur Blaufärbung laufen lassen (ca. 2 Minuten).
Mit 100 µl 2 M H₂SO₄ die Reaktion abstoppen.
Die Absorption wird bei 405 nm gemessen

Peroxidasesubstrat (kurz vorher ansetzten):
0,1 ml TMB-Lösung (42 mM TMB in DMSO)
+ 10 ml Substratpuffer (0,1 M Natriumacetat pH 4,9)
+ 14,7 µl H₂O₂ 3%ig

BSA = Bovine serum albumin
PBS = Phosphat gepufferte Salzlösung
Tween 20 = Polyoxyethylene sorbitan monolaureate, n ca. 20
TMB = 3,5,3,'5' Tetramethylbenzidin

Ein beispielhaft für KBD-B durchgeführter Bindungs-Test an Haar zeigte eine deutliche Überlegenheit der Bindung von KBD-B an Haar gegenüber einer wesentlichen schlechteren Bindung des Vergleichsproteins YaaD:

**Tabelle 1.:**

| | | |
|---|---|---|
| 1 | Puffer | A_{405 nm} = 0,000 |
| 2 | Vergleichsprotein YaaD | A_{405 nm} = 0,088 |
| 3 | KBD-B denaturiert | A_{405 nm}= 0,254 |
| 4 | KBD-B renaturiert | A_{405 nm} = 1,591 |

| | | |
|---|---|---|
| Quantitativer KBD Aktivitäts-Test Haar: 1) Puffer; 2) Vergleichsprotein Y-aaD; 3) KBD-B denaturiert; 4) KBD-B renaturiert. Die Tabelle zeigt die gemessenen Absorptionswerte bei 405 nm. | | |

### Beispiel 10a: Kopplung eines Farbstoffes an die KBD-B

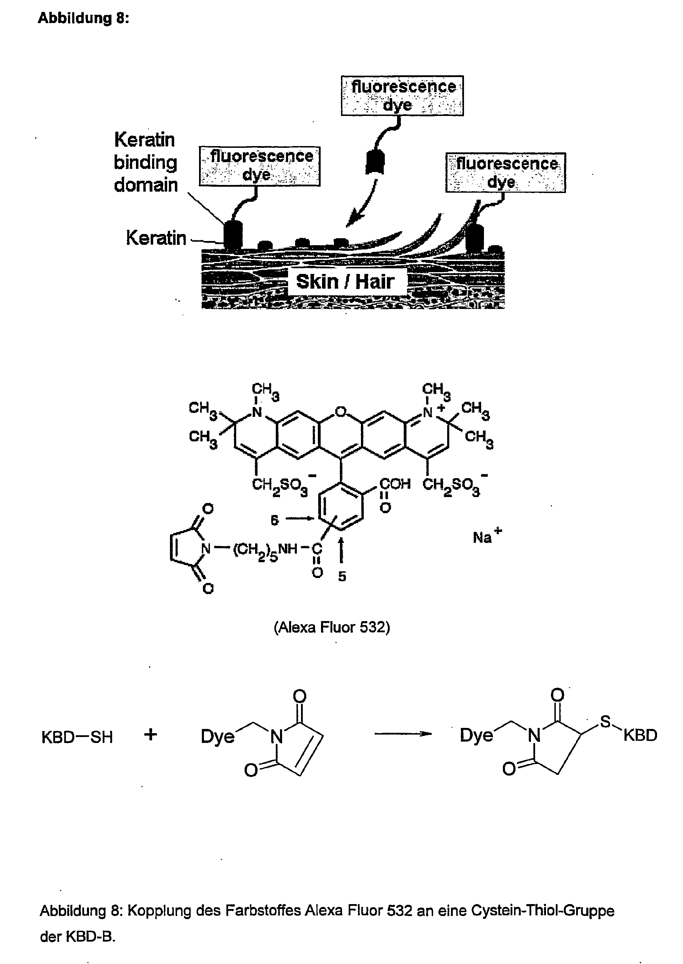

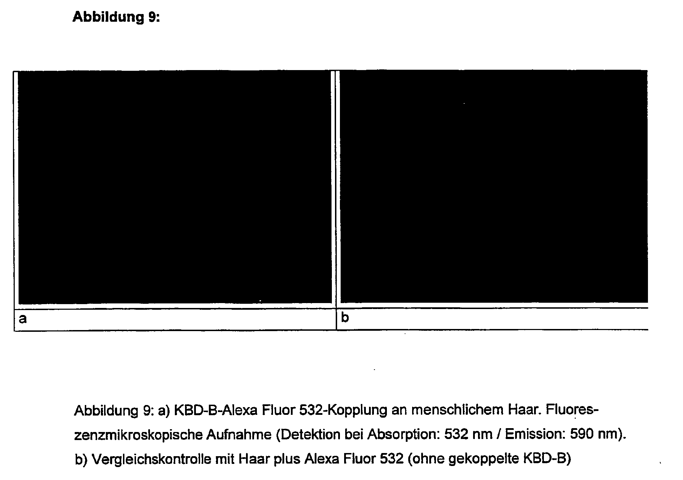

Um einen Fluoreszenz-Farbstoff (Alexa Fluor 532, Firma Molecular Probes / Invitrogen) an das Protein KBD-B zu koppeln, wurde der Farbstoff über einen Maleinsäurediimid-Linker an eine Cystein-Thiolgruppe nach folgendem Protokoll gekoppelt. Die Reaktion ist in Abbildung 8 dargestellt.
- 1 mg Alexa Fluor 532 wurde in 150 µl PBS-Puffer pH 7.0 gelöst; anschließend wurde kurz zentrifugiert, um evt. nicht gelöste Bestandteile abzutrennen
- Zu 100 µg KBD-B (1 mg/ml) wurden 10 µl gelöster Farbstoff gegeben
- Der Ansatz wurde mit Alufolie abgedeckt 1 Stunde bei 24° C auf dem Schüttler bei 450 rpm inkubiert
- Es wurden 10µl 1 M DTT dazugegeben, um die Maleinsäurediimid-Funktion von nicht umgesetzten Alexa Fluor 532 zu inaktivieren
- Anschließend wurde 30 Minunten bei 24° C (mit Alufolie abgedeckt) bei 450 rpm inkubiert

Die Kopplung der KBD-B mit gekoppeltem Alexa Fluor 532 an Haut / Haar kann durch einen Aktivitätstest (siehe Beispiel 9 und 10) bestimmt werden. Die analog Beispiel 9 bzw. 10 an Haut oder Haar gebundene KBD-B-Alexa Fluor 532-Kopplung lässt sich sehr gut an Haar im Fluoreszenzmikroskop (Detektion bei Absorption: 532 nm / Emission: 590 nm bzw. auch mit bloßem Auge an blondiertem Haar nachweisen.

### Beispiel 11: Verwendung der KBD in einer Emulsion zur Tagespflege - Typ O/W

| WS 1%: | | |
|---|---|---|
| | % | Inhaltsstoff (INCl) |
| A | 1,7 | Ceteareth-6, Stearyl Alcohol |
| | 0,7 | Ceteareth-25 |
| | 2,0 | Diethylamino Hydroxybenzoyl Hexyl Benzoate |
| | 2,0 | PEG-14 Dimethicone |
| | 3,6 | Cetearyl Alcohol |
| | 6,0 | Ethylhexyl Methoxycinnamate |
| | 2,0 | Dibutyl Adipate |
| B | 5,0 | Glycerin |
| | 0,2 | Disodium EDTA |
| | 1,0 | Panthenol |
| | q.s. | Konservierungsmittel |
| | 67,8 | Aqua dem. |
| C | 4,0 | Caprylic/Capric Triglyceride, Sodium Acrylates Copolymer |
| D | 0,2 | Sodium Ascorbyl Phosphate |
| | 1,0 | Tocopheryl Acetate |
| | 0,2 | Bisabolol |
| | 1,0 | Caprylic/Capric Triglyceride, Sodium Ascorbate, Tocopherol, Retinol |
| | 1,0 | wässrige Lösung mit ca. 5% Keratin-Bindedomäne-Wirkstoff |
| E | q.s. | Sodium Hydroxide |

| WS 5%: | | |
|---|---|---|
| | % | Inhaltsstoff (INCl) |
| A | 1,7 | Ceteareth-6, Stearyl Alcohol |
| | 0,7 | Ceteareth-25 |
| | 2,0 | Diethylamino Hydroxybenzoyl Hexyl Benzoate |
| | 2,0 | PEG-14 Dimethicone |
| | 3,6 | Cetearyl Alcohol |
| | 6,0 | Ethylhexyl Methoxycinnamate |
| | 2,0 | Dibutyl Adipate |
| B | 5,0 | Glycerin |
| | 0,2 | Disodium EDTA |
| | 1,0 | Panthenol |
| | q.s. | Konservierungsmittel |
| | 63,8 | Aqua dem. |
| C | 4,0 | Caprylic/Capric Triglyceride, Sodium Acrylates Copolymer |
| D | 0,2 | Sodium Ascorbyl Phosphate |
| | 1,0 | Tocopheryl Acetate |
| | 0,2 | Bisabolol |
| | 1,0 | Caprylic/Capric Triglyceride, Sodium Ascorbate, Tocopherol, Retinol |
| | 5,0 | wässrige Lösung mit ca. 5% Keratin-Bindedomäne-Wrkstoff |
| E | q.s. | Sodium Hydroxide |

Herstellung: Die Phasen A und B getrennt voneinander auf ca. 80°C erwärmen. Phase B in Phase A einrühren und homogenisieren. Phase C in die kombinierten Phasen A und B einrühren und nochmals homogenisieren. Unter Rühren auf ca. 40°C abkühlen, Phase D zugeben, den pH-Wert mit Phase E auf etwa 6.5 einstellen, homogenisieren und unter Rühren auf Raumtemperatur abkühlen.

Hinweis: Die Formulierung wird ohne Schutzgas hergestellt. Die Abfüllung muß in sauerstoffundurchlässige Verpackungen, z.B. Aluminiumtuben erfolgen.

### Beispiel 12: Verwendung der KBD in einer schützenden Tagescreme - Typ O/W

| WS 1 %: | | |
|---|---|---|
| | % | Inhaltsstoff (INCl) |
| A | 1,7 | Ceteareth-6, Stearyl Alcohol |
| | 0,7 | Ceteareth-25 |
| | 2,0 | Diethylamino Hydroxybenzoyl Hexyl Benzoate |
| | 2,0 | PEG-14 Dimethicone |
| | 3,6 | Cetearyl Alcohol |
| | 6,0 | Ethylhexyl Methoxycinnamate |
| | 2,0 | Dibutyl Adipate |
| B | 5,0 | Glycerin |
| | 0,2 | Disodium EDTA |
| | 1,0 | Panthenol |
| | q.s. | Konservierungsmittel |
| | 68,6 | Aqua dem. |
| C | 4,0 | Caprylic/Capric Triglyceride, Sodium Acrylates Copolymer |
| D | 1,0 | Sodium Ascorbyl Phosphate |
| | 1,0 | Tocopheryl Acetate |
| | 0,2 | Bisabolol |
| | 1,0 | wässrige Lösung mit ca. 5% Keratin-Bindedomäne-Wirkstoff |
| E | q.s. | Sodium Hydroxide |

| WS 5%: | | |
|---|---|---|
| | % | Inhaltsstoff (INCl) |
| A | 1,7 | Ceteareth-6, Stearyl Alcohol |
| | 0,7 | Ceteareth-25 |
| | 2,0 | Diethylamino Hydroxybenzoyl Hexyl Benzoate |
| | 2,0 | PEG-14 Dimethicone |
| | 3,6 | Cetearyl Alcohol |
| | 6,0 | Ethylhexyl Methoxycinnamate |
| | 2,0 | Dibutyl Adipate |
| B | 5,0 | Glycerin |
| | 0,2 | Disodium EDTA |
| | 1,0 | Panthenol |
| | q.s. | Konservierungsmittel |
| | 64,6 | Aqua dem. |
| C | 4,0 | Caprylic/Capric Triglyceride, Sodium Acrylates Copolymer |
| D | 1,0 | Sodium Ascorbyl Phosphate |
| | 1,0 | Tocopheryl Acetate |
| | 0,2 | Bisabolol |
| | 5,0 | wässrige Lösung mit ca. 5% Keratin-Bindedomäne-Wirkstoff |
| E | q.s. | Sodium Hydroxide |

Herstellung: Die Phasen A und B getrennt voneinander auf ca. 80°C erwärmen. Phase B in Phase A einrühren und homogenisieren. Phase C in die kombinierten Phasen A und B einarbeiten und homogenisieren. Unter Rühren auf ca. 40°C abkühlen. Phase D hinzugeben, den pH-Wert mit Phase E auf ca. 6.5 einstellen und homogenisieren. Unter Rühren auf Raumtemperatur abkühlen.

### Beispiel 13: Verwendung der KBD in einer Gesichtsreinigungslotion -Typ O/W

| WS 1%: | | |
|---|---|---|
| | % | Inhaltsstoff (INCl) |
| A | 10,0 | Cetearyl Ethylhexanoate |
| | 10,0 | Caprylic/Capric Triglyceride |
| | 1,5 | Cyclopentasiloxane, Cyclohexasilosane |
| | 2,0 | PEG-40 Hydrogenated Castor Oil |
| B | 3,5 | Caprylic/Capric Triglyceride, Sodium Acrylates Copolymer |
| C | 1,0 | Tocopheryl Acetate |
| | 0,2 | Bisabolol |
| | q.s. | Konservierungsmittel |
| | q.s. | Parfümöl |
| D | 3,0 | Polyquaternium-44 |
| | 0,5 | Cocotrimonium Methosulfate |
| | 0,5 | Ceteareth-25 |
| | 2,0 | Panthenol, Propylene Glycol |
| | 4,0 | Propylene Glycol |
| | 0,1 | Disodium EDTA |
| | 1,0 | wässrige Lösung mit ca. 5% Keratin-Bindedomäne-Wirkstoff |
| | 60,7 | Aqua dem. |

| WS 5%: | | |
|---|---|---|
| | % | Inhaltsstoff (INCl) |
| A | 10,0 | Cetearyl Ethylhexanoate |
| | 10,0 | Caprylic/Capric Triglyceride |
| | 1,5 | Cyclopentasiloxane, Cyclohexasilosane |
| | 2,0 | PEG-40 Hydrogenated Castor Oil |
| B | 3,5 | Caprylic/Capric Triglyceride, Sodium Acrylates Copolymer |
| C | 1,0 | Tocopheryl Acetate |
| | 0,2 | Bisabolol |
| | q.s. | Konservierungsmittel |
| | q.s. | Parfümöl |
| D | 3,0 | Polyquaternium-44 |
| | 0,5 | Cocotrimonium Methosulfate |
| | 0,5 | Ceteareth-25 |
| | 2,0 | Panthenol, Propylene Glycol |
| | 4,0 | Propylene Glycol |
| | 0,1 | Disodium EDTA |
| | 5,0 | wässrige Lösung mit ca. 5 % Keratin-Bindedomäne-Wirkstoff |
| | 56,7 | Aqua dem. |

Herstellung: Phase A lösen. Phase B in Phase A einrühren, Phase C in die kombinierten Phasen A und B einarbeiten. Phase D lösen, in die kombinierten Phasen A, B und C einrühren und homogenisieren. 15min nachrühren.

### Beispiel 14: Verwendung der KBD in einem Daily Care Body Spray

| WS 1 %: | | |
|---|---|---|
| | % | Inhaltsstoff (INCl) |
| A | 3,0 | Ethylhexyl Methoxycinnamate |
| | 2,0 | Diethylamino Hydroxybenzoyl Hexyl Benzoate |
| | 1,0 | Polyquaternium-44 |
| | 3,0 | Propylene Glycol |
| | 2,0 | Panthenol, Propylene Glycol |
| | 1,0 | Cyclopentasiloxane, Cyclohexasiloxane |
| | 10,0 | Octyldodecanol |
| | 0,5 | PVP |
| | 10,0 | Caprylic/Capric Triglyceride |
| | 3,0 | C12-15 Alkyl Benzoate |
| | 3,0 | Glycerin |
| | 1,0 | Tocopheryl Acetate |
| | 0,3 | Bisabolol |
| | 1,0 | wässrige Lösung mit ca. 5% Keratin-Bindedomäne-Wirkstoff |
| | 59,2 | Alcohol |

| WS 5%: | | |
|---|---|---|
| | % | Inhaltsstoff (INCl) |
| A | 3,0 | Ethylhexyl Methoxycinnamate |
| | 2,0 | Diethylamino Hydroxybenzoyl Hexyl Benzoate |
| | 1,0 | Polyquaternium-44 |
| | 3,0 | Propylene Glycol |
| | 2,0 | Panthenol, Propylene Glycol |
| | 1,0 | Cyclopentasiloxane, Cyclohexasiloxane |
| | 10,0 | Octyldodecanol |
| | 0,5 | PVP |
| | 10,0 | Caprylic/Capric Triglyceride |
| | 3,0 | C12-15 Alkyl Benzoate |
| | 3,0 | Glycerin |
| | 1,0 | Tocopheryl Acetate |
| | 0,3 | Bisabolol |
| | 5,0 | wässrige Lösung mit ca. 5% Keratin-Bindedomäne-Wirkstoff |
| | 55,2 | Alcohol |

Herstellung: Die Komponenten der Phase A einwiegen und klar lösen.

### Beispiel 15: Verwendung der KBD in einem Hautpflegegel

| WS 1%: | | |
|---|---|---|
| | % | Inhaltsstoff (INCl) |
| A | 3,6 | PEG-40 Hydrogenated Castor Oil |
| | 15,0 | Alcohol |
| | 0,1 | Bisabolol |
| | 0,5 | Tocopheryl Acetate |
| | q.s. | Parfümöl |
| B | 3,0 | Panthenol |
| | 0,6 | Carbomer |
| | 1,0 | wässrige Lösung mit ca. 5% Keratin-Bindedomäne-Wirkstoff |
| | 75,4 | Aqua dem, |
| C | 0,8 | Triethanolamine |

| WS 5%: | | |
|---|---|---|
| | % | Inhaltsstoff (INCI) |
| A | 3,6 | PEG-40 Hydrogenated Castor Oil |
| | 15,0 | Alcohol |
| | 0,1 | Bisabolol |
| | 0,5 | Tocopheryl Acetate |
| | q.s. | Parfümöl |
| B | 3,0 | Panthenol |
| | 0,6 | Carbomer |
| | 5,0 | wässrige Lösung mit ca. 5% Keratin-Bindedomäne-Wirkstoff |
| | 71,4 | Aqua dem, |
| C | 0,8 | Triethanolamine |

Herstellung: Die Phase A klar lösen. Phase B quellen lassen und mit Phase C neutralisieren. Phase A in die homogenisierte Phase B einrühren und homogenisieren.

### Beispiel 16: Verwendung der KBD in einer After Shave Lotion

| WS 1%: | | |
|---|---|---|
| | % | Inhaltsstoff (INCl) |
| A | 10,0 | Cetearyl Ethylhexanoate |
| | 5,0 | Tocopheryl Acetate |
| | 1,0 | Bisabolol |
| | 0,1 | Parfümöl |
| | 0,3 | Acrylates/C10-30 Alkyl Acrylate Crosspolymer |
| B | 15,0 | Alcohol |
| | 1,0 | Panthenol |
| | 3,0 | Glycerin |
| | 1,0 | wässrige Lösung mit ca. 5% Keratin-Bindedomäne-Wirkstoff |
| | 0,1 | Triethanolamine |
| | 63,5 | Aqua dem. |

| WS 5%: | | |
|---|---|---|
| | % | Inhaltsstoff (INCI) |
| A | 10,0 | Cetearyl Ethylhexanoate |
| | 5,0 | Tocopheryl Acetate |
| | 1,0 | Bisabolol |
| | 0,1 | Parfümöl |
| | 0,3 | Acrylates/C10-30 Alkyl Acrylate Crosspolymer |
| B | 15,0 | Alcohol |
| | 1,0 | Panthenol |
| | 3,0 | Glycerin |
| | 5,0 | wässrige Lösung mit ca. 5% Keratin-Bindedomäne-Wirkstoff |
| | 0,1 | Triethanolamine |
| | 59,5 | Aqua dem. |

Herstellung: Die Komponenten der Phase A mischen. Phase B lösen, in Phase A einarbeiten und homogenisieren.

### Beispiel 17: Verwendung der KBD in einer After Sun Lotion

| WS 1%: | | |
|---|---|---|
| | % | Inhaltsstoff (INCl) |
| A | 0,4 | Acrylates/C10-30 Alkyl Acrylate Crosspolymer |
| | 15,0 | Cetearyl Ethylhexanoate |
| | 0,2 | Bisabolol |
| | 1,0 | Tocopheryl Acetate |
| | q.s. | Parfümöl |
| B | 1,0 | Panthenol |
| | 15,0 | Alcohol |
| | 3,0 | Glycerin |
| | 1,0 | wässrige Lösung mit ca. 5% Keratin-Bindedomäne-Wirkstoff |
| | 63,2 | Aqua dem, |
| C | 0,2 | Triethanolamine |

| WS 5%: | | |
|---|---|---|
| | % | Inhaltsstoff (INCI) |
| A | 0,4 | Acrylates/C10-30 Alkyl Acrylate Crosspolymer |
| | 15,0 | Cetearyl Ethylhexanoate |
| | 0,2 | Bisabolol |
| | 1,0 | Tocopheryl Acetate |
| | q.s. | Parfümöl |
| B | 1,0 | Panthenol |
| | 15,0 | Alcohol |
| | 3,0 | Glycerin |
| | 5,0 | wässrige Lösung mit ca. 5% Keratin-Bindedomäne-Wirkstoff |
| | 59,2 | Aqua dem, |
| C | 0,2 | Triethanolamine |

Herstellung: Die Komponenten der Phase A mischen. Phase B unter Homogenisieren in Phase A einrühren. Mit Phase C neutralisieren und erneut homogenisieren.

### Beispiel 18: Verwendung der KBD in einer Sonnenschutzlotion

| WS 1%: | | |
|---|---|---|
| | % | Inhaltsstoff (INCI) |
| A | 4,5 | Ethylhexyl Methoxycinnamate |
| | 2,0 | Diethylamino Hydroxybenzoyl Hexyl Benzoate |
| | 3,0 | Octocrylene |
| | 2,5 | Di-C12-13 Alkyl Malate |
| | 0,5 | Tocopheryl Acetate |
| | 4,0 | Polyglyceryl-3 Methyl Glucose Distearate |
| B | 3,5 | Cetearyl Isononanoate |
| | 1,0 | VP/Eicosene Copolymer |
| | 5,0 | Isohexadecane |
| | 2,5 | Di-C12-13 Alkyl Malate |
| | 3,0 | Titanium Dioxide, Trimethoxycaprylylsilane |
| C | 5,0 | Glycerin |
| | 1,0 | Sodium Cetearyl Sulfate |
| | 0,5 | Xanthan Gum |
| | 59,7 | Aqua dem. |
| D | 1,0 | wässrige Lösung mit ca. 5% Keratin-Bindedomäne-Wirkstoff |
| | 1,0 | Phenoxyethanol, Methylparaben, Ethylparaben, Butylparaben, Propyl- |
| | | paraben, Isobutylparaben |
| | 0,3 | Bisabolol |

| WS 5%: | | |
|---|---|---|
| | % | Inhaltsstoff (INCl) |
| A | 4,5 | Ethylhexyl Methoxycinnamate |
| | 2,0 | Diethylamino Hydroxybenzoyl Hexyl Benzoate |
| | 3,0 | Octocrylene |
| | 2,5 | Di-C12-13 Alkyl Malate |
| | 0,5 | Tocopheryl Acetate |
| | 4,0 | Polyglyceryl-3 Methyl Glucose Distearate |
| B | 3,5 | Cetearyl Isononanoate |
| | 1,0 | VP/Eicosene Copolymer |
| | 5,0 | Isohexadecane |
| | 2,5 | Di-C12-13 Alkyl Malate |
| | 3,0 | Titanium Dioxide, Trimethoxycaprylylsilane |
| C | 5,0 | Glycerin |
| | 1,0 | Sodium Cetearyl Sulfate |
| | 0,5 | Xanthan Gum |
| | 55,7 | Aqua dem. |
| D | 5,0 | wässrige Lösung mit ca. 5% Keratin-Bindedomäne-Wirkstoff |
| | 1,0 | Phenoxyethanol, Methylparaben, Ethylparaben, Butylparaben, Propylparaben, Isobutylparaben |
| | 0,3 | Bisabolol |

Herstellung: Die Komponenten der Phasen A und B getrennt voneinander auf ca. 80°C erwärmen. Phase B in Phase A einrühren und homogenisieren. Phase C auf ca. 80°C erwärmen und unter Homogenisieren in die kombinierten Phasen A und B einrühren. Unter Rühren auf ca. 40°C abkühlen, Phase D zugeben und nochmals homogenisieren.

### Beispiel 19: Verwendung der KBD in einer Sonnenschutzlotion - Typ O/W

| WS 1%: | | |
|---|---|---|
| | % | Inhaltsstoff (INCl) |
| A | 2,0 | Ceteareth-6, Stearyl Alcohol |
| | 2,0 | Ceteareth-25 |
| | 3,0 | Tribehenin |
| | 2,0 | Cetearyl Alcohol |
| | 2,0 | Cetearyl Ethylhexanoate |
| | 5,0 | Ethylhexyl Methoxycinnamate |
| | 1,0 | Ethylhexyl Triazone |
| | 1,0 | VP/Eicosene Copolymer |
| | 7,0 | Isopropyl Myristate |
| B | 5,0 | Zinc Oxide, Triethoxycaprylylsilane |
| C | 0,2 | Xanthan Gum |
| | 0,5 | Hydroxyethyl Acrylate/Sodium Acryloyldimethyl Taurate Copolymer, Squalane, Polysorbate 60 |
| | 0,2 | Disodium EDTA |
| | 5,0 | Propylene Glycol |
| | 0,5 | Panthenol |
| | 60,9 | Aqua dem. |
| D | 1,0 | wässrige Lösung mit ca. 5% Keratin-Bindedomäne-Wirkstoff |
| | 0,5 | Phenoxyethanol, Methylparaben, Butylparaben, Ethylparaben, Propylparaben, Isopropylparaben |
| | 1,0 | Tocopheryl Acetate |
| | 0,2 | Bisabolol |

| WS 5%: | | |
|---|---|---|
| | % | Inhaltsstoff (INCl) |
| A | 2,0 | Ceteareth-6, Stearyl Alcohol |
| | 2,0 | Ceteareth-25 |
| | 3,0 | Tribehenin |
| | 2,0 | Cetearyl Alcohol |
| | 2,0 | Cetearyl Ethylhexanoate |
| | 5,0 | Ethylhexyl Methoxycinnamate |
| | 1,0 | Ethylhexyl Triazone |
| | 1,0 | VP/Eicosene Copolymer |
| | 7,0 | Isopropyl Myristate |
| B | 5,0 | Zinc Oxide, Triethoxycaprylylsilane |
| C | 0,2 | Xanthan Gum |
| | 0,5 | Hydroxyethyl Acrylate/Sodium Acryloyldimethyl Taurate Copolymer, |
| | | Squalane, Polysorbate 60 |
| | 0,2 | Disodium EDTA |
| | 5,0 | Propylene Glycol |
| | 0,5 | Panthenol |
| | 56,9 | Aqua dem. |
| D | 5,0 | wässrige Lösung mit ca. 5% Keratin-Bindedomäne-Wirkstoff |
| | 0,5 | Phenoxyethanol, Methylparaben, Butylparaben, Ethylparaben, Propylpa- |
| | | raben, Isopropylparaben |
| | 1,0 | Tocopheryl Acetate |
| | 0,2 | Bisabolol |

Herstellung: Phase A auf ca. 80°C erwärmen, Phase B einrühren und 3min homogenisieren. Phase C ebenfalls auf 80°C erwärmen und unter Homogenisieren in die kombinierten Phasen A und B einrühren. Abkühlen auf ca. 40°C, Phase D einrühren und nochmals homogenisieren.

### Beispiel 20: Verwendung der KBD in einer Sonnenschutlotion - Typ O/W

| WS 1 %: | | |
|---|---|---|
| | % | Inhaltsstoff (INCl) |
| A | 3,5 | Ceteareth-6, Stearyl Alcohol |
| | 1,5 | Ceteareth-25 |
| | 7,5 | Ethylhexyl Methoxycinnamate |
| | 2,0 | Diethylamino Hydroxybenzoyl Hexyl Benzoate |
| | 2,0 | Cyclopentasiloxane, Cyclohexasiloxane |
| | 0,5 | Bees Wax |
| | 3,0 | Cetearyl Alcohol |
| | 10,0 | Caprylic/Capric Triglyceride |
| B | 5,0 | Titanium Dioxide, Silica, Methicone, Alumina |
| C | 3,0 | Glycerin |
| | 0,2 | Disodium EDTA |
| | 0,3 | Xanthan Gum |
| | 1,0 | Decyl Glucoside |
| | 2,0 | Panthenol, Propylene Glycol |
| | 56,3 | Aqua dem. |
| D | 1,0 | wässrige Lösung mit ca. 5% Keratin-Bindedomäne-Wirkstoff |
| | 1,0 | Tocopheryl Acetate |
| | 0,2 | Bisabolol |
| | q.s. | Parfümöl |
| | q.s. | Konservierungsmittel |

| WS 5%: | | |
|---|---|---|
| | % | Inhaltsstoff (INCl) |
| A | 3,5 | Ceteareth-6, Stearyl Alcohol |
| | 1,5 | Ceteareth-25 |
| | 7,5 | Ethylhexyl Methoxycinnamate |
| | 2,0 | Diethylamino Hydroxybenzoyl Hexyl Benzoate |
| | 2,0 | Cyclopentasiloxane, Cyclohexasiloxane |
| | 0,5 | Bees Wax |
| | 3,0 | Cetearyl Alcohol |
| | 10,0 | Caprylic/Capric Triglyceride |
| B | 5,0 | Titanium Dioxide, Silica, Methicone, Alumina |
| C | 3,0 | Glycerin |
| | 0,2 | Disodium EDTA |
| | 0,3 | Xanthan Gum |
| | 1,0 | Decyl Glucoside |
| | 2,0 | Panthenol, Propylene Glycol |
| | 52,3 | Aqua dem. |
| D | 5,0 | wässrige Lösung mit ca. 5% Keratin-Bindedomäne-Wirkstoff |
| | 1,0 | Tocopheryl Acetate |
| | 0,2 | Bisabolol |
| | q.s. | Parfümöl |
| | q.s. | Konservierungsmittel |

Herstellung: Phase A auf ca. 80°C erwärmen, Phase B einrühren und 3min homogenisieren. Phase C ebenfalls auf 80°C erwärmen und unter Homogenisieren in die kombinierten Phasen A und B einrühren. Abkühlen auf ca. 40°C, Phase D einrühren und nochmals homogenisieren.

### Beispiel 21: Verwendung der KBD in einem Fußbalsam

| WS 1 %: | | |
|---|---|---|
| | % | Inhaltsstoff (INCl) |
| A | 2,0 | Ceteareth-6, Stearyl Alcohol |
| | 2,0 | Ceteareth-25 |
| | 5,0 | Cetearyl Ethylhexanoate |
| | 4,0 | Cetyl Alcohol |
| | 4,0 | Glyceryl Stearate |
| | 5,0 | Mineral Oil |
| | 0,2 | Menthol |
| | 0,5 | Camphor |
| B | 69,3 | Aqua dem. |
| | q.s. | Konservierungsmittel |
| C | 1,0 | Bisabolol |
| | 1,0 | Tocopheryl Acetate |
| D | 1,0 | wässrige Lösung mit ca. 5% Keratin-Bindedomäne-Wirkstoff |
| | 5,0 | Witch Hazel Extract |

| WS 5%: | | |
|---|---|---|
| | % | Inhaltsstoff (INCl) |
| A | 2,0 | Ceteareth-6, Stearyl Alcohol |
| | 2,0 | Ceteareth-25 |
| | 5,0 | Cetearyl Ethylhexanoate |
| | 4,0 | Cetyl Alcohol |
| | 4,0 | Glyceryl Stearate |
| | 5,0 | Mineral Oil |
| | 0,2 | Menthol |
| | 0,5 | Camphor |
| B | 65,3 | Aqua dem. |
| | q.s. | Konservierungsmittel |
| C | 1,0 | Bisabolol |
| | 1,0 | Tocopheryl Acetate |
| D | 5,0 | wässrige Lösung mit ca. 5% Keratin-Bindedomäne-Wirkstoff |
| | 5,0 | Witch Hazel Extract |

Herstellung: Die Komponenten der Phasen A und B getrennt voneinander auf ca. 80°C erwärmen. Phase B in Phase A unter Homogenisieren einrühren. Unter Rühren abkühlen auf ca. 40°C, die Phasen C und D hinzugeben und kurz nachhomogenisieren. Unter Rühren auf Raumtemperatur abkühlen.

### Beispiel 22: Verwendung der KBD in einer W/O Emulsion mit Bisabolol

| WS 1 %: | | |
|---|---|---|
| | % | Inhaltsstoff (INCI) |
| A | 6,0 | PEG-7 Hydrogenated Castor Oil |
| | 8,0 | Cetearyl Ethylhexanoate |
| | 5,0 | Isopropyl Myristate |
| | 15,0 | Mineral Oil |
| | 0,3 | Magnesium Stearate |
| | 0,3 | Aluminum Stearate |
| | 2,0 | PEG-45/Dodecyl Glycol Copolymer |
| B | 5,0 | Glycerin |
| | 0,7 | Magnesium Sulfate |
| | 55,6 | Aqua dem. |
| C | 1,0 | wässrige Lösung mit ca. 5% Keratin-Bindedomäne-Wirkstoff |
| | 0,5 | Tocopheryl Acetate |
| | 0,6 | Bisabolol |

| WS 5%: | | |
|---|---|---|
| | % | Inhaltsstoff (INCI) |
| A | 6,0 | PEG-7 Hydrogenated Castor Oil |
| | 8,0 | Cetearyl Ethylhexanoate |
| | 5,0 | Isopropyl Myristate |
| | 15,0 | Mineral Oil |
| | 0,3 | Magnesium Stearate |
| | 0,3 | Aluminum Stearate |
| | 2,0 | PEG-45/Dodecyl Glycol Copolymer |
| B | 5,0 | Glycerin |
| | 0,7 | Magnesium Sulfate |
| | 51,6 | Aqua dem. |
| C | 5,0 | wässrige Lösung mit ca. 5% Keratin-Bindedomäne-Wirkstoff |
| | 0,5 | Tocopheryl Acetate |

Herstellung: Die Phasen A und B getrennt voneinander auf ca. 85°C erwärmen. Phase B in Phase A einrühren und homogenisieren. Unter Rühren auf ca. 40°C abkühlen, Phase C hinzugeben und nochmals kurz homogenisieren. Unter Rühren auf Raumtemperatur abkühlen.
Zusammenstellung Rezepturen für Patent Keratin-Bindedomäne - Haircare

### Beispiel 23: Schaumconditioner mit Festiger

| WS 1% | | |
|---|---|---|
| | % | Inhaltsstoff (INCl) |
| A | 10,0 | PVP/VA Copolymer |
| | 0,2 | Hydroxyethyl Cetyldimonium Phosphate |
| | 0,2 | Ceteareth-25 |
| | 0,5 | Dimethicone Copolyol |
| | q.s. | Parfümöl |
| | 10,0 | Alcohol |
| | 1,0 | wässrige Lösung mit ca. 5% Keratin-Bindedomäne-Wirkstoff |
| | 68,1 | Aqua dem. |
| | 10,0 | Propane/Butane |

| WS 5% | | |
|---|---|---|
| | % | Inhaltsstoff (INCl) |
| A | 10,0 | PVP/VA Copolymer |
| | 0,2 | Hydroxyethyl Cetyldimonium Phosphate |
| | 0,2 | Ceteareth-25 |
| | 0,5 | Dimethicone Copolyol |
| | q.s. | Parfümöl |
| | 10,0 | Alcohol |
| | 5,0 | wässrige Lösung mit ca. 5% Keratin-Bindedomäne-Wirkstoff |
| | 64,1 | Aqua dem. |
| | 10,0 | Propane/Butane |

Herstellung: Die Komponenten der Phase A zusammenwiegen, rühren bis alles gelöst ist und abfüllen.

### Beispiel 24: Schaumconditioner

| WS1% | | |
|---|---|---|
| | % | Inhaltsstoff (INCl) |
| A | 1,0 | Polyquaternium-4 |
| | 0,5 | Hydroxyethyl Cetyldimonium Phosphate |
| | 1,0 | wässrige Lösung mit ca. 5% Keratin-Bindedomäne-Wirkstoff |
| | q.s. | Parfümöl |
| | q.s. | Konservierungsmittel |
| | 91,5 | Aqua dem. |
| | 6,0 | Propane/Butane |

| WS 5% | | |
|---|---|---|
| | % | Inhaltsstoff (INCl) |
| A | 1,0 | Polyquatemium-4 |
| | 0,5 | Hydroxyethyl Cetyldimonium Phosphate |
| | 5,0 | wässrige Lösung mit ca. 5% Keratin-Bindedomäne-Wirkstoff |
| | q.s. | Parfümöl |
| | q.s. | Konservierungsmittel |
| | 87,5 | Aqua dem. |
| | 6,0 | Propane/Butane |

Herstellung: Die Komponenten der Phase A zusammenwiegen, rühren bis alles klar gelöst ist und abfüllen.

### Beispiel 25: Schaumconditioner

| WS1% | | |
|---|---|---|
| | % | Inhaltsstoff (INCl) |
| A | 1,0 | Polyquaternium-11 |
| | 0,5 | Hydroxyethyl Cetyldimonium Phosphate |
| | 1,0 | wässrige Lösung mit ca. 5% Keratin-Bindedomäne-Wirkstoff |
| | q.s. | Parfümöl |
| | q.s. | Konservierungsmittel |
| | 91,5 | Aqua dem. |
| | 6,0 | Propane/Butane |

| WS 5% | | |
|---|---|---|
| | % | Inhaltsstoff (INCl) |
| A | 1,0 | Polyquaternium-11 |
| | 0,5 | Hydroxyethyl Cetyldimonium Phosphate |
| | 5,0 | wässrige Lösung mit ca. 5% Keratin-Bindedomäne-Wirkstoff |
| | q.s. | Parfümöl |
| | q.s. | Konservierungsmittel |
| | 87,5 | Aqua dem. |
| | 6,0 | Propane/Butane |

Herstellung: Die Komponenten der Phase A zusammenwiegen, rühren bis alles klar gelöst ist und abfüllen.

### Beispiel 26: Styling Schaum

| WS 1 % | | |
|---|---|---|
| | % | Inhaltsstoff (INCl) |
| A | 0,5 | Laureth-4 |
| | q.s. | Parfümöl |
| | | |
| B | 77,3 | Aqua dem. |
| | 10,0 | Polyquatemium-28 |
| | | |
| | 1,0 | wässrige Lösung mit ca. 5% Keratin-Bindedomäne-Wirkstoff |
| | 0,5 | Dimethicone Copolyol |
| | 0,2 | Ceteareth-25 |
| | 0,2 | Panthenol |
| | 0,1 | PEG-25 PABA |
| | 0,2 | Hydroxyethylcellulose |
| | | |
| C | 10,0 | HFC 152 A |

| WS 5% | | |
|---|---|---|
| | % | Inhaltsstoff (INCl) |
| A | 0,5 | Laureth-4 |
| | q.s. | Parfümöl |
| | | |
| B | 73,3 | Aqua dem. |
| | 10,0 | Polyquatemium-28 |
| | 5,0 | wässrige Lösung mit ca. 5% Keratin-Bindedomäne-Wirkstoff |
| | 0,5 | Dimethicone Copolyol |
| | 0,2 | Ceteareth-25 |
| | 0,2 | Panthenol |
| | 0,1 | PEG-25 PABA |
| | 0,2 | Hydroxyethylcellulose |
| | | |
| C | 10,0 | HFC 152 A |

Herstellung: Die Komponenten der Phase A mischen. Die Komponenten der Phase B eine nach der anderen zugeben und lösen. Mit Phase C abfüllen.

### Beispiel 27: Styling Schaum

| WS 1 % | | |
|---|---|---|
| | % | Inhaltsstoff (INCl) |
| A | 2,0 | Cocotrimonium Methosulfate |
| | q.s. | Parfümöl |
| | | |
| B | 78,5 | Aqua dem. |
| | 6,7 | Acrylates Copolymer |
| | | |
| | 0,6 | AMP |
| | 1,0 | wässrige Lösung mit ca. 5% Keratin-Bindedomäne-Wirkstoff |
| | 0,5 | Dimethicone Copolyol |
| | 0,2 | Ceteareth-25 |
| | 0,2 | Panthenol |
| | 0,1 | PEG-25 PABA |
| | 0,2 | Hydroxyethylcellulose |
| | | |
| C | 10,0 | HFC 152 A |

| WS 5% | | |
|---|---|---|
| | % | Inhaltsstoff (INCl) |
| A | 2,0 | Cocotrimonium Methosulfate |
| | q.s. | Parfümöl |
| | | |
| B | 74,5 | Aqua dem. |
| | 6,7 | Acrylates Copolymer |
| | | |
| | 0,6 | AMP |
| | 5,0 | wässrige Lösung mit ca. 5% Keratin-Bindedomäne-Wirkstoff |
| | 0,5 | Dimethicone Copolyol |
| | 0,2 | Ceteareth-25 |
| | 0,2 | Panthenol |
| | 0,1 | PEG-25 PABA |
| | 0,2 | Hydroxyethylcellulose |
| | | |
| C | 10,0 | HFC 152 A |

Herstellung: Die Komponenten der Phase A mischen. Die Komponenten der Phase B eine nach der anderen zugeben und lösen. Mit Phase C abfüllen.

### Beispiel 28: Styling Schaum

| WS 1 % | | |
|---|---|---|
| | % | Inhaltsstoff (INCl) |
| A | 2,0 | Cocotrimonium Methosulfate |
| | q.s. | Parfümöl |
| | | |
| B | 7,70 | Polyquaternium-44 |
| | 1,0 | wässrige Lösung mit ca. 5% Keratin-Bindedomäne-Wirkstoff |
| | | |
| | q.s. | Konservierungsmittel |
| | 79,3 | Aqua dem. |
| | | |
| C | 10,0 | Propane/Butane |

| WS 5% | | |
|---|---|---|
| | % | Inhaltsstoff (INCl) |
| A | 2,0 | Cocotrimonium Methosulfate |
| | q.s. | Parfümöl |
| | | |
| B | 7,70 | Polyquaternium-44 |
| | 5,0 | wässrige Lösung mit ca. 5% Keratin-Bindedomäne-Wirkstoff |
| | q.s. | Konservierungsmittel |
| | | |
| | 75,3 | Aqua dem. |
| | | |
| C | 10,0 | Propane/Butane |

Herstellung: Die Komponenten der Phase A mischen. Die Komponenten der Phase B klar lösen, dann Phase B in Phase A einrühren. Den pH-Wert auf 6-7 einstellen, mit Phase C abfüllen.

### Beispiel 29: Styling Schaum

| WS 1 % | | |
|---|---|---|
| | % | Inhaltsstoff (INCl) |
| A | 2,00 | Cocotrimonium Methosulfate |
| | q.s. | Parfümöl |
| | | |
| B | 72,32 | Aqua dem. |
| | 2,00 | VP/Acrylates/Lauryl Methacrylate Copolymer |
| | 0,53 | AMP |
| | 1,00 | wässrige Lösung mit ca. 5% Keratin-Bindedomäne-Wirkstoff |
| | 0,20 | Ceteareth-25 |
| | 0,50 | Panthenol |
| | 0,05 | Benzophenone-4 |
| | 0,20 | Amodimethicone, Cetrimonium Chloride, Trideceth-12 |
| | 15,00 | Alcohol |
| | | |
| C | 0,20 | Hydroxyethylcellulose |
| | | |
| D | 6,00 | Propane/Butane |

| WS 5% | | |
|---|---|---|
| | % | Inhaltsstoff (INCl) |
| A | 2,00 | Cocotrimonium Methosulfate |
| | q.s. | Parfümöl |
| | | |
| B | 68,32 | Aqua dem. |
| | 2,00 | VP/Acrylates/Lauryl Methacrylate Copolymer |
| | 0,53 | AMP |
| | 5,00 | wässrige Lösung mit ca. 5% Keratin-Bindedomäne-Wirkstoff |
| | 0,20 | Ceteareth-25 |
| | 0,50 | Panthenol |
| | 0,05 | Benzophenone-4 |
| | 0,20 | Amodimethicone, Cetrimonium Chloride, Trideceth-12 |
| | | |
| | 15,00 | Alcohol |
| C | 0,20 | Hydroxyethylcellulose |
| | | |
| D | 6,00 | Propane/Butane |

Herstellung: Die Komponenten der Phase A mischen. Die Komponenten der Phase B eine nach der anderen zugeben und lösen. Phase C in der Mischung aus A und B lösen, dann den pH-Wert auf 6-7 einstellen. Mit Phase D abfüllen

### Beispiel 30: Styling Schaum

| WS 1 % | | |
|---|---|---|
| | % | Inhaltsstoff (INCI) |
| A | 2,00 | Cetrimonium Chloride |
| | q.s. | Parfümöl |
| | | |
| B | 67,85 | Aqua dem. |
| | 7,00 | Polyquatemium-46 |
| | 1,00 | wässrige Lösung mit ca. 5% Keratin-Bindedomäne-Wirkstoff |

| | | |
|---|---|---|
| | 0,20 | Ceteareth-25 |
| | 0,50 | Panthenol |
| | 0,05 | Benzophenone-4 |
| | 0,20 | Amodimethicone, Cetrimonium Chloride, Trideceth-12 |
| | 15,00 | Alcohol |
| | | |
| C | 0,20 | Hydroxyethylcellulose |
| | | |
| D | 6,00 | Propane/Butane |

| WS 5% | | |
|---|---|---|
| | % | Inhaltsstoff (INCl) |
| A | 2,00 | Cetrimonium Chloride |
| | q.s. | Parfümöl |
| | | |
| B | 63,85 | Aqua dem. |
| | 7,00 | Polyquatemium-46 |
| | 5,00 | wässrige Lösung mit ca. 5% Keratin-Bindedomäne-Wirkstoff |
| | 0,20 | Ceteareth-25 |
| | 0,50 | Panthenol |
| | 0,05 | Benzophenone-4 |
| | 0,20 | Amodimethicone, Cetrimonium Chloride, Trideceth-12 |
| | 15,00 | Alcohol |
| | | |
| C | 0,20 | Hydroxyethylcellulose |
| | | |
| D | 6,00 | Propane/Butane |

Herstellung: Die Komponenten der Phase A mischen. Die Komponenten der Phase B eine nach der anderen zugeben und lösen. Phase C in der Mischung aus A und B lösen, dann den pH-Wert auf 6-7 einstellen. Mit Phase D abfüllen.

### Beispiel 31: Styling Schaum

| WS 1 % | | |
|---|---|---|
| | % | Inhaltsstoff (INCl) |
| A | q.s. | PEG-40 Hydrogenated Castor Oil |
| | q.s. | Parfümöl |
| | | |
| | 85,5 | Aqua dem. |
| B | 7,0 | Sodium Polystyrene Sulfonate |
| | 1,0 | wässrige Lösung mit ca. 5% Keratin-Bindedomäne-Wirkstoff |
| | 0,5 | Cetrimonium Bromide |
| | q.s. | Konservierungsmittel |
| | | |
| C | 6,0 | Propane/Butane |

### Styling Schaum

| WS 5% | | |
|---|---|---|
| | % | Inhaltsstoff (INCl) |
| A | q.s. | PEG-40 Hydrogenated Castor Oil |
| | q.s. | Parfümöl |
| | | |
| | 81,5 | Aqua dem. |
| B | 7,0 | Sodium Polystyrene Sulfonate |
| | 5,0 | wässrige Lösung mit ca. 5% Keratin-Bindedomäne-Wirkstoff |
| | 0,5 | Cetrimonium Bromide |
| | q.s. | Konservierungsmittel |
| | | |
| C | 6,0 | Propane/Butane |

Herstellung: Phase A solubilisieren. Phase B in Phase A einwiegen und klar lösen. Den pH-Wert auf 6-7 einstellen, mit Phase C abfüllen.

### Beispiel 32: Styling Schaum

| WS 1% | | |
|---|---|---|
| | % | Inhaltsstoff (INCl) |
| A | q.s. | PEG-40 Hydrogenated Castor Oil |
| | | |
| | q.s. | Parfümöl |
| | 92,0 | Aqua dem. |
| B | 0,5 | Polyquaternium-10 |
| | 1,0 | wässrige Lösung mit ca. 5% Keratin-Bindedomäne-Wirkstoff |
| | 0,5 | Cetrimonium Bromide |
| | q.s. | Konservierungsmittel |
| | | |
| C | 6,0 | Propane/Butane |

| WS 5% | | |
|---|---|---|
| | % | Inhaltsstoff (INCl) |
| A | q.s. | PEG-40 Hydrogenated Castor Oil |
| | q.s. | Parfümöl |
| | | |
| | 88,0 | Aqua dem. |
| B | 0,5 | Polyquaternium-10 |
| | 5,0 | wässrige Lösung mit ca. 5% Keratin-Bindedomäne-Wirkstoff |
| | 0,5 | Cetrimonium Bromide |
| | q.s. | Konservierungsmittel |
| | | |
| C | 6,0 | Propane/Butane |

Herstellung: Phase A solubilisieren. Phase B in Phase A einwiegen und klar lösen. Den pH-Wert auf 6-7 einstellen, mit Phase C abfüllen.

### Beispiel 32: Styling Schaum

| WS 1 % | | |
|---|---|---|
| | % | Inhaltsstoff (INCl) |
| A | q.s. | PEG-40 Hydrogenated Castor Oil |
| | q.s. | Parfümöl |
| | | |
| | 82,5 | Aqua dem. |
| B | 10,0 | Polyquaternium-16 |
| | 1,0 | wässrige Lösung mit ca. 5% Keratin-Bindedomäne-Wirkstoff |
| | 0,5 | Hydroxyethyl Cetyldimonium Phosphate |
| | q.s. | Konservierungsmittel |
| | | |
| C | 6,0 | Propane/Butane |

| WS 5% | | |
|---|---|---|
| | % | Inhaltsstoff (INCI) |
| A | q.s. | PEG-40 Hydrogenated Castor Oil |
| | q.s. | Parfümöl |
| | | |
| | 78,5 | Aqua dem. |
| B | 10,0 | Polyquaternium-16 |
| | 5,0 | wässrige Lösung mit ca. 5% Keratin-Bindedomäne-Wirkstoff |
| | 0,5 | Hydroxyethyl Cetyldimonium Phosphate |
| | q.s. | Konservierungsmittel |
| | | |
| C | 6,0 | Propane/Butane |

Herstellung: Phase A solubilisieren. Phase B in Phase A einwiegen und klar lösen. Den pH-Wert auf 6-7 einstellen, mit Phase C abfüllen.

### Beispiel 33: Styling Schaum

| WS1% | | |
|---|---|---|
| | % | Inhaltsstoff (INCl) |
| A | 2,0 | Cocotrimonium Methosulfate |
| | q.s. | Parfümöl |
| | | |
| B | 84,0 | Aqua dem. |
| | 2,0 | Chitosan |
| | 1,0 | wässrige Lösung mit ca. 5% Keratin-Bindedomäne-Wirkstoff |
| | 0,5 | Dimethicone Copolyol |
| | 0,2 | Ceteareth-25 |
| | 0,2 | Panthenol |
| | 0,1 | PEG-25 PABA |
| | | |
| C | 10,0 | HFC 152 A |

| WS 5% | | |
|---|---|---|
| | % | Inhaltsstoff (INCl) |
| A | 2,0 | Cocotrimonium Methosulfate |
| | q.s. | Parfümöl |
| | | |
| B | 80,0 | Aqua dem. |
| | 2,0 | Chitosan |
| | 5,0 | wässrige Lösung mit ca. 5% Keratin-Bindedomäne-Wirkstoff |
| | 0,5 | Dimethicone Copolyol |
| | 0,2 | Ceteareth-25 |
| | 0,2 | Panthenol |
| | 0,1 | PEG-25 PABA |
| | | |
| C | 10,0 | HFC 152 A |

Herstellung: Die Komponenten der Phase A mischen. Die Komponenten der Phase B eine nach der anderen zugeben und lösen. Mit Phase C abfüllen.

### Beispiel 34: Pflegeshampoo

| WS 1 % | | |
|---|---|---|
| | % | Inhaltsstoff (INCl) |
| A | 30,0 | Sodium Laureth Sulfate |
| | 6,0 | Sodium Cocoamphoacetate |
| | 6,0 | Cocamidopropyl Betaine |
| | 3,0 | Sodium Laureth Sulfate, Glycol Distearate, Cocamide MEA, Laureth-10 |
| | 1,0 | wässrige Lösung mit ca. 5% Keratin-Bindedomäne-Wirkstoff |
| | 7,7 | Polyquaternium-44 |
| | 2,0 | Amodimethicone |
| | q.s. | Parfümöl |
| | q.s. | Konservierungsmittel |
| | 1,0 | Sodium Chloride |
| | 43,3 | Aqua dem. |
| B | q.s. | Citric Acid |

| WS 5% | | |
|---|---|---|
| | % | Inhaltsstoff (INCI) |
| A | 30,0 | Sodium Laureth Sulfate |
| | 6,0 | Sodium Cocoamphoacetate |
| | 6,0 | Cocamidopropyl Betaine |
| | 3,0 | Sodium Laureth Sulfate, Glycol Distearate, Cocamide MEA, Laureth-10 |
| | 5,0 | wässrige Lösung mit ca. 5% Keratin-Bindedomäne-Wirkstoff |
| | 7,7 | Polyquaternium-44 |
| | 2,0 | Amodimethicone |
| | q.s. | Parfümöl |
| | q.s. | Konservierungsmittel |
| | 1,0 | Sodium Chloride |
| | 39,3 | Aqua dem. |
| B | q.s. | Citric Acid |

Herstellung: Die Komponenten der Phase A mischen und lösen. Den pH-Wert mit Citronensäure auf 6-7 einstellen.

### Beispiel 35: Duschgel

| WS 1 % | | |
|---|---|---|
| | % | Inhaltsstoff (INCI) |
| A | 40,0 | Sodium Laureth Sulfate |
| | 5,0 | Decyl Glucoside |
| | 5,0 | Cocamidopropyl Betaine |
| | 1,0 | wässrige Lösung mit ca. 5% Keratin-Bindedomäne-Wirkstoff |
| | 1,0 | Panthenol |
| | q.s. | Parfümöl |
| | q.s. | Konservierungsmittel |
| | 2,0 | Sodium Chloride |
| | 46,0 | Aqua dem. |
| B | q.s. | Citric Acid |

| WS 5% | | |
|---|---|---|
| | % | Inhaltsstoff (INCI) |
| A | 40,0 | Sodium Laureth Sulfate |
| | 5,0 | Decyl Glucoside |
| | 5,0 | Cocamidopropyl Betaine |
| | 5,0 | wässrige Lösung mit ca. 5% Keratin-Bindedomäne-Wirkstoff |
| | 1,0 | Panthenol |
| | q.s. | Parfümöl |
| | q.s. | Konservierungsmittel |
| | 2,0 | Sodium Chloride |
| | 42,0 | Aqua dem. |
| B | q.s. | Citric Acid |

Herstellung: Die Komponenten der Phase A mischen und lösen. Den pH-Wert mit Citronensäure auf 6-7 einstellen.

### Beispiel 36: Shampoo

| WS1% | | |
|---|---|---|
| | % | Inhaltsstoff (INCl) |
| A | 40,0 | Sodium Laureth Sulfate |
| | 5,0 | Sodium C12-15 Pareth-15 Sulfonate |
| | 5,0 | Decyl Glucoside |
| | q.s. | Parfümöl |
| | 0,1 | Phytantriol |
| | 44,6 | Aqua dem. |
| | 1,0 | wässrige Lösung mit ca. 5% Keratin-Bindedomäne-Wirkstoff |
| | 0,3 | Polyquaternium-10 |
| | 1,0 | Panthenol |
| | q.s. | Konservierungsmittel |
| | 1,0 | Laureth-3 |
| | 2,0 | Sodium Chloride |

| WS 5% | | |
|---|---|---|
| | % | Inhaltsstoff (INCI) |
| A | 40,0 | Sodium Laureth Sulfate |
| | 5,0 | Sodium C12-15 Pareth-15 Sulfonate |
| | 5,0 | Decyl Glucoside |
| | q.s. | Parfümöl |
| | 0,1 | Phytantriol |
| | 40,6 | Aqua dem. |
| | 5,0 | wässrige Lösung mit ca. 5% Keratin-Bindedomäne-Wirkstoff |
| | 0,3 | Polyquaternium-10 |
| | 1,0 | Panthenol |
| | q.s. | Konservierungsmittel |
| | 1,0 | Laureth-3 |
| | 2,0 | Sodium Chloride |

Herstellung: Die Komponenten der Phase A mischen und lösen. Den pH-Wert mit Citronensäure auf 6-7 einstellen.

### Beispiel 37: Shampoo

| WS1% | | |
|---|---|---|
| | % | Inhaltsstoff (INCl) |
| A | 15,00 | Cocamidopropyl Betaine |
| | 10,00 | Disodium Cocoamphodiacetate |
| | 5,00 | Polysorbate 20 |
| | 5,00 | Decyl Glucoside |
| | q.s. | Parfümöl |
| | q.s. | Konservierungsmittel |
| | 1,00 | wässrige Lösung mit ca. 5% Keratin-Bindedomäne-Wirkstoff |
| | 0,15 | Guar Hydroxypropyltrimonium Chloride |
| | 2,00 | Laureth-3 |
| | 58,00 | Aqua dem. |
| | q.s. | Citric Acid |
| | | |
| B | 3,00 | PEG-150 Distearate |

| WS 5% | | |
|---|---|---|
| | % | Inhaltsstoff (INCl) |
| A | 15,00 | Cocamidopropyl Betaine |
| | 10,00 | Disodium Cocoamphodiacetate |
| | 5,00 | Polysorbate 20 |
| | 5,00 | Decyl Glucoside |
| | q.s. | Parfümöl |
| | q.s. | Konservierungsmittel |
| | 5,00 | wässrige Lösung mit ca. 5% Keratin-Bindedomäne-Wirkstoff |
| | 0,15 | Guar Hydroxypropyltrimonium Chloride |
| | 2,00 | Laureth-3 |
| | 54,00 | Aqua dem. |
| | q.s. | Citric Acid |
| | | |
| B | 3,00 | PEG-150 Distearate |

Herstellung: Die Komponenten der Phase A einwiegen und lösen. Den pH-Wert auf 6-7 einstellen. Phase B zugeben und auf ca. 50°C erwärmen. Unter Rühren auf Raumtemperatur abkühlen.

### Beispiel 38: Feuchtigkeitsspendende Körperpflegecreme

| WS1% | | |
|---|---|---|
| | % | Inhaltsstoff (INCl) |
| A | 2,0 | Ceteareth-25 |
| | 2,0 | Ceteareth-6, Stearyl Alcohol |
| | 3,0 | Cetearyl Ethylhexanoate |
| | 1,0 | Dimethicone |
| | 4,0 | Cetearyl Alcohol |
| | 3,0 | Glyceryl Stearate SE |
| | 5,0 | Mineral Oil |
| | 4,0 | Simmondsia Chinensis (Jojoba) Seed Oil |
| | 3,0 | Mineral Oil, Lanolin Alcohol |
| | | |
| B | 5,0 | Propylene Glycol |
| | 1,0 | wässrige Lösung mit ca. 5% Keratin-Bindedomäne-Wirkstoff |
| | 1,0 | Panthenol |
| | 0,5 | Magnesium Aluminum Silicate |
| | q.s | Konservierungsmittel |
| | 65,5 | Aqua dem. |
| | | |
| C | q.s. | Parfümöl |
| | | |
| D | q.s. | Citric Acid |

| WS 5% | | |
|---|---|---|
| | % | Inhaltsstoff (INCl) |
| A | 2,0 | Ceteareth-25 |
| | 2,0 | Ceteareth-6, Stearyl Alcohol |
| | 3,0 | Cetearyl Ethylhexanoate |
| | 1,0 | Dimethicone |
| | 4,0 | Cetearyl Alcohol |
| | 3,0 | Glyceryl Stearate SE |
| | 5,0 | Mineral Oil |
| | 4,0 | Simmondsia Chinensis (Jojoba) Seed Oil |
| | 3,0 | Mineral Oil, Lanolin Alcohol |
| | | |
| B | 5,0 | Propylene Glycol |
| | 5,0 | wässrige Lösung mit ca. 5% Keratin-Bindedomäne-Wirkstoff |
| | 1,0 | Panthenol |
| | 0,5 | Magnesium Aluminum Silicate |
| | q.s | Konservierungsmittel |
| | 61,5 | Aqua dem. |
| | | |
| C | q.s. | Parfümöl |
| | | |
| D | q.s. | Citric Acid |

Herstellung: Die Phasen A und B getrennt auf ca. 80°C erwärmen. Phase B kurz vorhomogenisieren, dann Phase B in Phase A einrühren und erneut homogenisieren.Abkühlen auf ca. 40°C, Phase C zugeben und nochmals gut homogenisieren. Den pH-Wert mit Citronensäure auf 6-7 einstellen.

### Beispiel 39: Feuchtigkeitsspendende Körperpflegecreme

| WS1% | | |
|---|---|---|
| | % | Inhaltsstoff (INCI) |
| A | 6,0 | PEG-7 Hydrogenated Castor Oil |
| | 10,0 | Cetearyl Ethylhexanoate |
| | 5,0 | Isopropyl Myristate |
| | 7,0 | Mineral Oil |
| | 0,5 | Shea Butter (Butyrospermum Parkii) |
| | 0,5 | Aluminum Stearate |
| | 0,5 | Magnesium Stearate |
| | 0,2 | Bisabolol |
| | 0,7 | Quaternium-18-Hectorite |
| | | |
| B | 5,0 | Dipropylene Glycol |
| | 0,7 | Magnesium Sulfate |
| | q.s. | Konservierungsmittel |
| | 62,9 | Aqua dem. |
| | | |
| C | q.s. | Parfümöl |
| | 1,0 | wässrige Lösung mit ca. 5% Keratin-Bindedomäne-Wirkstoff |

| WS 5% | | |
|---|---|---|
| | % | Inhaltsstoff (INCI) |
| A | 6,0 | PEG-7 Hydrogenated Castor Oil |
| | 10,0 | Cetearyl Ethylhexanoate |
| | 5,0 | Isopropyl Myristate |
| | 7,0 | Mineral Oil |
| | 0,5 | Shea Butter (Butyrospermum Parkii) |
| | 0,5 | Aluminum Stearate |
| | 0,5 | Magnesium Stearate |
| | 0,2 | Bisabolol |
| | 0,7 | Quaternium-18-Hectorite |
| | | |
| B | 5,0 | Dipropylene Glycol |
| | 0,7 | Magnesium Sulfate |
| | q.s. | Konservierungsmittel |
| | 58,9 | Aqua dem. |
| | | |
| C | q.s. | Parfümöl |
| | 5,0 | wässrige Lösung mit ca. 5% Keratin-Bindedomäne-Wirkstoff |

Herstellung: Die Phasen A und B getrennt auf ca. 80°C erwärmen. Phase B in Phase A einrühren und homogenisieren. Unter Rühren auf ca. 40°C abkühlen, Phase C zugeben und nochmals homogenisieren. Unter Rühren auf Raumtemperatur abkühlen lassen.

### Beispiel 40: Flüssiges Make-up - Typ O/W

| WS 1 % | | |
|---|---|---|
| | % | Inhaltsstoff (INCI) |
| A | 2,0 | Ceteareth-6, Stearyl Alcohol |
| | 2,0 | Ceteareth-25 |
| | 6,0 | Glyceryl Stearate |
| | 1,0 | Cetyl Alcohol |
| | 8,0 | Mineral Oil |
| | 7,0 | Cetearyl Ethylhexanoate |
| | 0,2 | Dimethicone |
| | | |
| B | 3,0 | Propylene Glycol |
| | 1,0 | Panthenol |
| | q.s. | Konservierungsmittel |
| | 61,9 | Aqua dem. |
| | | |
| C | 0,1 | Bisabolol |
| | 1,0 | wässrige Lösung mit ca. 5% Keratin-Bindedomäne-Wirkstoff |
| | q.s. | Parfümöl |
| | | |
| D | 5,7 | C. I. 77 891, Titanium Dioxide |
| | 1,1 | Iron Oxides |

| WS 5% | | |
|---|---|---|
| | % | Inhaltsstoff (INCI) |
| A | 2,0 | Ceteareth-6, Stearyl Alcohol |
| | 2,0 | Ceteareth-25 |
| | 6,0 | Glyceryl Stearate |
| | 1,0 | Cetyl Alcohol |
| | 8,0 | Mineral Oil |
| | 7,0 | Cetearyl Ethylhexanoate |
| | 0,2 | Dimethicone |
| | | |
| B | 3,0 | Propylene Glycol |
| | 1,0 | Panthenol |
| | q.s. | Konservierungsmittel |
| | 57,9 | Aqua dem. |
| | | |
| C | 0,1 | Bisabolol |
| | 5,0 | wässrige Lösung mit ca. 5% Keratin-Bindedomäne-Wirkstoff |
| | q.s. | Parfümöl |
| | | |
| D | 5,7 | C. I. 77 891, Titanium Dioxide |
| | 1,1 | Iron Oxides |

Herstellung: Die Phasen A und B getrennt auf ca. 80°C erwärmen. Phase B in Phase A einrühren und homogenisieren. Unter Rühren auf ca. 40°C abkühlen, Phasen C und D zugeben und nochmals gründlich homogenisieren. Unter Rühren auf Raumtemperatur abkühlen lassen.

### Beispiel 41

In den folgenden beispielhaften Rezepturen wurde als Wirkstoff eine ca. 5 Gew.-% ige wäßrige Lösung einer Keratin-Bindedomäne, bzw. eines Keratin-bindenden Effektormoleküls eingesetzt. Die folgenden Angaben sind Gewichtsteile.

| **Klares Shampoo** | | | | | |
|---|---|---|---|---|---|
| **Inhaltsstoffe (INCI)** | **1** | **2** | **3** | **4** | **5** |
| Texapon N 70 | 13,00 | 15,00 | 10,50 | 12,50 | 10,00 |
| Dehyaufn PK 45 | 7,50 | 7,00 | 5,00 | 5,50 | 10,00 |
| Cetiol HE | 2,00 | 2,50 | 3,50 | 5,00 | 2,30 |
| Fragrance | 0,10 | 0,10 | 0,10 | 0,10 | 0,10 |
| Wässrige Lösung mit Keratin Binde-Domäne-Wirkstoff | 1,0 | 5,0 | 0,1 | 0,5 | 10,0 |
| D-Panthenol USP | 1,00 | 1,50 | 1,80 | 1,70 | 1,40 |
| Konservierungsmittele | 0,10 | 0,10 | 0,10 | 0,10 | 0,10 |
| Citric Säure | 0,10 | 0,10 | 0,10 | 0,10 | 0,10 |
| Luviquat Ultra Care | 1,50 | 1,00 | 1,50 | 1,20 | 1,10 |
| Sodium Chloride | 1,50 | 1,40 | 1,40 | 1,30 | 1,50 |
| Aqua dem. | ad 100 | ad 100 | ad 100 | ad 100 | ad 100 |

| **Shampoo** | | | | | |
|---|---|---|---|---|---|
| **Inhaltsstoffe (INCI)** | **1** | **2** | **3** | **4** | **5** |
| Texapon NSO | 35,00 | 40,00 | 30,00 | 45,00 | 27,00 |
| Plantacare 2000 | 5,00 | 5,50 | 4,90 | 3,50 | 7,00 |
| Tego Betain L7 | 10,00 | 5,00 | 12,50 | 7,50 | 15,00 |
| Fragrance | 0,10 | 0,10 | 0,10 | 0,10 | 0,10 |
| Wässrige Lösung mit Keratin Binde-Domäne-Wirkstoff | 1,0 | 5,0 | 0,1 | 0,5 | 10,0 |
| D-Panthenol USP | 0,50 | 1,00 | 0,80 | 1,50 | 0,50 |
| Konservierungsmittele | 0,10 | 0,10 | 0,10 | 0,10 | 0,10 |
| Citric Säure | 0,10 | 0,10 | 0,10 | 0,10 | 0,10 |
| Rewopal LA 3 | 0,50 | 2,00 | 0,50 | 0,50 | 2,00 |
| Sodium Chloride | 1,50 | 1,50 | 1,50 | 1,50 | 1,50 |
| Aqua dem. | ad 100 | ad 100 | ad 100 | ad 100 | ad 100 |

| **Klares Conditioner Shampoo** | | | | | |
|---|---|---|---|---|---|
| **Inhaltsstoffe (INCI)** | **1** | **2** | **3** | **4** | **5** |
| Amphotensid GB 2009 | 10,00 | 15,00 | 20,00 | 12,00 | 17,00 |
| Plantacare 2000 | 5,00 | 6,00 | 7,00 | 8,00 | 4,00 |
| Tego Betain L7 | 15,00 | 12,00 | 10,00 | 18,00 | 20,00 |
| Luviquat FC 550 | 0,30 | 0,20 | 0,20 | 0,20 | 0,30 |
| Fragrance | 0,10 | 0,10 | 0,10 | 0,10 | 0,10 |
| Wässrige Lösung mit Keratin Binde-Domäne-Wirkstoff | 20,0 | 5,0 | 1,0 | 0,5 | 10,0 |
| Cremophor PS 20 | 5,00 | 1,00 | 1,00 | 7,00 | 5,00 |
| Konservierungsmittele | 0,10 | 0,10 | 0,10 | 0,10 | 0,10 |
| Rewopal LA 3 | 2,00 | 1,00 | 0,50 | 2,00 | 2,00 |
| Citric Säure | 0,20 | 0,20 | 0,20 | 0,20 | 0,20 |
| Stepan PEG 600 DS | 3,00 | 2,00 | 2,00 | 3,00 | 2,50 |
| Aqua dem. | ad 100 | ad 100 | ad 100 | ad 100 | ad 100 |

| **Schaum O/W-Emulsionen** | | | | |
|---|---|---|---|---|
| | Emulsion 1 | | Emulsion 2 | |
| | Gew. -% | Vol-% | Gew. -% | Vol-% |
| StearicSäure | 5,00 | | 1,00 | |
| Cetylalkohol | 5,50 | | | |
| Cetylstearylalkohol | | | 2,00 | |
| PEG-40 Stearat | 8,50 | | | |
| PEG-20 Stearat | | | 1,00 | |
| Caprylsäure/Caprinsäuretriglyceride | 4,00 | | 2,00 | |
| C12-15 Alkylbenzoat | 10,00 | | 15,00 | |
| Cyclomethicon | 4,00 | | | |
| Dimethicon | | | 0,50 | |
| Wässrige Lösung mit Keratin Binde-Domäne-Wirkstoff | 5,0 | | 10,0 | |
| Octylisostearat | | | 5,00 | |
| Myristyl Myristat | | | 2,00 | |
| Ceresin | 1,50 | | | |
| Glycerin | | | 3,00 | |
| Filter | | | | |
| Hydroxypropyldistärkephosphat | 1,00 | | 3,50 | |
| BHT | | | 0,02 | |
| Disodium EDTA | 0,50 | | 0,10 | |
| Parfüm, Konservierungsmittel | q.s. | | q.s. | |
| Colorant | q.s. | | q.s. | |
| Kaliumhydroxid | q.s. | | q.s. | |
| Aqua dem. | ad 100 | | ad 100 | |
| | pH einstellend auf 6,5-7,5 | | pH einstellend auf 5,0-6,0 | |
| Emulsion 1 | | 70 | | |
| Emulsion 2 | | | | 35 |
| Gas (Sticksaufff) | | 30 | | |
| Gas (Helium) | | | | 65 |

| **Conditioner Shampoo mit Pearlescent** | | | |
|---|---|---|---|
| | **1** | **2** | **3** |
| Polyquarternium-10 | 0,50 | 0,50 | 0,40 |
| Sodiumlaurethsulfat | 9,00 | 8,50 | 8,90 |
| Cocoamidopropylbetain | 2,50 | 2,60 | 3,00 |
| Benzophenon-4 | 1,50 | 0,50 | 1,00 |
| Wässrige Lösung mit Keratin Binde-Domäne-Wirkstoff | 1,0 | 5,0 | 0,5 |
| Pearlescent Wässrige Lösung mit Keratin Binde-Domäne-Wirkstoff | 2,00 | 2,50 | |
| Disodium EDTA | 0,10 | 0,15 | 0,05 |
| Konservierungsmittele, Parfüm, thickener | q.s. | q.s. | q.s. |
| Aqua dem. | ad 100 | ad 100 | ad 100 |
| pH einstellen 6,0 | | | |

| **Klares Conditioner Shampoo** | | | |
|---|---|---|---|
| | **1** | **2** | **3** |
| Polyquarternium-10 | 0,50 | 0,50 | 0,50 |
| Sodiumlaurethsulfat | 9,00 | 8,50 | 9,50 |
| Wässrige Lösung mit Keratin Binde-Domäne-Wirkstoff | 5,0 | 0,1 | 3,0 |
| Benzophenon-3 | 1,00 | 1,50 | 0,50 |
| ImidosuccinicSäure, Na | 0,20 | 0,20 | 0,80 |
| Konservierungsmittele, Parfüm, thickener | q.s. | q.s. | q.s. |
| Aqua dem. | ad 100 | ad 100 | ad 100 |
| pH einstellen 6,0 | | | |

| **Klares Conditioner Shampoo mit Volumenn Effekt** | | | |
|---|---|---|---|
| | **1** | **2** | **3** |
| Natriumlaurethsulfat | 10,00 | 10,50 | 11,00 |
| Ethylhexyl Methoxycinnamat | 2,00 | 1,50 | 2,30 |
| Wässrige Lösung mit Keratin Binde-Domäne-Wirkstoff | 10,0 | 0,1 | 0,5 |
| Cocoamidopropylbetain | 2,50 | 2,60 | 2,20 |
| Disodium EDTA | 0,01 | 0,10 | 0,01 |
| Konservierungsmittele, Parfüm, thickener | q.s. | q.s. | q.s. |
| Aqua dem. | ad 100 | ad 100 | ad 100 |
| pH einstellen 6,0 | | | |

| **Gelcreme** | | | | |
|---|---|---|---|---|
| | **1** | **2** | **3** | **4** |
| Acrylat/C10-30 Alkylacrylat Crosspolymer | 0,40 | 0,35 | 0,40 | 0,35 |
| PolyacrylicSäure | 0,20 | 0,22 | 0,20 | 0,22 |
| Xanthan Gummi | 0,10 | 0,13 | 0,10 | 0,13 |
| Cetearylalkohol | 3,00 | 2,50 | 3,00 | 2,50 |
| C12-15 Alkylbenzoat | 4,00 | 4,50 | 4,00 | 4,50 |
| Caprylic/Capric Triglycerid | 3,00 | 3,50 | 3,00 | 3,50 |
| Uvinul A Plus^{™} | 2,00 | 1,50 | 0,75 | 1,00 |
| UVASorb K2A | | 3,00 | | |
| Ethylhexyl Methoxycinnamat | 3,00 | | 1,00 | |
| Bis-Ethylhexyloxyphenol methoxyphenyl Triazin | | 1,50 | | 2,00 |
| Butyl Methoxydibenzoylmethan | | | 2,00 | |
| Disodium Phenyl Dibenzimidazol Tetrasulfonat | 2,50 | | 0,50 | 2,00 |
| Ethyhexyl Triazon | 4,00 | | 3,00 | 4,00 |
| Octocrylen | | 4,00 | | |
| Diethylhexyl Butamido Triazon | 1,00 | | | 2,00 |
| Phenylbenzimidazol Sulfonsäure | 0,50 | | 3,00 | |
| Methylen Bis-Benzotriazolyl Tetramethylbutylp-henol | 2,00 | | 0,50 | 1,50 |
| Ethylhexysalicylat | | | 3,00 | |
| Drometrizol Trisiloxan | | | 0,50 | |
| Terephthaliden Dicamphor Sulfonsäure | | 1,50 | | 1,00 |
| Diethylhexyl-2,6-naphthalat | 3,50 | 4,00 | 7,00 | 9,00 |
| Titaniumdioxide- microfine | 1,00 | | 3,00 | |
| Zincoxide- microfine | | | | 0,25 |
| Wässrige Lösung mit Keratin Binde-Domäne-Wirkstoff | 0,1 | 0,5 | 1,0 | 0,02 |
| Cyclisches Dimethylpolysiloxan | 5,00 | 5,50 | 5,00 | 5,50 |
| Dimethicon Polydimethylsiloxan | 1,00 | 0,60 | 1,00 | 0,60 |
| Glycerin | 1,00 | 1,20 | 1,00 | 1,20 |
| Natriumhydroxid | q.s. | q.s. | q.s. | q.s. |
| Konservierungsmittele | 0,30 | 0,23 | 0,30 | 0,23 |
| Parfüm | 0,20 | | 0,20 | |
| Aqua dem. | ad 100 | ad 100 | ad 100 | ad 100 |
| pH einstellen auf 6,0 | | | | |

| **OW Sunscreenformulation** | | | | | | | |
|---|---|---|---|---|---|---|---|
| | **1** | **2** | **3** | **4** | **5** | **6** | **7** |
| Glycerinmonostearat SE | 0,50 | 1,00 | 3,00 | | | 1,50 | |
| Glycerl Stearat Citrat | 2,00 | | 1,00 | 2,00 | 4,00 | | |
| StearicSäure | | 3,00 | | 2,00 | | | |
| PEG-40 Stearat | 0,50 | | | | | 2,00 | |
| Cetyl Phosphat | | | | | | 1,00 | |
| Cetearyl Sulfat | | | | | | | 0,75 |
| Stearyl Alkohol | | | 3,00 | | | 2,00 | 0,60 |
| Cetyl Alkohol | 2,50 | 1,10 | | 1,50 | 0,60 | | 2,00 |
| Wässrige Lösung mit Keratin Binde-Domäne-Wirkstoff | 10,0 | 0,5 | 3,0 | 5,0 | 0,1 | 0,02 | 7,5 |
| Uvinul A Plus^{™} | 2,00 | 1,50 | 0,75 | 1,00 | 2,10 | 4,50 | 5,00 |
| UVASorb K2A | | | | | | | |
| Ethylhexyl Methoxycinnamat | | | | | 5,00 | 6,00 | 8,00 |
| Bis-Ethylhexyloxyphenol methoxyphenyl Triazin | | 1,50 | | 2,00 | 2,50 | | 2,50 |
| Butyl Methoxydibenzoylmethan | | | 2,00 | | 2,00 | 1,50 | |
| Dinatrium Phenyl Dibenzimidazol Tetrasulfonat | 2,50 | | 0,50 | 2,00 | | 0,30 | |
| Ethyhexyl Triazon | 4,00 | | 3,00 | 4,00 | | 2,00 | |
| Octocrylen | | 4,00 | | | | | 7,50 |
| Diethylhexyl Butamido Triazon | 1,00 | | | 2,00 | 1,00 | | 1,00 |
| Phenylbenzimidazol Sulfonsäure | 0,50 | | 3,00 | | | | |
| Methylen Bis-Benzotriazolyl Tetramethylbutylphenol | 2,00 | | 0,50 | 1,50 | 2,50 | | |
| Ethylhexysalicylat | | | 3,00 | | | | 5,00 |
| Drometrizol Trisiloxan | | | 0,50 | | | 1,00 | |
| Terephthaliden Dicamphor Sulfon Säu-re | | 1,50 | | 1,00 | 1,00 | | 0,50 |
| Diethylhexyl-2,6-naphthalat | 3,50 | | 7,00 | | 6,00 | 9,00 | |
| Titandioxid- microfine | 1,00 | | 3,00 | | 3,50 | | 1,50 |
| Zinkoxid- microfine | | | | 0,25 | | 2,00 | |
| C12-15 Alkyl Benzoat | | 0,25 | | | 4,00 | 7,00 | |
| Dicapryl Ether | | | 3,50 | | 2,00 | | |
| Butylenglycol Dicaprylat/Dicaprat | 5,00 | | 6,00 | | | | |
| Cocoglyceride | | | 6,00 | | 2,00 | | |
| Dimethicon | 0,50 | | 1,00 | | 2,00 | | |
| Cyclomethicon | 2,00 | | 0,50 | | 0,50 | | |
| Shea Butter | | 2,00 | | | | | |
| PVP Hexadecen Copolymer | 0,20 | | | 0,50 | | 1,00 | |
| Glycerin | 3,00 | 7,50 | | 7,50 | 5,00 | | 2,50 |
| Xanthan Gummi | 0,15 | | 0,05 | | | 0,30 | |
| Sodium Carbomer | | 0,20 | | 0,15 | 0,25 | | |
| Vitamin E Acetat | 0,60 | | 0,23 | | 0,70 | 1,00 | |
| Fucogel 1000 | | 3,00 | 10,00 | | | | |
| Glycin Soja | | | | 0,50 | | 1,50 | 1,00 |
| Ethylhexyloxyglycin | 0,30 | | | | | | |
| DMDM Hydanaufin | | 0,60 | 0,40 | 0,20 | | | |
| Glyacil-L | | | | 0,18 | 0,20 | | |
| Methylparaben | 0,15 | | 0,25 | | 0,50 | | |
| Phenoxyethanol | 1,00 | 0,40 | | | 0,40 | 0,50 | 0,40 |
| Trinatrium EDTA | 0,02 | | 0,05 | | | | |
| IminosuccinicSäure | | | | 0,25 | 1,00 | | |
| Ethanol | 2,00 | 1,50 | | 3,00 | | 1,20 | 5,00 |
| Parfüm | 0,10 | 0,25 | 0,30 | | 0,40 | 0,20 | |
| Aqua dem. | ad 100 | ad 100 | ad 100 | ad 100 | ad 100 | ad100 | ad 100 |

| **Hydrodispersion** | | | | | |
|---|---|---|---|---|---|
| | **1** | **2** | **3** | **4** | **5** |
| Ceteaereth-20 | 1,00 | | | 0,50 | |
| Cetyl Alkohol | | | 1,00 | | |
| Sodium Carbomer | | 0,20 | | 0,30 | |
| Acrylat/C10-30 Alkyl Acrylat Crosspolymer | 0,50 | | 0,40 | 0,10 | 0,50 |
| Xanthan Gummi | | 0,30 | 0,15 | | |
| Wässrige Lösung mit Keratin Binde-Domäne-Wirkstoff | 5,0 | 0,5 | 3,0 | 0,1 | 10,0 |
| Uvinul A Plus^{™} | 2,00 | 1,50 | 0,75 | 1,00 | 2,10 |
| UVASorb K2A | | 3,50 | | | |
| Ethylhexyl Methoxycinnamat | | | | | 5,00 |
| Bis-Ethylhexyloxyphenol methoxyphenyl Triazin | | 1,50 | | 2,00 | 2,50 |
| Butyl Methoxydibenzoylmethan | | | 2,00 | | 2,00 |
| Dinatrium Phenyl Dibenzimidazol Tetrasul-fonat | 2,50 | | 0,50 | 2,00 | |
| Ethyhexyl Triazon | 4,00 | | 3,00 | 4,00 | |
| Octocrylen | | 4,00 | | | |
| Diethylhexyl Butamido Triazon | 1,00 | | | 2,00 | 1,00 |
| Phenylbenzimidazol Sulfonsäure | 0,50 | | 3,00 | | |
| Methylen Bis-Benzotriazolyl Tetramethylbutylphenol | 2,00 | | 0,50 | 1,50 | 2,50 |
| Ethylhexysalicylat | | | 3,00 | | |
| Drometrizol Trisiloxan | | | 0,50 | | |
| Terephthaliden Dicamphor Sulfonsäure | | 1,50 | | 1,00 | 1,00 |
| Diethylhexyl-2,6-naphthalat | | | 7,00 | | 9,00 |
| Titaniumdioxide- microfine | 1,00 | | 3,00 | | 3,50 |
| Zincoxide- microfine | | | | 0,25 | |
| C12-15 Alkyl Benzoat | 2,00 | 2,50 | | | |
| Dicapryl Ether | | 4,00 | | | |
| Butylenglycol Dicaprylat/Dicaprat | 4,00 | | 2,00 | 6,00 | |
| Dicapryl Carbonat | | 2,00 | 6,00 | | |
| Dimethicon | | 0,50 | 1,00 | | |
| Phenyltrimethicon | 2,00 | | 0,50 | | |
| Shea Butter | | 2,00 | | 5,00 | |
| PVP Hexadecen Copolymer | 0,50 | | | 0,50 | 1,00 |
| Tricontanyl PVP | 0,50 | | 1,00 | | |
| Ethylhexylglycerin | | | 1,00 | | 0,80 |
| Glycerin | 3,00 | 7,50 | | 7,50 | 8,50 |
| Gylcin Soja | | | 1,50 | | 1,00 |
| Vitamin E Acetat | 0,50 | | 0,25 | | 1,00 |
| Alpha-Glucosilrutin | 0,60 | | | 0,25 | |
| Fucogel 1000 | | 2,50 | 0,50 | | 2,00 |
| DMDM Hydanaufin | | 0,60 | 0,45 | 0,25 | |
| Glyacil-S | 0,20 | | | | |
| Methylparaben | 0,50 | | 0,25 | 0,15 | |
| Phenoxyethanol | 0,50 | 0,40 | | 1,00 | |
| Trinatrium EDTA | | 0,01 | 0,05 | | 0,10 |
| Ethanol | 3,00 | 2,00 | 1,50 | | 7,00 |
| Parfüm | 0,20 | | 0,05 | 0,40 | |
| Aqua dem. | ad 100 | ad 100 | ad 100 | ad 100 | ad 100 |

| **WO Sunscreen Emulsion** | | | | | |
|---|---|---|---|---|---|
| | **1** | **2** | **3** | **4** | **5** |
| Cetyldimethicon Copolyol | | 2,50 | | 4,00 | |
| Polyglyceryl-2-dipolyhydroxystearat | 5,00 | | | | 4,50 |
| PEG-30-dipolyhydroxystearat | | | 5,00 | | |
| Wässrige Lösung mit Keratin Binde-Domäne-Wirkstoff | 5,0 | 1,0 | 10,0 | 0,5 | 0,1 |
| Uvinul A Plus^{™} | 2,00 | 1,50 | 0,75 | 1,00 | 2,10 |
| UVASorb K2A | | 2,00 | | | |
| Ethylhexyl Methoxycinnamat | | | | | 5,00 |
| Bis-Ethylhexyloxyphenol methoxyphenyl Triazin | | 1,50 | | 2,00 | 2,50 |
| Butyl Methoxydibenzoylmethan | | | 2,00 | | 2,00 |
| Dinatrium Phenyl Dibenzimidazol Tetrasulfonat | 2,50 | | 0,50 | 2,00 | |
| Ethyhexyl Triazon | 4,00 | | 3,00 | 4,00 | |
| Octocrylen | | 4,00 | | | |
| Diethylhexyl Butamido Triazon | 1,00 | | | 2,00 | 1,00 |
| Phenylbenzimidazol Sulfonsäure | 0,50 | | 3,00 | | |
| Methylen Bis-Benzotriazolyl Tetramethylbutylphenol | 2,00 | | 0,50 | 1,50 | 2,50 |
| Ethylhexysalicylat | | | 3,00 | | |
| Drometrizol Trisiloxan | | | 0,50 | | |
| Terephthaliden Dicamphor Sulfonsäure | | 1,50 | | 1,00 | 1,00 |
| Diethylhexyl-2,6-naphthalat | | | 7,00 | | 4,00 |
| Titaniumdioxide- microfine | 1,00 | | 3,00 | | 3,50 |
| Zincoxide- microfine | | | | 0,25 | |
| MineralÖl | | 12,00 | 10,00 | | 8,00 |
| C12-15 Alkyl Benzoat | | | | 9,00 | |
| Dicaprylyl Ether | 10,00 | | | | 7,00 |
| Butylenglycol Dicaprylat/Dicaprat | | | 2,00 | 8,00 | 4,00 |
| Dicaprylyl Carbonat | 5,00 | | 6,00 | | |
| Dimethicon | | 4,00 | 1,00 | 5,00 | |
| Cyclomethicon | 2,00 | 25,00 | | | 2,00 |
| Shea Butter | | | 3,00 | | |
| Vaseline | | 4,50 | | | |
| PVP Hexadecen Copolymer | 0,50 | | | 0,50 | 1,00 |
| Ethylhexylglycerin | | 0,30 | 1,00 | | 0,50 |
| Glycerin | 3,00 | 7,50 | | 7,50 | 8,50 |
| Glycin Soja | | 1,00 | 1,50 | | 1,00 |
| MgSO4 | 1,00 | 0,50 | | 0,50 | |
| MgCl2 | | | 1,00 | | 0,70 |
| Vitamin E Acetat | 0,50 | | 0,25 | | 1,00 |
| Ascorbyl Palmitat | 0,50 | | | 2,00 | |
| Fucogel 1000 | | | | 3,50 | 1,00 |
| DMDM Hydanaufin | | 0,60 | 0,40 | 0,20 | |
| Methylparaben | 0,50 | | 0,25 | 0,15 | |
| Phenoxyethanol | 0,50 | 0,40 | | 1,00 | |
| Trisodium EDTA | 0,12 | 0,05 | | 0,30 | |
| Ethanol | 3,00 | | 1,50 | | 5,00 |
| Parfüm | 0,20 | | 0,40 | 0,35 | |
| Aqua dem. | ad 100 | ad 100 | ad 100 | ad 100 | ad 100 |

| **Sticks** | | | | |
|---|---|---|---|---|
| | **1** | **2** | **3** | **4** |
| Caprylic/Capric Triglycerid | 12,00 | 10,00 | 6,00 | |
| Octyldodecanol | 7,00 | 14,00 | 8,00 | 3,00 |
| Butylene Glycol Dicaprylat/Dicaprat | | | | 12,00 |
| Pentaerythrityl Tetraisostearat | 10,00 | 6,00 | 8,00 | 7,00 |
| Polyglyceryl-3 Diisostearat | 2,50 | | | |
| Bis-Diglyceryl Polyacyladipate-2 | 9,00 | 8,00 | 10,00 | 8,00 |
| Cetearyl Alcohol | 8,00 | 11,00 | 9,00 | 7,00 |
| Myristyl Myristate | 3,50 | 3,00 | 4,00 | 3,00 |
| Beeswax | 5,00 | 5,00 | 6,00 | 6,00 |
| Cera Camauba | 1,50 | 2,00 | 2,00 | 1,50 |
| Cera Alba | 0,50 | 0,50 | 0,50 | 0,40 |
| C16-40 Alkyl Stearat | | 1,50 | 1,50 | 1,50 |
| Wässrige Lösung mit Keratin Binde-Domäne-Wirkstoff | 0,5 | 3,0 | 1,0 | 5,0 |
| Uvinul A Plus^{™} | 2,00 | 1,50 | 0,75 | 9,00 |
| UVASorb K2A | | 2,00 | | 4,00 |
| Ethylhexyl Methoxycinnamat | | 3,00 | | |
| Bis-Ethylhexyloxyphenol methoxyphenyl Triazin | | 1,50 | | 2,00 |
| Butyl Methoxydibenzoylmethan | | | 2,00 | |
| Dinatrium Phenyl Dibenzimidazol Tetrasulfonat | 2,50 | | 0,50 | 2,00 |
| Ethyhexyl Triazon | 4,00 | | 3,00 | 4,00 |
| Octocrylen | | 4,00 | | |
| Diethylhexyl Butamido Triazon | 1,00 | | | 2,00 |
| Phenylbenzimidazol Sulfonsäure | 0,50 | | 3,00 | |
| Methylen Bis-Benzotriazolyl Tetramethylbutylphenol | 2,00 | | 0,50 | 1,50 |
| Ethylhexysalicylat | | | 3,00 | |
| Drometrizol Trisiloxan | | | 0,50 | |
| Terephthaliden Dicamphor Sulfonsäure | | 1,50 | | 1,00 |
| Diethylhexyl-2,6-naphthalat | | | 7,00 | |
| Titaniumdioxide- microfine | 1,00 | | 3,00 | |
| Zincoxide- microfine | | | | 0,25 |
| Vitamin E Acetat | 0,50 | 1,00 | | |
| Ascorbyl Palmitat | 0,05 | | 0,05 | |
| Buxux Chinensis | 2,00 | 1,00 | | 1,00 |
| Parfüm, BHT | 0,10 | 0,25 | | 0,35 |
| Ricinus Communis | ad 100 | ad 100 | ad 100 | ad 100 |

| **PIT-Emulsion** | | | | | | | | |
|---|---|---|---|---|---|---|---|---|
| | **1** | **2** | **3** | **4** | **5** | **6** | **7** | **8** |
| Glycerinmonostearat SE | 0,50 | 2,00 | 3,00 | 5,00 | | 0,50 | 4,00 | |
| Glyceryl Isostearat | | | | | 3,50 | 4,00 | 2,00 | |
| Isoceteth-20 | | 0,50 | | | 2,00 | | | |
| Ceteareth-12 | | 5,00 | | 1,00 | | | 3,50 | 5,00 |
| Ceteareth-20 | | 5,00 | | 1,00 | | | | 3,50 |
| PEG-100 Stearat | | | | 2,80 | | 2,30 | 3,30 | |
| Cetyl Alkohol | 5,20 | | 1,20 | 1,00 | 1,30 | | 0,50 | 0,30 |
| Cetyl Palmitat | 2,50 | 1,20 | | 1,50 | | 0,50 | | 1,50 |
| Cetyl Dimethicon Copolyol | | | | 0,50 | | 1,00 | | |
| Polyglyceryl-2 | | | | 0,75 | 0,30 | | | |
| Wässrige Lösung mit Keratin Binde-Domäne-Wirkstoff | 0,1 | 5,0 | 0,01 | 0,5 | 3,0 | 0,25 | 10,0 | 3,0 |
| Uvinul A Plus^{™} | 2,00 | 1,50 | 0,75 | 1,00 | 2,10 | 4,50 | 5,00 | 2,10 |
| UVASorb K2A | | | 4,00 | | | | 1,50 | |
| Ethylhexyl Methoxycinnamat | | | | | 5,00 | 6,00 | 8,00 | 5,00 |
| Bis-Ethylhexyloxyphenol methoxyphenyl Triazin | | 1,50 | | 2,00 | 2,50 | | 2,50 | 2,50 |
| Butyl Methoxydibenzoylmethan | | | 2,00 | | 2,00 | 1,50 | | 2,00 |
| Dinatrium Phenyl Dibenzimidazol Tetrasulfonat | 2,50 | | 0,50 | 2,00 | | 0,30 | | |
| Ethyhexyl Triazon | 4,00 | | 3,00 | 4,00 | | 2,00 | | |
| Octocrylen | | 4,00 | | | | | 7,50 | |
| Diethylhexyl Butamido Triazon | 1,00 | | | 2,00 | 1,00 | | 1,00 | 1,00 |
| Phenylbenzimidazol Sulfonsäure | 0,50 | | 3,00 | | | | | |
| Methylen Bis-Benzotriazolyl Tetramethylbutylphenol | 2,00 | | 0,50 | 1,50 | 2,50 | | | 2,50 |
| Ethylhexysalicylat | | | 3,00 | | | | 5,00 | |
| Drometrizol Trisiloxan | | | 0,50 | | | 1,00 | | |
| Terephthaliden Dicamphor Sulfonsäure | | 1,50 | | 1,00 | 1,00 | | 0,50 | 1,00 |
| Diethylhexyl-2,6-naphthalat | | | 7,00 | | 10,0 0 | 7,50 | | 8,00 |
| Titandioxid- microfine | 1,00 | | 3,00 | | 3,50 | | 1,50 | 3,50 |
| Zinkoxid- microfine | | | | 0,25 | | 2,00 | | |
| C12-15 Alkyl Benzoat | 3,50 | | | 6,35 | | | | 0,10 |
| Cocoglyceride | | 3,00 | | 3,00 | | | | 1,00 |
| Dicapryl Ether | 4,50 | | | | | | | |
| Dicaprylyl Carbonat | | 4,30 | | 3,00 | | | | 7,00 |
| Dibutyl Adipate | | | | 0,50 | | | | 0,30 |
| Phenyltrimethicone | 2,00 | | | 3,50 | | 2,00 | | |
| Cyclomethicon | | 3,00 | | | | | | |
| Ethyl Galakaufmannan | | 0,50 | | | 2,00 | | | |
| Hydrierte Coco-Glyceride | | | | | 3,00 | 4,00 | | |
| Abil Wax 2440 | | | | | | 1,50 | 2,00 | |
| PVP Hexadecen Copolymer | | | | 1,00 | 1,20 | | | |
| Glycerin | 4,00 | 6,00 | 5,00 | | 8,00 | 10,0 0 | | |
| Vitamin E Acetat | 0,20 | 0,30 | 0,40 | | 0,30 | | | |
| Shea Butter | | 2,00 | | 3,60 | | 2,00 | | |
| lodopropyl Butylcarbamat | 0,12 | | | | 0,20 | | | |
| Fucogel 1000 | | | | 0,10 | | | | |
| DMDM Hydanaufin | 0,10 | | | | 0,12 | | 0,13 | |
| Methylparaben | | 0,50 | 0,30 | | 0,35 | | | |
| Phenoxyethanol | 0,50 | 0,40 | | 1,00 | | | | |
| Ocaufxyglycerin | | 0,30 | | | 1,00 | | 0,35 | |
| Ethanol | 2,00 | | 2,00 | | | 5,00 | | |
| Trinatrium EDTA | 0,40 | | 0,15 | | | 0,20 | | |
| Parfüm | 0,20 | | 0,20 | | 0,24 | 0,16 | 0,10 | 0,10 |
| Aqua dem. | ad 100 | ad 100 | ad 100 | ad 100 | ad 100 | ad 100 | ad 100 | ad 100 |

| **Gelcreme** | | | | |
|---|---|---|---|---|
| | **1** | **2** | **3** | **4** |
| Acrylat/C10-30 Alkylacrylat Crosspolymer | 0,40 | 0,35 | 0,40 | 0,35 |
| PolyacrylicSäure | 0,20 | 0,22 | 0,20 | 0,22 |
| Luvigel EM | 1,50 | 2,50 | 2,80 | 3,50 |
| Xanthan Gummi | 0,10 | 0,13 | 0,10 | 0,13 |
| Cetearylalkohol | 3,00 | 2,50 | 3,00 | 2,50 |
| C12-15 Alkylbenzoat | 4,00 | 4,50 | 4,00 | 4,50 |
| Caprylic/Capric Triglycerid | 3,00 | 3,50 | 3,00 | 3,50 |
| Titandioxid- microfine | 1,00 | | 1,50 | |
| Zincoxide- microfine | | 2,00 | | 0,25 |
| Wässrige Lösung mit Keratin Binde-Domäne-Wirkstoff | 0,5 | 10,0 | 3,0 | 5,0 |
| Dihydroxyaceaufn | | | 3,00 | 5,00 |
| Cyclisches Dimethylpolysiloxan | 5,00 | 5,50 | 5,00 | 5,50 |
| Dimethicon Polydimethylsiloxan | 1,00 | 0,60 | 1,00 | 0,60 |
| Glycerin | 1,00 | 1,20 | 1,00 | 1,20 |
| Natriumhydoxid | q.s. | q.s. | q.s. | q.s. |
| Konservierungsmitteles | 0,30 | 0,23 | 0,30 | 0,23 |
| Parfüm | 0,20 | | 0,20 | |
| Aqua dem. | ad 100 | ad 100 | ad 100 | ad 100 |
| pH einstellen auf 6,0 | | | | |

| **OW Formulations Selbstbräuner** | | | | | | | |
|---|---|---|---|---|---|---|---|
| | **1** | **2** | **3** | **4** | **5** | **6** | **7** |
| Glycerinmonostearat SE | 0,50 | 1,00 | 3,00 | | | 1,50 | |
| Glycerl Stearat Citrat | 2,00 | | 1,00 | 2,00 | 4,00 | | |
| StearicSäure | | 3,00 | | 2,00 | | | |
| PEG-40 Stearat | 0,50 | | | | | 2,00 | |
| Cetyl Phosphat | | | | | | 1,00 | |
| Cetearyl Sulfat | | | | | | | 0,75 |
| Stearyl Alkohol | | | 3,00 | | | 2,00 | 0,60 |
| Cetyl Alkohol | 2,50 | 1,10 | | 1,50 | 0,60 | | 2,00 |
| Wässrige Lösung mit Keratin Binde-Domäne-Wirkstoff | 0,1 | 0,5 | 0,025 | 5,0 | 3,0 | 10,0 | 1,0 |
| Dihydroxyaceaufn | | | 3,00 | 5,00 | | 4 | |
| Titandioxid- microfine | 1,00 | | | | 1,50 | | 1,50 |
| Zinkoxid- microfine | | | | 0,25 | | 2,00 | |
| C12-15 Alkyl Benzoat | | 0,25 | | | 4,00 | 7,00 | |
| Dicapryl Ether | | | 3,50 | | 2,00 | | |
| Butylenglycol Dicaprylat/Dicaprat | 5,00 | | 6,00 | | | | |
| Cocoglyceride | | | 6,00 | | 2,00 | | |
| Dimethicon | 0,50 | | 1,00 | | 2,00 | | |
| Cyclomethicon | 2,00 | | 0,50 | | 0,50 | | |
| Shea Butter | | 2,00 | | | | | |
| PVP Hexadecen Copolymer | 0,20 | | | 0,50 | | 1,00 | |
| Glycerin | 3,00 | 7,50 | | 7,50 | 5,00 | | 2,50 |
| Xanthan Gummi | 0,15 | | 0,05 | | | 0,30 | |
| Sodium Carbomer | | 0,20 | | 0,15 | 0,25 | | |
| Vitamin E Acetat | 0,60 | | 0,23 | | 0,70 | 1,00 | |
| Fucogel 1000 | | 3,00 | 10,00 | | | | |
| Glycin Soja | | | | 0,50 | | 1,50 | 1,00 |
| Ethylhexyloxyglycin | 0,30 | | | | | | |
| DMDM Hydanaufin | | 0,60 | 0,40 | 0,20 | | | |
| Glyacil-L | | | | 0,18 | 0,20 | | |
| Methylparaben | 0,15 | | 0,25 | | 0,50 | | |
| Phenoxyethanol | 1,00 | 0,40 | | | 0,40 | 0,50 | 0,40 |
| Trinatrium EDTA | 0,02 | | 0,05 | | | | |
| Iminobernsteinsäure | | | | 0,25 | 1,00 | | |
| Ethanol | 2,00 | 1,50 | | 3,00 | | 1,20 | 5,00 |
| Parfüm | 0,10 | 0,25 | 0,30 | | 0,40 | 0,20 | |
| Aqua dem. | ad 100 | ad 100 | ad 100 | ad 100 | ad 100 | ad100 | ad 100 |

| **OW Make Up** | | | | | | | |
|---|---|---|---|---|---|---|---|
| | **1** | **2** | **3** | **4** | **5** | **6** | **7** |
| Glycerinmonostearat SE | 0,50 | 1,00 | 3,00 | | | 1,50 | |
| Glycerl Stearat Citrat | 2,00 | | 1,00 | 2,00 | 4,00 | | |
| StearicSäure | | 3,00 | | 2,00 | | | |
| PEG-40 Stearat | 0,50 | | | | | 2,00 | |
| Cetyl Phosphat | | | | | | 1,00 | |
| Cetearyl Sulfat | | | | | | | 0,75 |
| Stearyl Alkohol | | | 3,00 | | | 2,00 | 0,60 |
| Cetyl Alkohol | 2,50 | 1,10 | | 1,50 | 0,60 | | 2,00 |
| Wässrige Lösung mit Keratin Binde-Domäne-Wirkstoff | 3,0 | 5,0 | 2,0 | 0,5 | 1,0 | 5,0 | 10,0 |
| Titaniumoxide | 10,00 | 12,00 | 9,00 | 8,50 | 11,00 | 9,50 | 10,00 |
| Ironoxide | 2,00 | 4,00 | 3,00 | 5,00 | 3,40 | 6,00 | 4,40 |
| Zincoxide | | 4,00 | | 2,00 | | 3,00 | |
| C12-15 Alkyl Benzoat | | 0,25 | | | 4,00 | 7,00 | |
| Dicapryl Ether | | | 3,50 | | 2,00 | | |
| Butylenglycol Dicaprylat/Dicaprat | 5,00 | | 6,00 | | | | |
| Cocoglyceride | | | 6,00 | | 2,00 | | |
| Dimethicon | 0,50 | | 1,00 | | 2,00 | | |
| Cyclomethicon | 2,00 | | 0,50 | | 0,50 | | |
| Shea Butter | | 2,00 | | | | | |
| PVP Hexadecen Copolymer | 0,20 | | | 0,50 | | 1,00 | |
| Glycerin | 3,00 | 7,50 | | 7,50 | 5,00 | | 2,50 |
| Xanthan Gummi | 0,15 | | 0,05 | | | 0,30 | |
| Sodium Carbomer | | 0,20 | | 0,15 | 0,25 | | |
| Vitamin E Acetat | 0,60 | | 0,23 | | 0,70 | 1,00 | |
| Glycin Soja | | | | 0,50 | | 1,50 | 1,00 |
| Ethylhexyloxyglycin | 0,30 | | | | | | |
| DMDM Hydanaufin | | 0,60 | 0,40 | 0,20 | | | |
| Glyacil-L | | | | 0,18 | 0,20 | | |
| Methylparaben | 0,15 | | 0,25 | | 0,50 | | |
| Phenoxyethanol | 1,00 | 0,40 | | | 0,40 | 0,50 | 0,40 |
| Trinatrium EDTA | 0,02 | | 0,05 | | | | |
| IminosuccinicSäure | | | | 0,25 | 1,00 | | |
| Ethanol | 2,00 | 1,50 | | 3,00 | | 1,20 | 5,00 |
| Parfüm | 0,10 | 0,25 | 0,30 | | 0,40 | 0,20 | |
| Aqua dem. | ad 100 | ad 100 | ad 100 | ad 100 | ad 100 | ad100 | ad 100 |

| **Hydrodispersion Selbstbräuner** | | | | | |
|---|---|---|---|---|---|
| | **1** | **2** | **3** | **4** | **5** |
| Ceteaereth-20 | 1,00 | | | 0,50 | |
| Cetyl Alkohol | | | 1,00 | | |
| Luvigel EM | | 2,00 | | 2,50 | 2,00 |
| Acrylat/C10-30 Alkyl Acrylat Crosspolymer | 0,50 | | 0,40 | 0,10 | 0,50 |
| Xanthan Gummi | | 0,30 | 0,15 | | |
| Wässrige Lösung mit Keratin Binde-Domäne-Wirkstoff | 3,0 | 1,0 | 0,5 | 0,1 | 5,0 |
| Dihydroxyaceaufn | | | 3,00 | 5,00 | |
| Uvinul A Plus^{™} | 2,00 | 1,50 | 0,75 | 1,00 | 2,10 |
| Titandioxid- microfine | 1,00 | | 1,00 | | 1,00 |
| Zinkoxid- microfine | | 1,90 | | 0,25 | |
| C12-15 Alkyl Benzoat | 2,00 | 2,50 | | | |
| Dicapryl Ether | | 4,00 | | | |
| Butylenglycol Dicaprylat/Dicaprat | 4,00 | | 2,00 | 6,00 | |
| Dicapryl Carbonat | | 2,00 | 6,00 | | |
| Dimethicon | | 0,50 | 1,00 | | |
| Phenyltrimethicon | 2,00 | | 0,50 | | |
| Shea Butter | | 2,00 | | 5,00 | |
| PVP Hexadecen Copolymer | 0,50 | | | 0,50 | 1,00 |
| Tricontanyl PVP | 0,50 | | 1,00 | | |
| Ethylhexylglycerin | | | 1,00 | | 0,80 |
| Glycerin | 3,00 | 7,50 | | 7,50 | 8,50 |
| Gylcin Soja | | | 1,50 | | 1,00 |
| Vitamin E Acetat | 0,50 | | 0,25 | | 1,00 |
| Alpha-Glucosilrutin | 0,60 | | | 0,25 | |
| DMDM Hydanaufin | | 0,60 | 0,45 | 0,25 | |
| Glyacil-S | 0,20 | | | | |
| Methylparaben | 0,50 | | 0,25 | 0,15 | |
| Phenoxyethanol | 0,50 | 0,40 | | 1,00 | |
| Trinatrium EDTA | | 0,01 | 0,05 | | 0,10 |
| Ethanol | 3,00 | 2,00 | 1,50 | | 7,00 |
| Parfüm | 0,20 | | 0,05 | 0,40 | |
| Wasser | ad 100 | ad 100 | ad 100 | ad 100 | ad 100 |

| **Hydrodispersion After sun** | | | | | |
|---|---|---|---|---|---|
| | **1** | **2** | **3** | **4** | **5** |
| Ceteaereth-20 | 1,00 | | | 0,50 | |
| Cetyl Alkohol | | | 1,00 | | |
| Luvigel EM | | 2,00 | | 2,50 | 2,00 |
| Acrylat/C10-30 Alkyl Acrylat Crosspolymer | 0,50 | 0,30 | 0,40 | 0,10 | 0,50 |
| Xanthan Gummi | | 0,30 | 0,15 | | |
| Wässrige Lösung mit Keratin Binde-Domäne-Wirkstoff | 0,1 | 5,0 | 0,5 | 3,0 | 1,0 |
| C12-15 Alkyl Benzoat | 2,00 | 2,50 | | | |
| Dicapryl Ether | | 4,00 | | | |
| Butylenglycol Dicaprylat/Dicaprat | 4,00 | | 2,00 | 6,00 | |
| Dicapryl Carbonat | | 2,00 | 6,00 | | |
| Dimethicon | | 0,50 | 1,00 | | |
| Phenyltrimethicon | 2,00 | | 0,50 | | |
| Tricontanyl PVP | 0,50 | | 1,00 | | |
| Ethylhexylglycerin | | | 1,00 | | 0,80 |
| Glycerin | 3,00 | 7,50 | | 7,50 | 8,50 |
| Gylcin Soja | | | 1,50 | | 1,00 |
| Vitamin E Acetat | 0,50 | | 0,25 | | 1,00 |
| Alpha-Glucosilrutin | 0,60 | | | 0,25 | |
| Trinatrium EDTA | | 0,01 | 0,05 | | 0,10 |
| Ethanol | 15,00 | 10,00 | 8,00 | 12,00 | 9,00 |
| Parfüm | 0,20 | | 0,05 | 0,40 | |
| Aqua dem. | ad 100 | ad 100 | ad 100 | ad 100 | ad 100 |

| **WO-Emulsions** | | | | | |
|---|---|---|---|---|---|
| | **1** | **2** | **3** | **4** | **5** |
| Cetyldimethicon Copolyol | | 2,50 | | 4,00 | |
| Polyglyceryl-2-dipolyhydroxystearat | 5,00 | | | | 4,50 |
| PEG-30-dipolyhydroxystearat | | | 5,00 | | |
| Wässrige Lösung mit Keratin Binde-Domäne-Wirkstoff | 5,0 | 10,0 | 0,1 | 0,5 | 1,0 |
| Uvinul A Plus^{™} | 2,00 | 1,50 | 0,75 | 1,00 | 2,10 |
| Titaniumdioxide- microfine | 1,00 | | 3,00 | | 3,50 |
| Zincoxide- microfine | | 0,90 | | 0,25 | |
| Mineralöl | | 12,00 | 10,00 | | 8,00 |
| C12-15 Alkyl Benzoat | | | | 9,00 | |
| Dicaprylyl Ether | 10,00 | | | | 7,00 |
| Butylenglycol Dicaprylat/Dicaprat | | | 2,00 | 8,00 | 4,00 |
| Dicaprylyl Carbonat | 5,00 | | 6,00 | | |
| Dimethicon | | 4,00 | 1,00 | 5,00 | |
| Cyclomethicon | 2,00 | 25,00 | | | 2,00 |
| Shea Butter | | | 3,00 | | |
| Vaseline | | 4,50 | | | |
| PVP Hexadecen Copolymer | 0,50 | | | 0,50 | 1,00 |
| Ethylhexylglycerin | | 0,30 | 1,00 | | 0,50 |
| Glycerin | 3,00 | 7,50 | | 7,50 | 8,50 |
| Glycin Soja | | 1,00 | 1,50 | | 1,00 |
| MgSO4 | 1,00 | 0,50 | | 0,50 | |
| MgCl2 | | | 1,00 | | 0,70 |
| Vitamin E Acetat | 0,50 | | 0,25 | | 1,00 |
| Ascorbyl Palmitat | 0,50 | | | 2,00 | |
| Fucogel 1000 | | | | 3,50 | 7,00 |
| DMDM Hydanaufin | | 0,60 | 0,40 | 0,20 | |
| Methylparaben | 0,50 | | 0,25 | 0,15 | |
| Phenoxyethanol | 0,50 | 0,40 | | 1,00 | |
| Trinatrium EDTA | 0,12 | 0,05 | | 0,30 | |
| Ethanol | 3,00 | | 1,50 | | 5,00 |
| Parfüm | 0,20 | | 0,40 | 0,35 | |
| Aqua dem. | ad 100 | ad 100 | ad 100 | ad 100 | ad 100 |

| **Solid stabilized Emulsions (Pickering Emulsions)** | | | | | |
|---|---|---|---|---|---|
| | **1** | **2** | **3** | **4** | **5** |
| MineralÖl | | | 16,00 | 16,00 | |
| Octyldodecanol | 9,00 | 9,00 | 5,00 | | |
| Caprylic/Capric Triglycerid | 9,00 | 9,00 | 6,00 | | |
| C12-15 Alkyl Benzoat | | | | 5,00 | 8,00 |
| Butylen Glycol Dicaprylat/Dicaprat | | | | | 8,00 |
| Dicaprylyl Ether | 9,00 | | | 4,00 | |
| Dicaprylyl Carbonat | | 9,00 | | | |
| Hydroxyoctacosanyl Hydroxystearat | 2,00 | 2,00 | 2,20 | 2,50 | 1,50 |
| Disteardimonium Hecaufrit | 1,00 | 0,75 | | 0,50 | 0,25 |
| Cera Microcristallina + Paraffinum Liquidum | | 0,35 | | | 5,00 |
| Hydroxypropyl Methylcellulose | | | 0,10 | | 0,05 |
| Dimethicon | | | | | 3,00 |
| Wässrige Lösung mit Keratin Binde-Domäne-Wirkstoff | 1,0 | 0,5 | 0,1 | 3,0 | 5,0 |
| Titaniumdioxide + Alumina + Simethicon + Aqua | | 3,00 | | | |
| Titaniumdioxide + Trimethoxycaprylylsilan | | 2,00 | 4,00 | 2,00 | 4,00 |
| Silica Dimethyl Silylat | 2,50 | | | 6,00 | 2,50 |
| Bornitrid | | | 1,00 | | |
| Stärke/-Natriummetaphosphat-Polymer | 2,00 | | | | |
| Tapioca Stärke | | 0,50 | | | |
| Sodium Chlorid | 5,00 | 7,00 | 8,50 | 3,00 | 4,50 |
| Glycerin | | | | 1,00 | |
| Trinatrium EDTA | 1,00 | 1,00 | 1,00 | 1,00 | 1,00 |
| Vitamin E Acetat | 5,00 | 10,00 | 3,00 | 6,00 | 10,00 |
| Ascorbyl Palmitat | 1,00 | 1,00 | | 1,00 | |
| Methylparaben | | 0,60 | | | 0,20 |
| Propylparaben | | | | | 0,20 |
| Phenoxyethanol | | | 0,20 | | |
| Hexamidin Diisethionat | | | 0,40 | 0,50 | 0,40 |
| Diazolidinyl Harnsaufff | | | | | 0,08 |
| Ethanol | | | 0,23 | 0,20 | |
| Parfüm | 5,00 | | 3,00 | 4,00 | |
| Aqua dem. | 0,20 | | 0,30 | 0,10 | |
| | ad 100 | ad 100 | ad 100 | ad 100 | ad 100 |

| **Sticks** | | | | |
|---|---|---|---|---|
| | **1** | **2** | **3** | **4** |
| Caprylic/Capric Triglycerid | 12,00 | 10,00 | 6,00 | |
| Octyldodecanol | 7,00 | 14,00 | 8,00 | 3,00 |
| Butylene Glycol Dicaprylat/Dicaprat | | | | 12,00 |
| Pentaerythrityl Tetraisostearat | 10,00 | 6,00 | 8,00 | 7,00 |
| Polyglyceryl-3 Diisostearat | 2,50 | | | |
| Bis-Diglyceryl Polyacyladipate-2 | 9,00 | 8,00 | 10,00 | 8,00 |
| Cetearyl Alcohol | 8,00 | 11,00 | 9,00 | 7,00 |
| Myristyl Myristate | 3,50 | 3,00 | 4,00 | 3,00 |
| Beeswax | 5,00 | 5,00 | 6,00 | 6,00 |
| Cera Camauba | 1,50 | 2,00 | 2,00 | 1,50 |
| Cera Alba | 0,50 | 0,50 | 0,50 | 0,40 |
| C16-40 Alkyl Stearat | | 1,50 | 1,50 | 1,50 |
| Wässrige Lösung mit Keratin Binde-Domäne-Wirkstoff | 10,0 | 1,0 | 3,0 | 0,1 |
| Uvinul A Plus^{™} | 2,00 | 1,50 | 0,75 | 9,00 |
| Titaniumdioxide- microfine | 1,00 | | 3,00 | |
| Zincoxide- microfine | | 1,00 | | 0,25 |
| Vitamin E Acetat | 0,50 | 1,00 | | |
| Ascorbyl Palmitat | 0,05 | | 0,05 | |
| Buxux Chinensis | 2,00 | 1,00 | | 1,00 |
| Parfüm, BHT | 0,10 | 0,25 | | 0,35 |
| Ricinus Communis | ad 100 | ad 100 | ad 100 | ad 100 |

| **PIT-Emulsionen Selbstbräuner** | | | | | | | | |
|---|---|---|---|---|---|---|---|---|
| | **1** | **2** | **3** | **4** | **5** | **6** | **7** | **8** |
| Glycerinmonostearat SE | 0,50 | 2,00 | 3,00 | 5,00 | | 0,50 | 4,00 | |
| Glyceryl Isostearat | | | | | 3,50 | 4,00 | 2,00 | |
| Isoceteth-20 | | 0,50 | | | 2,00 | | | |
| Ceteareth-12 | | 5,00 | | 1,00 | | | 3,50 | 5,00 |
| Ceteareth-20 | | 5,00 | | 1,00 | | | | 3,50 |
| PEG-100 Stearat | | | | 2,80 | | 2,30 | 3,30 | |
| Cetyl Alkohol | 5,20 | | 1,20 | 1,00 | 1,30 | | 0,50 | 0,30 |
| Cetyl Palmitat | 2,50 | 1,20 | | 1,50 | | 0,50 | | 1,50 |
| Cetyl Dimethicon Copolyol | | | | 0,50 | | 1,00 | | |
| Polyglyceryl-2 | | | | 0,75 | 0,30 | | | |
| Wässrige Lösung mit Keratin Binde-Domäne-Wirkstoff | 0,1 | 0,5 | 0,01 | 5,0 | 0,5 | 3,0 | 0,02 5 | 10,0 |
| Dihydroxyaceaufn | | | 3,00 | 5,00 | | | 4,00 | |
| Uvinul A Plus^{™} | 2,00 | 1,50 | 0,75 | 1,00 | 2,10 | 4,50 | 5,00 | 2,10 |
| Titandioxide- microfine | 1,00 | | 1,50 | | 3,50 | | 1,50 | 1,00 |
| Zinkoxide- microfine | | 1,00 | | 0,25 | | 2,00 | | 1,50 |
| C12-15 Alkyl Benzoat | 3,50 | | | 6,35 | | | | 0,10 |
| Cocoglyceride | | 3,00 | | 3,00 | | | | 1,00 |
| Dicapryl Ether | 4,50 | | | | | | | |
| Dicaprylyl Carbonat | | 4,30 | | 3,00 | | | | 7,00 |
| Dibutyl Adipate | | | | 0,50 | | | | 0,30 |
| Phenyltrimethicone | 2,00 | | | 3,50 | | 2,00 | | |
| Cyclomethicon | | 3,00 | | | | | | |
| Ethyl Galakaufmannan | | 0,50 | | | 2,00 | | | |
| Hydrogenated Coco-Glyceride | | | | | 3,00 | 4,00 | | |
| Abil Wax 2440 | | | | | | 1,50 | 2,00 | |
| PVP Hexadecen Copolymer | | | | 1,00 | 1,20 | | | |
| Glycerin | 4,00 | 6,00 | 5,00 | | 8,00 | 10,0 0 | | |
| Vitamin E Acetat | 0,20 | 0,30 | 0,40 | | 0,30 | | | |
| Shea Butter | | 2,00 | | 3,60 | | 2,00 | | |
| lodopropyl Butylcarbamat | 0,12 | | | | 0,20 | | | |
| DMDM Hydanaufin | 0,10 | | | | 0,12 | | 0,13 | |
| Methylparaben | | 0,50 | 0,30 | | 0,35 | | | |
| Phenoxyethanol | 0,50 | 0,40 | | 1,00 | | | | |
| Ocaufxyglycerin | | 0,30 | | | 1,00 | | 0,35 | |
| Ethanol | 2,00 | | 2,00 | | | 5,00 | | |
| Trinatrium EDTA | 0,40 | | 0,15 | | | 0,20 | | |
| Parfüm | 0,20 | | 0,20 | | 0,24 | 0,16 | 0,10 | 0,10 |
| Aqua dem. | ad 100 | ad 100 | ad 100 | ad 100 | ad 100 | ad 100 | ad 100 | ad 100 |

| **Ölgel** | | | | |
|---|---|---|---|---|
| | **1** | **2** | **3** | **4** |
| Caprylic/Capric Triglycerid | 12,00 | 10,00 | 6,00 | |
| Octyldodecanol | 7,00 | 14,00 | 8,00 | 3,00 |
| Butylene Glycol Dicaprylat/Dicaprat | | | | 12,00 |
| Pentaerythrityl Tetraisostearat | 10,00 | 6,00 | 8,00 | 7,00 |
| Polyglyceryl-3 Diisostearat | 2,50 | | | |
| Bis-Diglyceryl Polyacyladipate-2 | 9,00 | 8,00 | 10,00 | 8,00 |
| Myristyl Myristate | 3,50 | 3,00 | 4,00 | 3,00 |
| Benaufne -34 | 5,00 | 5,00 | 6,00 | 6,00 |
| Propylencarbonat | 15,00 | 20,00 | 18,00 | 19,50 |
| Wässrige Lösung mit Keratin Binde-Domäne-Wirkstoff | 1,0 | 0,5 | 3,0 | 5,0 |
| Vitamin E Acetat | 0,50 | 1,00 | | |
| Ascorbyl Palmitat | 0,05 | | 0,05 | |
| Buxux Chinensis | 2,00 | 1,00 | | 1,00 |
| Parfüm, BHT | 0,10 | 0,25 | | 0,35 |
| Ricinus Communis | ad 100 | ad 100 | ad 100 | ad 100 |

## Patentansprüche

1. Kosmetische Zusammensetzung enthaltend in einem kosmetisch verträglichen Medium mindestens eine Keratin-bindende Polypeptidsequenz (i), **dadurch gekennzeichnet, dass** die Polypeptidsequenz (i) mindestens eine der folgenden Polypeptidsequenzen umfasst,
(a) die Polypeptidsequenz SEQ ID NO:1 Position 2193 bis 2481
(b) die Polypeptidsequenz SEQ ID NO:1 Position 2606 bis 2871
(c) eine Polypeptidsequenz, die gegenüber (a) in bis zu 60 % der Aminosäuren verändert ist,
(d) eine Polypeptidsequenz, die gegenüber (b) in bis zu 50 % der Aminosäuren verändert ist,
mit der Maßgabe, dass die Keratin-Bindung der Polypeptidsequenz (c) oder (d) mindestens 10 % des Wertes beträgt, den die Polypeptidsequenz (a) oder (b) aufweist, gemessen in dem Test gemäß Beispiel 9 oder 10.

2. Kosmetische Zusammensetzung gemäß Anspruch 1, **dadurch gekennzeichnet, dass** sie die Polypeptidsequenz (i) in einer Menge von 0,01 bis 30 Gew.-% enthält.

3. Kosmetische Zusammensetzung gemäß Anspruch 1, **dadurch gekennzeichnet, dass** sie neben der Polypeptidsequenz (i) mindestens einen kosmetischen Wirkstoff enthält.

4. Kosmetische Zusammensetzung gemäß Anspruch 3, **dadurch gekennzeichnet, dass** der kosmetische Wirkstoff ausgewählt wird aus der Gruppe der natürlichen oder synthetischen Polymere, Pigmente, Feuchthaltemittel, Öle, Wachse, Enzyme, Mineralien, Vitamine, Sonnenschutzmittel, Farbstoffe, Duftstoffe, Antioxidantien und Konservierungsmittel.

5. Verwendung von kosmetischen Zusammensetzungen gemäß einem der Ansprüche 1 bis 4 zur Behandlung von keratinhaltigen Materialien.

6. Verwendung von kosmetischen Zusammensetzungen gemäß einem der Ansprüche 1 bis 4 zur Behandlung von Haut, Haaren, Nägel.

7. Verwendung von kosmetischen Zusammensetzungen gemäß einem der Ansprüche 1 - 6 zur Verbesserung der Kämmbarkeit von Haaren.

8. Verwendung von kosmetischen Zusammensetzungen gemäß einem der Ansprüche 1 - 6 zur Verbesserung der Festigung von Haaren.

9. Verwendung von kosmetischen Zusammensetzungen gemäß einem der Ansprüche 1 - 6 zur konditionierenden Wirkung auf der Haut.

10. Verwendung von kosmetischen Zusammensetzungen gemäß einem der Ansprüche 1 - 6 zur Herstellung von Zusammensetzungen für die Haut-, Nagel- und Haarkosmetik.

11. Kosmetische Zusammensetzung enthaltend Keratin-bindende Effektormoleküle bestehend aus
(i) mindestens einer Polypeptidsequenz (i), die eine Bindungsaffinität zu einem Keratin besitzt und mindestens eine der folgenden Polypeptidsequenzen umfasst,
(a) die Polypeptidsequenz SEQ ID NO:1 Postition 2269 bis 2508
(b) die Polypeptidsequenz SEQ ID NO:1 Position 2606 bis 2871
(c) eine Polypeptidsequenz, die gegenüber (a) in bis zu 70% der Aminosäuren verändert ist,
(d) eine Polypeptidsequenz, die gegenüber (b) in bis zu 70 % der Aminosäuren verändert ist,
mit der Maßgabe, dass die Keratin-Bindung der Polypeptidsequenz (c) oder (d) mindestens 10 % des Wertes beträgt, den die Polypeptidsequenz (a) oder (b) aufweist, gemessen in dem Test gemäß Beispiel 9 oder 10 und,
(ii) einem Effektormolekül (ii), das natürlicherweise nicht mit der Polypeptidsequenz (i) verknüpft ist.

12. Keratin-bindende Effektormoleküle nach Anspruch 11 wobei die Polypeptidsequenz (i) Bindungsaffinität zu menschlichem Haar-, Nagel- oder Hautkeratin besitzt.

13. Kosmetische Zusammensetzung enthaltend Keratin-bindende Effektormoleküle gemäß Anspruch 11, wobei das Effektormolekül (ii) eine Polypeptidsequenz umfasst.

14. Kosmetische Zusammensetzung enthaltend Keratin-bindende Effektormoleküle gemäß Anspruch 11, wobei das Effektormolekül (ii) eine enzymatische Aktivität besitzt.

15. Keratin-bindende Effektormoleküle gemäß Anspruch 11, wobei das Effektormolekül (ii) ein Farbstoff oder eine Farbstoffkomponente ist.

16. Keratin-bindende Effektormoleküle gemäß Anspruch 11, wobei das Effektormolekül (ii) ein UV-Filter ist.

17. Keratin-bindende Effektormoleküle gemäß Anspruch 11, wobei das Effektormolekül (ii) Antioxidant ist.

18. Keratin-bindende Effektormoleküle gemäß Anspruch 11, wobei das Effektormolekül (ii) ein Carotinoid ist.

19. Keratin-bindende Effektormoleküle gemäß Anspruch 11, wobei das Effektormolekül (ii) ein Fungizid, Insektizid oder Biozid ist.

20. Keratin-bindende Effektormoleküle gemäß Anspruch 11, wobei das Effektormolekül (ii) ein Vitamin oder Provitamin ist.

21. Verwendung von Keratin-bindenden Effektormolekülen gemäß Anspruch 11 zur Behandlung von Haut, Haaren, Nägel sowie in der Tier- und Lederpflege.

22. Verwendung von Keratin-bindenden Effektormolekülen gemäß Anspruch 11 zur Herstellung von Zusammensetzungen für die Haut-, Nagel und Haarkosmetik.

23. Keratin-bindende Effektormoleküle gemäß Ansprüchen 11 bis 12, bei denen das Effektormolekül (ii) durch kovalente Bindungen mit der Polypeptidsequenz (i) verbunden ist.

24. Keratin-bindende Effektormoleküle nach Ansprüchen 11 bis 12 oder 23, bei denen das Effektormolekül (ii) unter Ausnutzung zweier in (i) und (ii) bereits vorhandener chemischer Funktionen an funktionelle Gruppen der Seitenkette, den C- oder den N-Terminus des Polypeptids (i) kovalent gebunden ist.

25. Keratin-bindende Effektormoleküle nach Ansprüchen 11 bis 12 oder 23, bei denen ein oder mehrere Effektormoleküle (ii) über einen mindestens bifunktionellen Linker an funktionelle Gruppen der Seitenkette, den C- oder den N-Terminus des Polypeptids (i) kovalent gebunden ist.

26. Keratin-bindende Effektormoleküle nach Anspruch 25, wobei der bifunktionelle Linker eine Sulfhydryl reaktive Gruppe umfasst.

27. Keratin-bindende Effektormoteküle nach Anspruch 26, wobei der bifunktionelle Linker Maleimid ist.

28. Keratin-bindende Effektormoleküle nach Ansprüchen 11 bis 12, 23 bis 27, bei denen zwischen Effektormolekül (ii) und Polypeptid (i) Spacerelemente eingebaut sind.

29. Keratin-bindende Effektormoleküle nach Ansprüchen 25 bis 28, die zwischen Effektormolekül (ii) und Polypeptid (i) eine potentielle Spaltstelle für eine Protease, Lipase, Esterase, Phosphatase oder Hydrolase besitzen.

30. Keratin-bindende Effektormoleküle nach Ansprüchen 25 bis 29, die zwischen Effektormolekül (ii) und Polypeptid (i) eine weitere Polypeptidsequenz aufweisen, die die leichte Aufreinigung des Fusionsproteins gestattet.

31. Verfahren zur Herstellung eines Keratin-bindenden Effektormoleküls gemäß Anspruch 11 umfassend die Kopplung mindestens eines kosmetischen Wirkstoffes über einen Linker an mindestens eine Aminosäure des Keratin-bindenden Polypeptids (i).

## Claims

1. A cosmetic compositions comprising, in a cosmetically compatible medium, at least one keratin-binding polypeptide sequence (i), wherein the polypeptide sequence (i) comprises at least one of the following polypeptide sequences,
(a) the polypeptide sequence SEQ ID NO:1 position 2193 to 2481
(b) the polypeptide sequence SEQ ID NO:1 position 2606 to 2871
(c) a polypeptide sequence which is modified compared with (a) in up to 60% of the amino acids,
(d) a polypeptide sequence which is modified compared with (b) in up to 50% of the amino acids,
with the proviso that the keratin binding of polypeptide sequence (c) or (d) amounts to at least 10% of the value shown by polypeptide sequence (a) or (b), measured in the test as in example 9 or 10.

2. The cosmetic composition according to claim 1, which comprises the polypeptide sequence (i) in an amount of from 0.01 to 30% by weight.

3. The cosmetic composition according to claim 1, which, besides the polypeptide sequence (i), comprises at least one cosmetic active ingredient.

4. The cosmetic composition according to claim 3, wherein the cosmetic active ingredient is chosen from the group of natural or synthetic polymers, pigments, moisturizers, oils, waxes, enzymes, minerals, vitamins, sunscreens, dyes, fragrances, antioxidants and preservatives.

5. The use of cosmetic compositions according to any of claims 1 to 4 for treating keratin-containing materials.

6. The use of cosmetic compositions according to any of claims 1 to 4 for treating skin, hair, nails.

7. The use of cosmetic compositions according to any of claims 1 - 6 for improving the combability of hair.

8. The use of cosmetic compositions according to any of claims 1 - 6 for improving the setting of hair.

9. The use of cosmetic compositions according to any of claims 1 - 6 for a conditioning effect on the skin.

10. The use of cosmetic compositions according to any of claims 1 - 6 for preparing compositions for skin, nail and hair cosmetics.

11. A cosmetic composition comprising keratin-binding effector molecules consisting of
(i) at least one polypeptide sequence (i) which has a binding affinity for a keratin and includes at least one of the following polypeptide sequences,
(a) the polypeptide sequence SEQ ID NO:1 position 2269 to 2508
(b) the polypeptide sequence SEQ ID NO:1 position 2606 to 2871
(c) a polypeptide sequence which is modified compared with (a) in up to 70% of the amino acids,
(d) a polypeptide sequence which is modified compared with (b) in up to 70% of the amino acids,
with the proviso that the keratin binding of polypeptide sequence (c) or (d) amounts to at least 10% of the value shown by polypeptide sequence (a) or (b), measured in the assay described in example 9 or 10, and,
(ii) an effector molecule (ii) which is not naturally linked to the polypeptide sequence (i).

12. The keratin-binding effector molecule according to claim 11, where the polypeptide sequence (i) has binding affinity for the keratin of human hair, nails or skin.

13. The cosmetic composition comprising keratin-binding effector molecules according to claim 11, where the effector molecule (ii) includes a polypeptide sequence.

14. The cosmetic composition comprising keratin-binding effector molecules according to claim 11, where the effector molecule (ii) has an enzymatic activity.

15. The keratin-binding effector molecule according to claim 11, where the effector molecule (ii) is a dye or a dye component.

16. The keratin-binding effector molecule according to claim 11, where the effector molecule (ii) is a UV filter.

17. The keratin-binding effector molecule according to claim 11, where the effector molecule (ii) is antioxidant.

18. The keratin-binding effector molecule according to claim 11, where the effector molecule (ii) is a carotenoid.

19. The keratin-binding effector molecule according to claim 11, where the effector molecule (ii) is a fungicide, insecticide or biocide.

20. The keratin-binding effector molecule according to claim 11, where the effector molecule (ii) is a vitamin or provitamin.

21. The use of keratin-binding effector molecules according to claim 11 for treating skin, hair, nails and in animal care and leather care.

22. The use of keratin-binding effector molecules according to claim 11 for producing compositions for skin, nail and hair cosmetics.

23. The keratin-binding effector molecule according to claims 11 to 12, where the effector molecule (ii) is attached to the polypeptide sequence (i) by covalent bonds.

24. The keratin-binding effector molecule according to claims 11 to 12 or 23, where the effector molecule (ii) is covalently bonded to functional groups of the side chain, the C-terminus or the N-terminus of the polypeptide (i), utilising two chemical functions already present in (i) and (ii).

25. The keratin-binding effector molecule according to claims 11 to 12 or 23, where one or more effector molecules (ii) is/are covalently bonded to functional groups of the side chain, the C-terminus or the N-terminus of the polypeptide (i), by way of an at least bifunctional linker.

26. The keratin-binding effector molecule according to claim 25, where the bifunctional linker comprises a sulfhydryl-reactive group.

27. The keratin-binding effector molecule according to claim 26, where the difunctional linker is maleimide.

28. The keratin-binding effector molecule according to claims 11 to 12, 23 to 27, where a spacer element is incorporated between effector molecule (ii) and polypeptide (i).

29. The keratin-binding effector molecule according to claims 25 to 28 which has a potential cleavage site for a protease, lipase, esterase, phosphatase or hydrolase between effector molecule (ii) and polypeptide (i).

30. The keratin-binding effector molecule according to claims 25 to 29 which has a further polypeptide sequence which permits easy purification of the fusion protein between effector molecule (ii) and polypeptide (i).

31. A process for preparing a keratin-binding effector molecule according to claim 11 comprising the coupling of at least one cosmetic active ingredient by way of a linker to at least one amino acid of the keratin-binding polypeptide (i).

## Revendications

1. Composition cosmétique contenant dans un milieu cosmétiquement acceptable. au moins une séquence polypeptidique (i) se liant à la kératine, **caractérisée en ce que** la séquence polypeptidique (i) englobe au moins l'une des séquences polypeptidiques suivantes
(a) la séquence polypeptidique SEQ ID n° 1, positions 2193 à 2481
(b) la séquence polypeptidique SEQ ID n° 1, positions 2606 à 2871
(c) une séquence polypeptidique qui est modifiée dans jusqu'à 60 % des aminoacides par rapport à (a),
(d) une séquence polypeptidique qui est modifiée dans jusqu'à 50 % des aminoacides par rapport à (b),
étant entendu que la liaison à la kératine de la séquence polypeptidique (c) ou (d) représente au moins 10 % de la valeur que présente la séquence polypeptidique (a) ou (b), mesurée dans le test selon l'exemple 9 ou 10.

2. Composition cosmétique selon la revendication 1, **caractérisée en ce qu'**elle contient la séquence polypeptidique (i) en une quantité de 0,01 à 30 % en poids.

3. Composition cosmétique selon la revendication 1, **caractérisée en ce qu'**en plus de la séquence polypeptidique (i) elle contient au moins une substance active cosmétique.

4. Composition cosmétique selon la revendication 3, **caractérisée en ce que** la substance active cosmétique est choisie dans le groupe des polymères naturels ou synthétiques, pigments, agents de rétention de l'humidité, huiles, cires, enzymes, minéraux, vitamines, agents de protection solaire, colorants, parfums, antioxydants, et conservateurs.

5. Utilisation de compositions cosmétiques selon l'une quelconque des revendications 1 à 4, pour le traitement de matières contenant de la kératine.

6. Utilisation de compositions cosmétiques selon l'une quelconque des revendications 1 à 4, pour le traitement de la peau, des cheveux, des ongles.

7. Utilisation de compositions cosmétiques selon l'une quelconque des revendications 1 à 6, pour l'amélioration de l'aptitude des cheveux au passage du peigne.

8. Utilisation de compositions cosmétiques selon l'une quelconque des revendications 1 à 6, pour l'amélioration du fixage des cheveux.

9. Utilisation de compositions cosmétiques selon l'une quelconque des revendications 1 à 6, pour l'effet conditionnant sur la peau.

10. Utilisation de compositions cosmétiques selon l'une quelconque des revendications 1 à 6, pour la préparation de compositions pour la cosmétique de la peau, des ongles et des cheveux.

11. Composition cosmétique contenant des molécules d'effecteurs se liant à la kératine, constituées de
(i) au moins une séquence polypeptidique (i) qui possède une affinité de liaison pour une kératine et englobe au moins l'une des séquences polypeptidiques suivantes
(a) la séquence polypeptidique SEQ ID n° 1, positions 2269 à 2508
(b) la séquence polypeptidique SEQ ID n° 1, positions 2606 à 2871
(c) une séquence polypeptidique qui est modifiée dans jusqu'à 70 % des aminoacides par rapport à (a),
(d) une séquence polypeptidique qui est modifiée dans jusqu'à 70 % des aminoacides par rapport à (b),
étant entendu que la liaison à la kératine de la séquence polypeptidique (c) ou (d) représente au moins 10 % de la valeur que présente la séquence polypeptidique (a) ou (b), mesurée dans le test selon l'exemple 9 ou 10 et
(ii) une molécule d'effecteur (ii) qui à l'état naturel n'est pas liée à la séquence polypeptidique (i).

12. Molécules d'effecteurs se liant à la kératine selon la revendication 11, dans lesquelles la séquence polypeptidique (i) possède une affinité de liaison pour la kératine humaine des cheveux, des ongles ou de la peau.

13. Composition cosmétique contenant des molécules d'effecteurs se liant à la kératine selon la revendication 11, dans laquelle la molécule d'effecteur (ii) comprend une séquence polypeptidique.

14. Composition cosmétique contenant des molécules d'effecteurs se liant à la kératine selon la revendication 11, dans laquelle la molécule d'effecteur (ii) possède une activité enzymatique.

15. Molécules d'effecteurs se liant à la kératine selon la revendication 11, dans lesquelles la molécule d'effecteur (ii) est un colorant ou un composant de colorant.

16. Molécules d'effecteurs se liant à la kératine selon la revendication 11, dans lesquelles la molécule d'effecteur (ii) est un filtre UV.

17. Molécules d'effecteurs se liant à la kératine selon la revendication 11, dans lesquelles la molécule d'effecteur (ii) est un antioxydant.

18. Molécules d'effecteurs se liant à la kératine selon la revendication 11, dans lesquelles la molécule d'effecteur (ii) est un caroténoïde.

19. Molécules d'effecteurs se liant à la kératine selon la revendication 11, dans lesquelles la molécule d'effecteur (ii) est un fongicide, un insecticide ou un biocide.

20. Molécules d'effecteurs se liant à la kératine selon la revendication 11, dans lesquelles la molécule d'effecteur (ii) est une vitamine ou une provitamine.

21. Utilisation de molécules d'effecteurs se liant à la kératine selon la revendication 11, pour le traitement de la peau, des cheveux, des ongles ainsi que dans l'entretien du cuir et d'animaux.

22. Utilisation de molécules d'effecteurs se liant à la kératine selon la revendication 11, pour la fabrication de compositions pour la cosmétique de la peau, des ongles et des cheveux.

23. Molécules d'effecteurs se liant à la kératine selon les revendications 11 et 12, dans lesquelles la molécule d'effecteur (ii) est liée par des liaisons covalentes à la séquence polypeptidique (i).

24. Molécules d'effecteurs se liant à la kératine selon les revendications 11 et 12 ou 23, dans lesquelles la molécule d'effecteur (ii) est liée par covalence, avec utilisation de deux fonctions chimiques, déjà présentes dans (i) et (ii), sur des groupes fonctionnels de la chaîne latérale, à l'extrémité C-terminale ou à l'extrémité N-terminale du polypeptide (i).

25. Molécules d'effecteurs se liant à la kératine selon les revendications 11 et 12 ou 23, dans lesquelles une ou plusieurs molécules d'effecteurs (ii) est/sont liée(s) par covalence, au moyen d'un lieur au moins bifonctionnel sur des groupes fonctionnels de la chaîne latérale, à l'extrémité C-terminale ou à l'extrémité N-terminale du polypeptide (i).

26. Molécules d'effecteurs se liant à la kératine selon la revendication 25, dans lesquelles le lieur bifonctionnel comprend un groupe réactif sulfhydryle.

27. Molécules d'effecteurs se liant à la kératine selon la revendication 26, dans lesquelles le lieur bifonctionnel est le maléimide.

28. Molécules d'effecteurs se liant à la kératine selon les revendications 11 et 12, 23 à 27, dans lesquelles des éléments espaceurs sont incorporés entre molécule d'effecteur (ii) et polypeptide (i).

29. Molécules d'effecteurs se liant à la kératine selon les revendications 25 à 28, qui possèdent entre molécule d'effecteur (ii) et polypeptide (i) un site potentiel de coupure pour une protéase, lipase, estérase, phosphatase ou hydrolase.

30. Molécules d'effecteurs se liant à la kératine selon les revendications 25 à 29, qui comportent entre molécule d'effecteur (ii) et polypeptide (i) une autre séquence polypeptidique qui permet la purification aisée de la protéine de fusion.

31. Procédé pour la production d'une molécule d'effecteur se liant à la kératine selon la revendication 11, comprenant le couplage d'au moins une substance active cosmétique, au moyen d'un lieur, à au moins un aminoacide du polypeptide (i) se liant à la kératine
